(19) Europäisches Patentamt
European Patent Office
Office européen des brevets

(11) EP 0 814 078 B1

(12) **EUROPÄISCHE PATENTSCHRIFT**

(45) Veröffentlichungstag und Bekanntmachung des Hinweises auf die Patenterteilung:
**05.04.2000 Patentblatt 2000/14**

(51) Int Cl.[7]: **C07C 403/20**, C07C 67/00, C07C 69/734, C07C 69/602

(21) Anmeldenummer: **97109473.5**

(22) Anmeldetag: **11.06.1997**

(54) **Herstellung von Polyenestern und -säuren**

Process for the preparation of polyenic esters and -acids

Procédé pour la préparation d'esters et d'acides polyéniques

(84) Benannte Vertragsstaaten:
**AT BE CH DE DK ES FR GB IT LI NL**

(30) Priorität: **17.06.1996 EP 96109660**

(43) Veröffentlichungstag der Anmeldung:
**29.12.1997 Patentblatt 1997/52**

(73) Patentinhaber: **F. HOFFMANN-LA ROCHE AG**
**4070 Basel (CH)**

(72) Erfinder: **Rüttimann, August**
**4144 Arlesheim (CH)**

(74) Vertreter: **Kellenberger, Marcus, Dr. et al**
**F.Hoffmann-La Roche AG**
**Patent Department (PLP),**
**124 Grenzacherstrasse**
**4070 Basel (CH)**

(56) Entgegenhaltungen:

- **HERTLER W R ET AL: "Anion-catalyzed reactions of silyl ester polyenolates with electrophiles" J. ORG. CHEM. (JOCEAH,00223263);88; VOL.53 (15); PP.3532-9, E. I. DU PONT DE NEMOURS AND CO., INC.;CENT. RES. DEV. DEP.; WILMINGTON; 19898; DE; USA (US), XP002041171**
- **YAMAMOTO Y ET AL: "Carbon-carbon bond formation at the.gamma.-position of dienolates via the germanium masked dienolates" J. CHEM. SOC., CHEM. COMMUN. (JCCCAT,00224936);88; (24); PP.1639-40, TOHOKU UNIV.;FAC. SCI.; SENDAI; 980; JAPAN (JP), XP002041172**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein neues Verfahren zur Herstellung von Polyenestern und -säuren aus acetalierten Polyenaldehyden durch eine säurekatalysierte Kondensationsreaktion mit Vinylketenacetalen.

Lewissäure-katalysierte Additionen von α,β-ungesättigten Ethern (Enolethern) an Acetale sind schon lange bekannt und gehen auf die Arbeiten von Müller-Cunradi und Pieroh zurück (siehe US-Patentschrift 2.165.962). Hoaglin und Hirsch [J.A.C.S. 71, 3468 (1949)] haben diese Reaktion weiter untersucht und die Anwendungsmöglichkeiten erweitert, was ebenfalls Isler et al. in den fünfziger Jahren getan haben, und zwar auf die Synthese von β-Carotin, Crocetindialdehyd, Lycopin sowie β-Apocarotinoiden [siehe Helv. Chim. Acta 39, 249 ff. und 463 ff. (1956), ibid. 42, 854 ff. (1959) sowie US-Patentschriften 2.827.481 und 2.827.482]. Später erweiterte Mukaiyama [Angew. Chem. 89, 858 ff. (1977) und Org. Reactions 28, 203 ff. (1982)] die Reaktion durch Verwendung der leicht zugänglichen Trimethylsilylenolether.

Ueber die ersten Lewissäure-katalysierten Kondensationen von 1-Alkoxy-1,3-dienen (Dienolethern) mit α,β-ungesättigten Acetalen wurde von Nazarov und Krasnaya [J. Gen. Chem. USSR 28, 2477 ff. (1958)] und von Makin [Pure & Appl. Chem. 47, 173 ff. (1976), J. Gen. Chem. USSR 31, 3096 ff. (1961) und 32, 3112 ff. (1962)] berichtet. Dabei erfolgt die Kopplung des Acetals an den Dienolether soweit ersichtlich ausschliesslich an dessen γ-Stellung unter Bildung eines kettenverlängerten α,β-ungesättigten Acetals, das jedoch in Konkurrenz zum ersten Acetal mit weiterem Dienolether reagiert unter Bildung eines weiteren, kettenverlängerten α,β-ungesättigten Acetals usw. [Telomerbildung; siehe auch noch Chemla et al., Bull. Soc. Chim. Fr. 130, 200 ff. (1993)]. Aus diesem Grund ist eine derartige Kondensation für synthetische Zwecke, speziell für die Synthese von Apocarotinoiden, als nicht brauchbar befunden worden [Isler et al., Adv. Org. Chem. 4, 115 ff. (1963)].

Nicht nur 1-Alkoxy-1,3-diene, sondern auch Trimethylsilyloxydiene [vom Typ $CH_2=CH-CH=CH-OSi(CH_3)_3$] können in Gegenwart von Lewissäure-Katalysatoren mit Acetalen kondensiert werden, wie dies Mukaiyama et al. in Chem. Lett. 1975, 319 ff. offenbarten. Auch bei dieser Kopplung erfolgt der Angriff ausschliesslich am endständigen (γ-) Kohlenstoffatom des Diensystems, um "γ-Produkte" zu bilden [siehe Mukaiyama et al., Bull. Chem. Soc. Jap 50, 1161 ff. (1977) sowie Japanische Patentpublikation (Kokai) 36.645/1977]. Im Gegensatz zur Reaktion mit 1-Alkoxy-1,3-dienen wird bei der Umsetzung von Trimethylsilyloxydienen mit Acetalen ein Aldehyd gebildet, der mit dem Dien nicht weiterreagiert (keine Telomerbildung). Unter Verwendung dieser Methode konnten Mukaiyama et al. Vitamin A synthetisieren [siehe Kokai 36.645/1977, Chem. Lett. 1975, 1201 ff. sowie Bull. Chem. Soc. Japan 51, 2077 ff. (1978)] und Mitarbeiter von Rhône-Poulenc neue Zugänge zu Carotinoiden und Vitamin A entwickeln (siehe DOS 2.701.489 und A.E.C. Société de Chimie Organique et Biologique Nr. 7824350).

Analog zu den obenerwähnten Trimethylsilyloxydienen können auch silylierte Vinylketenacetale [vom Typ $CH_2=CH-CH=C(OAlkyl)(OSi(CH_3)_3)$] mit Acetalen reagieren [siehe Tetr. Lett. 20, 3209 ff. (1979) sowie Chimia 34, 265 ff. (1980)]. Wie aus u.a. Tetr. Lett. 22, 2833 ff. (1981), ibid. 26, 397 ff. (1985), DOS 3.244.273 und US-Patentschrift 4.937.308 ersichtlich, werden jedoch bei dieser Reaktion bis jetzt immer (nicht leicht trennbare) Gemische der beiden möglichen γ- und α-Kopplungsprodukte ["γ-Produkte" ....$CH(OAlkyl^1)-CH_2-CH=CH-COOAlkyl^2$; "α-Produkte" ....$CH(OAlkyl^1)-C(CH_3)(CH=CH_2)-COOAlkyl^2$] gebildet. Daher schien auch diese Reaktion für synthetische Zwecke auf dem Gebiet der Carotinoide kaum brauchbar zu sein. Interessant und nützlich würde diese Reaktion erst, wenn gänzliche γ-Selektivität erreicht werden könnte, beispielsweise zur Synthese von Polyenen, und zwar Apoestern, Crocetinestern usw.; denn durch Elimination des Alkohols $Alkyl^1OH$ aus dem γ-Produkt könnte gewünschtenfalls eine weitere (konjugierte) Doppelbindung gebildet werden, und zwar unter Bildung des Produktes ...$CH=CH-CH=CH-COOAlkyl^2$. Dadurch könnten solche Polyene ohne Einsatz der bisher zu diesem Zweck verwendeten Wittig- oder Horner-Reaktion hergestellt werden.

Ziel der vorliegenden Erfindung ist es, ausgehend von Polyen(di)acetalen und Vinylketenacetalen oder silylierten Analogen davon entsprechend kettenverlängerte Polyen(di)ester oder -säuren herzustellen, und zwar unter möglichst weitgehender Vermeidung der obenerwähnten Nachteile des Standes der Technik sowie mit Ersatz der bisher zu diesem Zweck verwendeten Wittig- oder Horner-Reaktion. Dieses Ziel wird erfindungsgemäss erreicht, indem man ein Polyen-(di-)O,O-dialkylacetal mit einem Vinyl-O,O-dialkyl- oder O,O-alkylen-ketenacetal oder einem O-mono- oder O,O-disilylierten Analog davon in Gegenwart einer Lewissäure zu dem entsprechend kettenverlängerten (bis-)δ-Alkoxy-γ,δ-gesättigten Polyenester oder der entsprechenden (Di-)Säure umsetzt, und anschliessend vom so gebildeten (Di-)Ester bzw. von der entsprechenden (Di-)Säure unter basischen Bedingungen das δ-ständige Alkanol eliminiert, um den erwünschten (konjugierten) Polyen(di)ester bzw. die entsprechende (Di-)Säure zu erhalten. Nicht nur ist die Reaktion des Vinyl-O,O-dialkyl- oder Vinyl-O,O-alkylen-ketenacetals oder des O-monosilylierten oder O,O-disilylierten Analogs mit dem Polyen-O,O-dialkylacetal neu, sondern es erfolgt dabei überraschenderweise ein (soweit ersichtlich) ausschliesslicher Angriff an der γ-Stellung des Vinylketenacetalderivats. Durch die anschliessende baseninduzierte Elimination des Alkanols wird eine (konjugierte) C-C-Doppelbindung gebildet, ohne dass dazu ein phosphor- oder siliziumhaltiges Reagens benötigt wird, was im Gegensatz zu der bisher auf diesem Gebiet üblicherweise verwendeten Methodik steht.

Demnach handelt es sich bei der vorliegenden Erfindung um ein Verfahren zur Herstellung eines Polyenesters oder einer Polyensäure der allgemeinen Formel

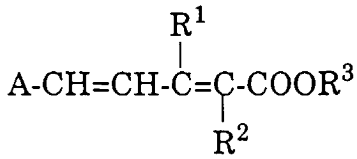

I'

oder

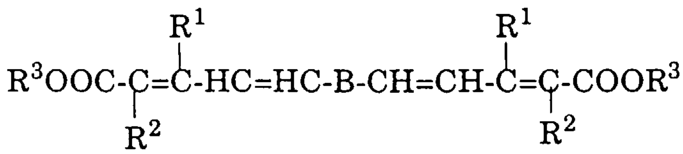

I''

worin

| | |
|---|---|
| A | eine monovalente, gegebenenfalls methylsubstituierte, konjugierte Polyengruppe, |
| B | eine bivalente, gegebenenfalls methylsubstituierte, konjugierte Polyengruppe, |
| $R^1$ und $R^2$ | jeweils Wasserstoff oder Methyl und |
| $R^3$ | Wasserstoff oder $C_{1-6}$-Alkyl bedeuten, wobei sich die Gruppe(n) $-CH=CH-C(R^1)=C(R^2)-COOR^3$ jeweils in der(den) Endstellung(en) der konjugierten Kette der Gruppe A bzw. B befindet(n), |

das dadurch gekennzeichnet ist, dass man ein Polyen-(di-)O,O-dialkylacetal der allgemeinen Formel

$$A\text{-}CH(OR^4)_2 \qquad \text{II'}$$

bzw.

$$(R^4O)_2HC\text{-}B\text{-}CH(OR^4)_2 \qquad \text{II''}$$

worin

| | |
|---|---|
| A und B | die oben angegebenen Bedeutungen besitzen, wobei sich in diesem Fall die Gruppe(n) $-CH(OR^4)_2$ jeweils in der(den) Endstellung(en) der konjugierten Kette der Gruppe A bzw. B befindet(n) und |
| $R^4$ | $C_{1-6}$-Alkyl bedeutet, |

mit einem Vinylketenacetal oder Analog davon der allgemeinen Formel

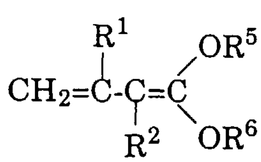

III

worin

| | |
|---|---|
| $R^1$ und $R^2$ | die oben angegebenen Bedeutungen besitzen und |
| $R^5$ | $C_{1-6}$-Alkyl und |

$R^6$ $C_{1-6}$-Alkyl oder Tri($C_{1-6}$-alkyl)silyl bedeuten,

oder $R^5$ und $R^6$ beide Tri($C_{1-6}$-alkyl)silyl bedeuten,
oder $R^5$ und $R^6$ zusammen 1,2-Ethylen oder 1,3-Trimethylen bilden,
in Gegenwart einer Lewissäure umsetzt, im Falle der Verwendung eines Vinylketenacetals der Formel III, in der $R^5$ und $R^6$ beide $C_{1-6}$-Alkyl oder beide Tri($C_{1-6}$-alkyl)silyl bedeuten oder zusammen 1,2-Ethylen oder 1,3-Trimethylen bilden, das Reaktionsgemisch hydrolysiert, anschliessend (in allen Fällen) von der so hergestellten Verbindung der allgemeinen Formel

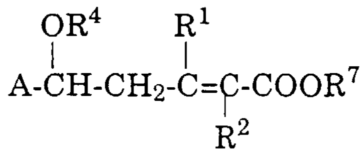

IV'

bzw.

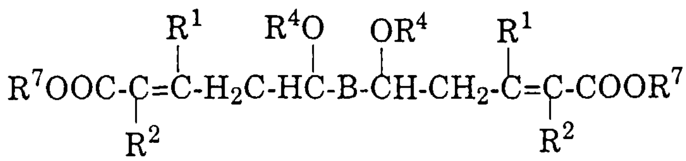

IV''

worin

A, B, $R^1$, $R^2$ und $R^4$ die oben angegebenen Bedeutungen besitzen, wobei sich in diesem Fall die Gruppe(n) $-CH(OR^4)-CH_2-C(R^1)=C(R^2)COOR^7$ jeweils in der(den) Endstellung(en) der konjugierten Kette der Gruppe A bzw. B befindet(n), und
$R^7$ $C_{1-6}$-Alkyl, Wasserstoff, 2-Hydroxyethyl oder 3-Hydroxy-n-propyl bedeutet,

unter stark basischen Bedingungen den Alkohol $R^4OH$ abspaltet und im Falle eines Unterschieds zwischen der(den) Gruppe(n) $-COOR^7$ und $-COOR^3$ die erstere(n) in die letztere(n) überführt.

Das erfindungsgemässe Verfahren kann im Prinzip bei allen obenerwähnten Polyen-O,O-dialkylacetalen der Formel II' oder Polyen-di(O,O-dialkylacetalen) der Formel II'', welche am Ende bzw. an beiden Enden der Polyenkette die Acetalgruppe $-CH(OR^4)_2$ aufweisen, Verwendung finden. Unter solchen Edukten befinden sich u.a. die folgenden UnterKlassen [wobei die in der Carotinoid-Chemie übliche (mit einfachen Strichen) abgekürzte Darstellungsweise für die Strukturformeln benutzt wird]:

Alicyclisch-aliphatische Polyen-O,O-dialkylacetale, die hauptsächlich dem Gebiet der Carotinoide [als Acetale asymmetrischer Carotinoid-Aldehyde mit einem sechsgliedrigen (Cyclohexen-)Ring] angehören, der allgemeinen Formel

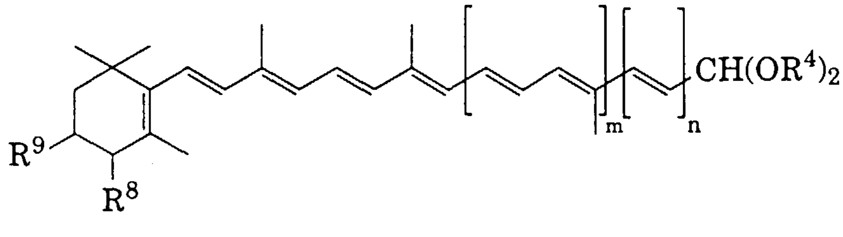

IIa

worin

$R^4$ die oben angegebene Bedeutung besitzt und
$R^8$ und $R^9$ unabhängig voneinander Wasserstoff, eine gegebenenfalls geschützte Hydroxygruppe oder eine gegebenenfalls geschützte Oxogruppe,

m 0, 1, 2, 3 oder 4 und
n 0 oder 1 bedeuten,

welche nach Durchführung des erfindungsgemässen mehrstufigen Verfahrens in die entsprechenden alicyclisch-aliphatischen Polyenester oder -säuren der allgemeinen Formel

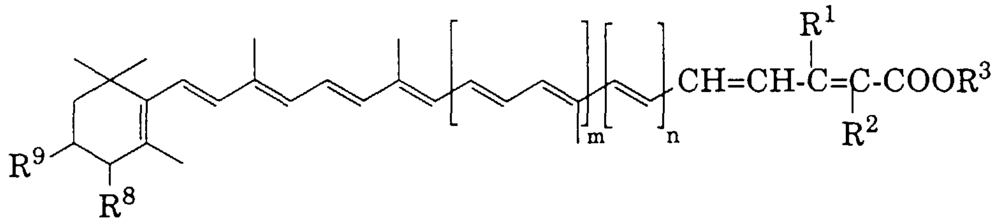

Ia

übergeführt werden;

Aliphatische Polyen-O,O-dialkylacetale, die ebenfalls hauptsächlich dem Gebiet der Carotinoide (als Acetale offenkettiger asymmetrischer Carotinoid-Aldehyde) angehören, der allgemeinen Formel

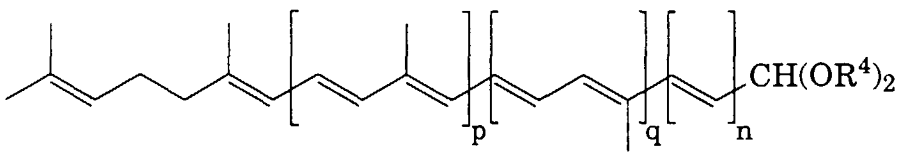

IIb

worin

$R^4$ die oben angegebene Bedeutung besitzt und
p 0, 1 oder 2,
q 0, 1, 2 oder 3 und
n 0 oder 1 bedeuten,

welche nach Durchführung des erfindungsgemässen mehrstufigen Verfahrens in die entsprechenden aliphatischen Polyenester oder -säuren der allgemeinen Formel

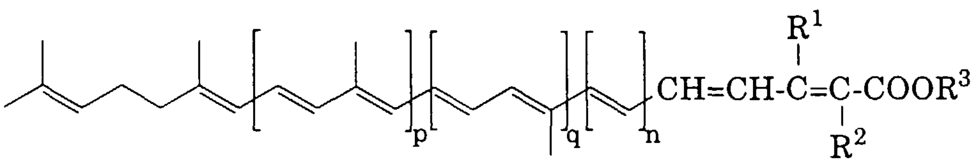

Ib

übergeführt werden;

Aliphatische Polyen-di(O,O-dialkylacetale), die ebenfalls hauptsächlich dem Gebiet der Carotinoide (als Acetale symmetrischer Carotinoid-Dialdehyde) angehören, der allgemeinen Formel

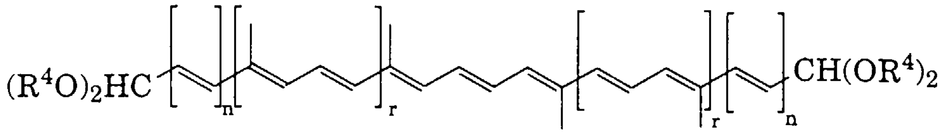

IIc

worin

$R^4$ die oben angegebene Bedeutung besitzt und
r 0, 1 oder 2 und

n 0 oder 1 bedeuten,

welche nach Durchführung des erfindungsgemässen mehrstufigen Verfahrens in die entsprechenden aliphatischen Polyendiester oder -disäuren der allgemeinen Formel

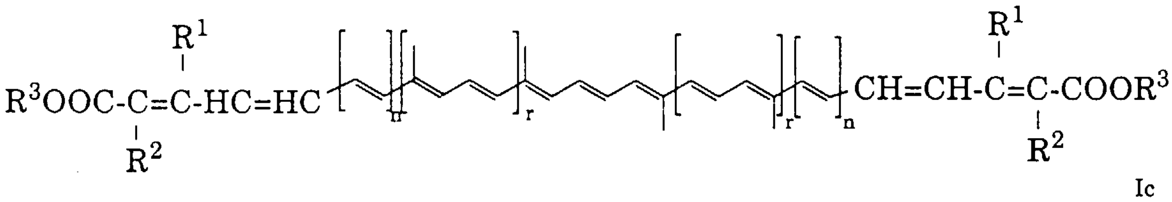

Ic

übergeführt werden.

Die Edukte der allgemeinen Formeln IIa, IIb und IIc können durch die allgemeine Formel II umfasst werden:

$$R - CH(OR^4)_2 \qquad II$$

worin

R eine Gruppe (a), (b) oder (c)

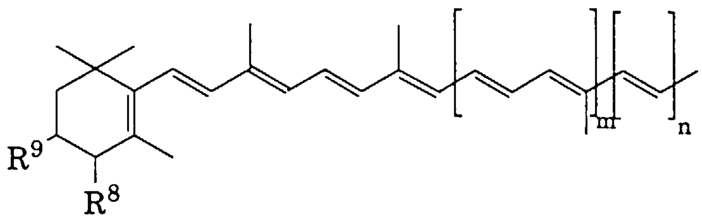

(a)

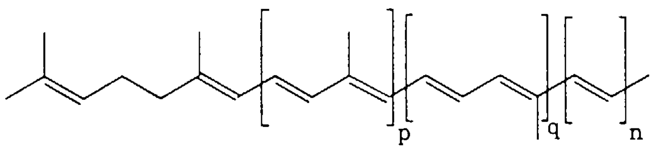

(b)

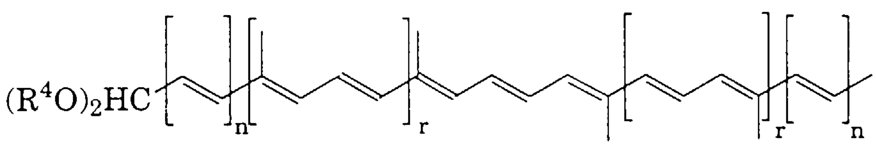

(c)

bedeutet und

$R^4$, $R^8$, $R^9$, m, n, p, q und r die oben angegebenen Bedeutungen besitzen.

Nach Durchführung des erfindungsgemässen mehrstufigen Verfahrens wird das Edukt der allgemeinen Formel II in das entsprechende Produkt der allgemeinen Formel I übergeführt:

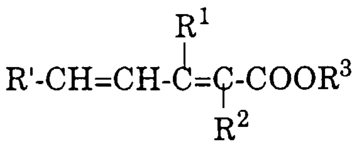

I

worin R' die oben angegebene Bedeutung von R hat, wobei, im Falle, dass R' eine Gruppe (c) bedeutet, die Dialkoxyethylgruppe $(R^4O)_2HC$- durch die Gruppe $R^3OOC-C(R^2)=C(R^1)-HC=HC$- ersetzt ist.

Die Formel I umfasst dann die Formeln Ia, Ib und Ic.

Falls das Produkt der Formel I, insbesondere der Formel Ia, am Cyclohexenring eine oder zwei geschützte Gruppen ($R^8$, $R^9$) aufweist, kann man gewünschtenfalls die vorhandene(n) Schutzgruppe(n) abspalten, was einen weiteren Aspekt der vorliegenden Erfindung darstellt.

Im Rahmen der vorliegenden Erfindung umfasst der Ausdruck "$C_{1-6}$-Alkyl" geradkettige und verzweigte Gruppen, wie beispielsweise Methyl, Ethyl und Isobutyl. Dies gilt für die $C_{1-6}$-Alkylgruppe einer diese enthaltenden Gruppe, z.B. Tri($C_{1-6}$-alkyl)silyl.

Der Ausdruck "geschützte Hydroxygruppe" umfasst übliche (besonders auf dem Gebiet der Carotinoide geläufige) geschützte Hydroxygruppen, insbesondere verätherte Hydroxygruppen und Acyloxygruppen. Bei den "verätherten Hydroxygruppen" handelt es sich beispielsweise um $C_{1-5}$-Alkoxygruppen, vorzugsweise Methoxy und Ethoxy; $C_{2-16}$-Alkoxyalkoxygruppen, vorzugsweise 1-Methoxy-1-methylethoxy; Arylalkoxygruppen, vorzugsweise Benzyloxy; Tetrahydropyranyloxy; und Tri($C_{1-5}$-alkyl)silyloxygruppen, vorzugsweise Trimethylsilyloxy. Die Acyloxygruppen umfassen insbesondere Alkanoyloxy- und Aroyloxygruppen mit bis zu 8 Kohlenstoffatomen, wie beispielsweise Formyloxy, Acetoxy, Propionyloxy und Benzoyloxy.

Der Ausdruck "geschützte Oxogruppe" umfasst ebenfalls übliche (besonders auf dem Gebiet der Carotinoide geläufige) geschützte Oxogruppen. Bevorzugt sind die acetalisierten Oxogruppen, insbesondere diejenigen, worin der Ausdruck geschützte Oxogruppe für zwei $C_{1-5}$-Alkoxygruppen (z.B. für zwei Methoxygruppen) oder für eine $C_{2-6}$-Alkylendioxygruppe (z.B. Ethylendioxy oder 2,3-Butylendioxy) steht. Ferner kann eine Oxogruppe auch als Enolether geschützt sein, und zwar vor allem im Falle von $\alpha$-Hydroxyketonen (z.B. $R^8$ und $R^9$ bedeuten Hydroxy resp. Oxo oder umgekehrt), wobei die Verätherung des Endiols vorzugsweise auch durch Bildung eines cyclischen Acetals oder Ketals (z.B. mit Aceton zum Acetonid) erfolgen kann. Die Oxogruppe kann auch beispielsweise als ein Imin geschützt sein.

Die im Rahmen der vorliegenden Erfindung offenbarten Formeln von Polyenen umfassen jeweils isomere Formen, z.B. optisch aktive und cis/trans bzw. E/Z-Isomere, sowie Gemische hiervon, sofern nicht ausdrücklich anders erwähnt. Als Beispiel eines chiralen (optischen aktiven) Zentrums sei erwähnt das den Rest $R^8$ oder $R^9$ tragende Kohlenstoffatom, falls $R^8$ bzw. $R^9$ eine gegebenenfalls geschützte Hydroxygruppe bedeutet (siehe Formeln Ia und IIa). Was die E/Z-Isomerie anbelangt, so sind im allgemeinen die (all-E)-Isomeren der Edukte und der Produkte des erfindungsgemässen Verfahrens bevorzugt.

Der erste Verfahrensschritt des erfindungsgemässen Verfahrens wird zweckmässigerweise durchgeführt, indem man das Polyen-(di-)O,O-dialkylacetal der Formel II' oder II'' mit dem Vinylketenacetal(-Analog) der Formel III in einem organischen Lösungsmittel bei Temperaturen im Bereich von etwa -40°C bis etwa 100°C, vorzugsweise im Temperaturbereich von etwa -20°C bis zur Raumtemperatur, und in Gegenwart einer Lewissäure umsetzt. Als organische Lösungsmittel eignen sich im allgemeinen alle aprotischen polaren oder unpolaren Lösungsmittel. Besonders bevorzugte derartige Lösungsmittel sind niedere aliphatische und cyclische Kohlenwasserstoffe, z.B. n-Pentan, n-Hexan und Cyclohexan; niedere, halogenierte aliphatische Kohlenwasserstoffe, z.B. Methylenchlorid und Chloroform; niedere aliphatische und cyclische Ether, z.B. Diethylether, tert.Butylmethylether und Tetrahydrofuran; niedere aliphatische Nitrile, z.B. Acetonitril; sowie Aromaten, z.B. Toluol. Beispiele der verwendbaren Lewissäuren sind Zinkchlorid, Zinkbromid, Titantetrachlorid, Lithiumperchlorat, Bortrifluoridetherat sowie Eisen(III)chlorid; diese werden im allgemeinen in katalytischen Mengen eingesetzt, und zwar zweckmässigerweise in einer Menge, die zwischen etwa 0,1 und 10 Molprozent bezogen auf die eingesetzte Menge des Polyen-(di-)O,O-dialkylacetals beträgt, und vorzugsweise im Molprozentbereich von 1 bis 3 liegt. Zudem verwendet man zweckmässigerweise von etwa 1,1 bis etwa 1,6 Aequivalente Vinylketenacetal (-Analog) pro Aequivalent Polyen-(di-)O,O-dialkylacetal, vorzugsweise etwa 1,3 bis etwa 1,4 Aequivalente. Ausserdem erfolgt die Umsetzung zweckmässigerweise bei Normaldruck, wobei im allgemeinen der Druck nicht kritisch ist.

Im Falle des Einsatzes eines Vinylketenacetals der Formel III, in der $R^5$ und $R^6$ beide $C_{1-6}$-Alkyl bedeuten oder zusammen 1,2-Ethylen oder 1,3-Trimethylen bilden, wird nach erfolgter Umsetzung des Polyen-(di-)O,O-dialkylacetals der Formel II' bzw. II'' mit diesem Vinylketenacetal noch keine Verbindung der Formel IV' bzw. IV'' hergestellt, sondern stattdessen ein Zwischenprodukt der allgemeinen Formel

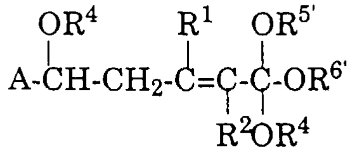

V'

bzw.

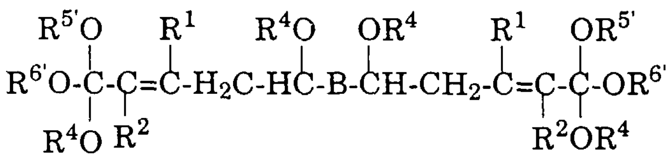

V''

worin

| | |
|---|---|
| A, B, $R^1$, $R^2$ und $R^4$ | die oben angegebenen Bedeutungen besitzen und |
| $R^{5'}$ und $R^{6'}$ | beide $C_{1-6}$-Alkyl bedeuten oder zusammen 1,2-Ethylen oder 1,3-Trimethylen bilden. |

Auch im Falle des Einsatzes eines Vinylketenacetals der Formel III, in der $R^5$ und $R^6$ beide Tri($C_{1-6}$-alkyl)silyl bedeuten, wird nach erfolgter Umsetzung des Polyen-(di-)O,O-dialkylacetals der Formel II' bzw. II'' mit diesem Vinylketenacetal noch keine Verbindung der Formel IV' bzw. IV'' hergestellt, sondern stattdessen in diesem Fall ein Zwischenprodukt der allgemeinen Formel

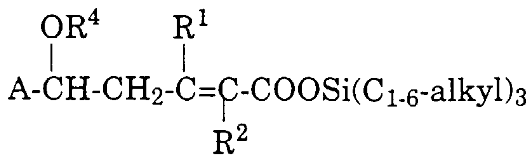

V'''

bzw.

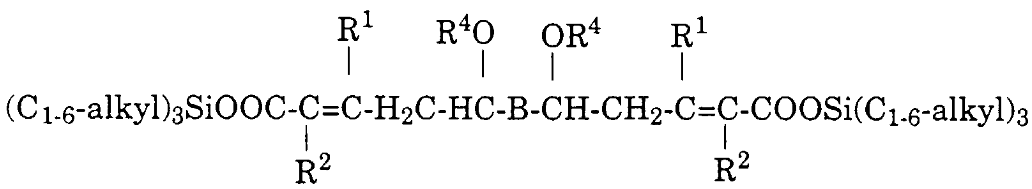

V''''

worin A, B, $R^1$, $R^2$ und $R^4$ die oben angegebenen Bedeutungen besitzen.

Gewünschtenfalls könnte das jeweilige Zwischenprodukt aus dem Reaktionsgemisch isoliert und anschliessend zur entsprechenden Verbindung der Formel IV' bzw. IV'' [in der $R^7$ jeweils $C_{1-6}$-Alkyl ($R^{5'}$ und $R^{6'}$ bedeuteten beide $C_{1-6}$-Alkyl), 2-Hydroxyethyl oder 3-Hydroxy-n-propyl ($R^{5'}$ und $R^{6'}$ bildeten zusammen 1,2-Ethylen bzw. 1,3-Trimethylen) bzw. Wasserstoff (ausgehend von dem Zwischenprodukt der Formel V''' oder V'''') bedeutet] hydrolysiert werden. Allerdings erweist es sich als zweckmässiger, keine derartige Isolierung und nachfolgende Hydrolyse vorzunehmen, sondern unmittelbar nach Beendigung der Reaktion II'/II'' + III → V'/V''/V'''/V'''' das Zwischenprodukt im Reaktionsgemisch selber zu hydrolysieren, um in diesen Fällen zur Verbindung der Formel IV' oder IV'' (nachfolgend als IV'/IV'' abgekürzt) zu gelangen. Ausgehend von einem Zwischenprodukt der Formel V' oder V'' kann die Hydrolyse geeigneterweise erfolgen, indem man zum Reaktionsgemisch eine wässrige Lösung einer schwachen Säure, vorzugsweise leicht verdünnte wässrige Essigsäure, gibt und das Gemisch anschliessend eine Weile, beispielsweise etwa 30 Minuten bis etwa 2 Stunden, rührt, und zwar zweckmässigerweise im Temperaturbereich von etwa 0°C bis zur Raumtemperatur. Im anderen Fall, d.h. ausgehend von einem Zwischenprodukt der Formel V''' oder V'''', erfolgt die Hydrolyse

noch viel leichter, und zwar mit Wasser allein; die Hydrolyse kann sogar im Rahmen der normalen Aufarbeitung erfolgen, bei der hauptsächlich Wasser als Reinigungsmittel eingesetzt wird.

Je nach eingesetztem Vinylketenacetal(-Analog) der Formel III erhält man nach Durchführung des ersten Verfahrensschrittes sowie einer eventuell benötigten Hydrolyse, wie oben näher erläutert, das entsprechende Produkt der Formel IV'/IV'', und zwar:

- nach Einsatz eines Vinylketenacetal(-Analog)s der Formel III, in der $R^5$ $C_{1-6}$-Alkyl und $R^6$ $C_{1-6}$-Alkyl oder Tri($C_{1-6}$-alkyl)silyl bedeuten, eine Verbindung der Formel IV'/IV'', in der $R^7$ $C_{1-6}$-Alkyl bedeutet (im Falle, dass $R^6$ Tri($C_{1-6}$-alkyl)silyl bedeutet, wird diese Gruppe eliminert);
- nach Einsatz eines Vinylketenacetal-Analogs der Formel III, in der $R^5$ und $R^6$ beide Tri($C_{1-6}$-alkyl)silyl bedeuten, eine Verbindung der Formel IV'/IV'', in der $R^7$ Wasserstoff bedeutet [beide Tri($C_{1-6}$-alkyl)silylgruppen werden eliminiert, und das Produkt weist eine bzw. zwei endständige 4-Carboxy-1-($C_{1-6}$-alkoxy)-3-butenylgruppe(n) oder entsprechende 3- und/oder 4-methylsubstituierte ($R^1$ und/oder $R^2$ = Methyl) Gruppe(n) $-CH(OR^4)-CH_2-C(R^1)=C(R^2)-COOH$ auf]; und
- nach Einsatz eines Vinylketenacetals der Formel III, in der $R^5$ und $R^6$ zusammen 1,2-Ethylen oder 1,3-Trimethylen bilden, eine Verbindung der Formel IV'/IV'', in der $R^7$ 2-Hydroxyethyl bzw. 3-Hydroxy-n-propyl bedeutet.

Das jeweilige Produkt kann in an sich bekannter Weise vom Reaktionsgemisch isoliert und gewünschtenfalls gereinigt werden. Typischerweise wird das Gemisch mit Wasser vereinigt und das Ganze mit einem mit Wasser nicht mischbaren organischen Lösungsmittel, wie beispielsweise mit einem niederen Alkan oder Dialkylether, z.B. n-Hexan bzw. tert.Butylmethylether, extrahiert und die organische Phase mit Wasser und/oder gesättigter wässriger Natriumchlorid- und/oder Natriumbicarbonat-Lösung gewaschen, getrocknet und eingeengt. Das so isolierte und zumindest einigermassen gewaschene Rohprodukt kann dann gewünschtenfalls weitergereinigt werden, beispielsweise durch Säulenchromatographie, z.B. unter Verwendung von solchen Eluierungsmitteln als n-Hexan, Ethylacetat, Toluol oder Gemischen davon, oder (Um)Kristallisation, beispielsweise aus einem Alkohol, z.B. Methanol oder Ethanol. Alternativ, und oft bevorzugt, kann das beispielsweise in ein niederes Alkanol aufgenommene Rohprodukt unmittelbar im letzten Verfahrensschritt (Abspaltung des Alkohols $R^4OH$) der vorliegenden Erfindung umgesetzt werden, und zwar im Rahmen eines "Durchprozesses" II'/II'' + III $\rightarrow$ IV'/IV'' $\rightarrow$ I'/I''.

Der letzte Verfahrensschritt wird zweckmässigerweise durchgeführt, indem man die in einem geeigneten organischen Lösungsmittel gelöste Verbindung der Formel IV'/IV'' stark basischen Bedingungen unterwirft, d.h. in Gegenwart einer Base unter Abspaltung des Alkohols $R^4OH$ zum(zur) entsprechenden Polyenester bzw. -säure umsetzt. Als organische Lösungsmittel eignen sich im allgemeinen polare oder unpolare, wie beispielsweise Alkohole, aliphatische oder cyclische Ether, z.B. Diethylether und Tetrahydrofuran, und aliphatische Ester, z.B. Ethylacetat; bzw. Aromaten, z.B. Toluol, aliphatische Kohlenwasserstoffe, z.B. n-Hexan, und niedere halogenierte aliphatische Kohlenwasserstoffe, z.B. Methylenchlorid, Chloroform und Tetrachlorkohlenstoff; oder auch Gemische eines Alkohols mit einem anderen hier erwähnten Lösungsmittel. Falls ein Alkanol der Formel $R^3OH$, worin $R^3$ verschieden von $R^7$ (als $C_{1-6}$-Alkyl, 2-Hydroxyethyl oder 3-Hydroxy-n-propyl) der Verbindung der Formel IV'/IV'' ist, als Lösungsmittel verwendet wird, weist in Folge der gegenüber diesem Zwischenprodukt überschüssigen Menge am Alkohol $R^3OH$ das Endprodukt der Formel I' bzw. I'' die diesbezügliche Estergruppe $-COOR^3$ auf: auf diese (ganz einfache) Weise, d.h. durch Umesterung, wird die Estergruppe $-COOR^7$ in die sich davon unterscheidende Estergruppe $-COOR^3$ übergeführt. Ansonsten wird eine separate Behandlung, d.h. Umesterung des isolierten Produktes, das die Estergruppe $-COOR^7$ aufweist, vorgenommen, um die Gruppe $-COOR^7$ in die erwünschte, sich davon unterscheidende Estergruppe $-COOR^3$ überzuführen, und zwar auf an sich bekannte Weise.

Die verwendete Base kann anorganisch oder organisch sein, und es eignen sich im allgemeinen starke Basen, wie beispielsweise Alkalimetallalkoholate, insbesondere Natriummethylat, Natriumethylat, Kaliummethylat, Kaliumethylat und Kalium-tert.butylat, sowie Alkalimetallhydride, insbesondere Natriumhydrid.

Man verwendet zweckmässigerweise mindestens ein Aequivalent Base pro Aequivalent der Verbindung der Formel IV' oder IV'', vorzugsweise etwa 1,5 bis etwa 2 Aequivalente. Die Umsetzung erfolgt geeigneterweise im Temperaturbereich von etwa 0°C bis etwa 100°C, vorzugsweise bei Temperaturen von etwa Raumtemperatur bis etwa 50°C. Ausserdem erfolgt die Umsetzung zweckmässigerweise bei Normaldruck, wobei im allgemeinen der Druck nicht kritisch ist.

Es hat sich als besonders vorteilhaft erwiesen, den letzten Verfahrensschritt unter Verwendung eines Natriumalkoxids als Base und des entsprechenden Alkanols als Lösungsmittel bei Temperaturen zwischen der Raumtemperatur und der Rückflusstemperatur des jeweiligen Reaktionsgemisches, vorzugsweise im Temperaturbereich von etwa 40°C bis etwa 60°C, durchzuführen. Dabei wird zweckmässigerweise im voraus entweder eine Lösung des Natriumalkoxids im Alkanol bereitgestellt, oder man stellt diese Lösung frisch aus metallischem Natrium und dem Alkanol her. Das Zusammenbringen der alkanolischen Lösung des Natriumalkoxids mit der vorzugsweise ebenfalls im voraus vorbereiteten Lösung der Verbindung der Formel IV'/IV'' in (dem gleichen) Alkanol kann in beliebiger Reihenfolge und

vorzugsweise bei Raumtemperatur erfolgen. Man rührt dann das Reaktionsgemisch anschliessend mehrere Stunden, und die Reaktion ist normalerweise spätestens nach 24 Stunden beendet.

Ungeachtet der gewählten Prozedur des letzten Verfahrensschrittes kann das Produkt in an sich bekannter Weise vom Reaktionsgemisch isoliert und gereinigt werden. Die jeweilige Aufarbeitung beinhaltet normalerweise das Neutralisieren der verbleibenden Base, und zwar durch Zugabe einer organischen oder anorganischen Säure, wie beispielsweise einer Carbonsäure, z.B. Essigsäure, bzw. einer wässrigen Mineralsäure, z.B. verdünnte Schwefelsäure.

Im besonderen Fall der oben beschriebenen Prozedur mit dem Natriumalkoxid als Base wird nach Beendigung der Reaktion das Gemisch zweckmässigerweise auf Raumtemperatur oder sogar bis auf etwa 0°C abgekühlt und danach vorzugsweise mit wässriger Essigsäure neutralisiert, was normalerweise zur (eventuell weiteren) Auskristallisation des Produktes der Formel I' oder I'' führt. Die Auskristallisation kann auch noch durch weiteres Abkühlen gefördert werden. Nach dessen Isolierung, geeigneterweise durch Abfiltration, kann das Produkt gewaschen, beispielsweise mit Wasser und/oder wässrigem Alkohol und schliesslich gegebenenfalls unter vermindertem Druck getrocknet werden. Gewünschtenfalls können weitere Methoden, wie beispielsweise Säulenchromatographie und Umkristallisation, eingesetzt werden, um zu einem noch reineren Produkt zu gelangen.

Die Abspaltung des Alkohols $R^4$OH von der Verbindung der Formel IV'/IV'' könnte auch unter sauren Bedingungen erfolgen, um als Alternative zum unter stark basischen Bedingungen erfolgenden letzten Verfahrensschritt des erfindungsgemässen Verfahrens zu dienen. Nach diesbezüglichen Versuchen mit katalytischen Mengen einer starken Säure, z.B. p-Toluolsulfonsäure, diese Abspaltung zu bewirken, wurde festgestellt, dass die Abspaltung tatsächlich relativ leicht gelingt. Beispielsweise erfolgte unter Einsatz von 12'-Methoxy-11',12'-dihydro-8'-apo-β-carotinsäure-ethylester mit einer katalytischen Menge von p-Toluolsulfonsäure in Methylenchlorid bei etwa 0°C nach etwa 30 Minuten die Abspaltung von Methanol, was rohen 8'-Apo-β-carotinsäure-ethylester in praktisch quantitativer Gewichtsausbeute ergab. Gemäss HPLC bestand das Rohprodukt zu etwa 71,5% aus dem (all E)-Isomeren sowie aus mehreren weiteren [vermutlich (Z-)]Isomeren. Der absolute Gehalt des Rohproduktes betrug gemäss HPLC jedoch nur etwa 37,5%. Möglicherweise erfolgt unter solchen stark sauren Bedingungen eine teilweise Polymerisierung des Polyensystems. Auch weitere versuchte Optimierungen dieser säurekatalytischen Abspaltung erbrachten keine besseren Ergebnisse. Ueberraschenderweise wurde festgestellt, dass die unter basischen Bedingungen erfolgende Abspaltung fast ausschliesslich zu den gewünschten (all E)-Isomeren führt, wobei praktisch keine (Z)- und weiteren unerwünschten Isomeren gebildet werden.

Je nach eingesetzter Verbindung der Formel IV' oder IV'' und eingesetztem Lösungsmittel (alkoholischem oder anderem Lösungsmittel) erhält man nach Durchführung des letzten Verfahrensschrittes das in bezug auf den Rest $R^3$ entsprechende Produkt der Formel I' bzw. I'' (nachfolgend als I'/I'' abgekürzt). Falls das Lösungsmittel kein Alkohol ist (so dass keine Umesterung stattfinden kann), erhält man:

- nach Einsatz einer Verbindung der Formel IV'/IV'' in der $R^7$ $C_{1-6}$ Alkyl bedeutet, einen Polyenester/-diester der Formel I'/I'', in der $R^3$ das entsprechende $C_{1-6}$-Alkyl bedeutet;
- nach Einsatz einer Verbindung der Formel IV'/IV'', in der $R^7$ Wasserstoff bedeutet, eine Polyensäure/-disäure der Formel I'/I'', in der $R^3$ Wasserstoff bedeutet; und
- nach Einsatz einer Verbindung der Formel IV'/IV'', in der $R^7$ 2-Hydroxyethyl oder 3-Hydroxy-n-propyl bedeutet, einen Polyenester/-diester der Formel I'/I'', in der $R^3$ nicht die bisher angegebene Bedeutung besitzt, sondern 2-Hydroxyethyl bzw. 3-Hydroxy-n-propyl bedeutet.

In jedem der obigen drei Fälle bleibt dann im Verlauf des letzten Verfahrensschrittes der Rest $R^7$ unverändert.

Falls hingegen ein Alkohol, insbesondere ein $C_{1-6}$-Alkanol, als Lösungsmittel verwendet wird, findet beim Einsatz der drei Typen von Verbindungen IV'/IV'' in zwei Fällen eine Umesterung (im ersten und dritten Fall) statt, wie dies bereits oben angedeutet ist. Auf diese Weise wird jeweils ein Polyenester der Formel I'/I'' gebildet, worin $R^3$ dem diesbezüglichen Alkylrest des Alkanols entspricht: die Gruppe -COOR$^7$ der Verbindung IV'/IV'' wird dann ohne besondere Massnahmen in die sich davon unterscheidende Gruppe -COOR$^3$ übergeführt, es sei denn, das alkoholische Lösungsmittel entspricht dem Alkohol $R^7$OH. Die Verwendung des diesbezüglichen Alkanols $R^3$OH ist aus diesem Grunde und auch deswegen, weil das in Alkohol schwer lösliche Produkt der Formel I schon während der Reaktion aus der Lösung auskristallisiert, bevorzugt.

Im erhaltenen Produkt der Formel I' oder I'' gegebenenfalls vorhandene Schutzgruppen ($R^8$ und/oder $R^9$ als geschützte Hydroxy- oder Oxogruppe) können gewünschtenfalls nach an sich bekannten Methoden, z.B. durch Hydrolyse mit Säure oder Base, abgespalten werden.

Während einige der Edukte des erfindungsgemässen Verfahrens bekannt sind, sind andere aus zum Teil bekannten Vorstufen nach an sich bekannten Methoden herstellbar.

So können beispielsweise die Polyen-O,O-dialkylacetale der Formel II' und die Polyen-di(O,O-dialkylacetale) der Formel II'' sehr einfach auf bekannte allgemeine Weise durch Umsetzung des Polyen-monoaldehyds der Formel A-CHO bzw. Polyen-dialdehyds der Formel OHC-B-CHO mit dem diesbezüglichen Orthoameisensäure-trialkylester

hergestellt werden, insbesondere in dem entsprechenden $C_{1-6}$-Alkanol, z.B. Methanol für das O,O-Dimethylacetal, und in Gegenwart einer katalytischen Menge einer organischen Säure oder einer Lewissäure, z.B. p-Toluolsulfonsäure bzw. Zinkchlorid [siehe beispielsweise Organikum, Organisch-chemisches Grundpraktikum, 6. Auflage, S. 377 ff. (1963)]. Die Reaktion verläuft in Suspension, d.h. der jeweilige Polyen-monoaldehyd oder -dialdehyd wird im Alkanol suspendiert, und dann werden zweckmässigerweise etwa zwei bzw. vier Moläquivalente des Orthoameisensäure-trialkylesters zur Suspension gegeben, gefolgt von einer Spur sauren Katalysators, z.B. p-Toluolsulfonsäure. Dabei löst sich der Mono- oder Dialdehyd langsam auf, und das gebildete Polyen-O,O-dialkylacetal bzw. -di(O,O-dialkylacetal) der Formel II'/II" kristallisiert gleichzeitig langsam aus. Die Umsetzung wird zweckmässigerweise im Temperaturbereich von etwa 0°C bis etwa 40°C durchgeführt und dauert in der Regel von etwa 2 bis etwa 4 Stunden. Als weitere Literaturstellen, welche die allgemein bekannte Acetalisierungsmethode veranschaulichen, wird auf die Europäischen Patentpublikationen 252 389 und 391 033 sowie auf J. Mol. Cat. 79, 117 ff. (1993) verwiesen.

Die Polyen-monoaldehyde A-CHO und -dialdehyde OHC-B-CHO ihrerseits sind entweder bekannt, insbesondere aus der Fachliteratur betreffend Carotinoide, oder - falls neu - können nach an sich bekannten einschlägigen Methoden hergestellt werden. So sind beispielsweise die Umsetzung von verschiedenen $C_{15}$-Wittigsalzen mit 2,7-Dimethyl-2,4,6-octatriendial (dem sogenannten "$C_{10}$-Dialdehyd") zu den entsprechenden Monoaldehyden, die Umsetzung von verschiedenen $C_5$-Wittigaldehyden mit langkettigen Polyenaldehyden ebenfalls zu solchen Monoaldehyden sowie die zweifache Umsetzung des $C_{10}$-Dialdehyds mit $C_5$- oder $C_{10}$-Wittigaldehyden zu verschiedenen Dialdehyden aus dieser Literatur bekannt geworden. Das Lehrbuch "Carotenoids" (O. Isler, Birkhäuser Verlag Basel und Stuttgart, 1971), besonders dessen Kapitel VI und XII und die darin erwähnte weitere Literatur, liefert viele nützliche Hinweise auf die Herstellung und das Vorkommen der bekannten Mono- und Dialdehyde. Falls Edukte eingesetzt werden, welche geschützte Hydroxy-, Oxo- bzw. Formylgruppen aufweisen, so können solche "geschützte" Edukte beispielsweise unmittelbar aus den entsprechenden ungeschützten nach an sich bekannten Methoden hergestellt werden.

Die Vinylketenacetale oder Analoge davon der Formel III sind zum Teil bekannte Verbindungen; der grösste Teil dieser Edukte ist allerdings neu.

Gewisse Verbindungen der Formel III, in der $R^5$ und $R^6$ unabhängig voneinander $C_{1-6}$-Alkyl bedeuten, sind bekannt: das sind insbesondere 1,1-Dimethoxy-1,3-butadien und 1,1-Diethoxy-3-methyl-1,3-butadien (der Formel III, worin $R^1$ und $R^2$ beide Wasserstoff und $R^5$ und $R^6$ beide Methyl, bzw. $R^1$ Methyl, $R^2$ Wasserstoff und $R^5$ und $R^6$ beide Ethyl, bedeuten). Die restlichen Verbindungen dieser Unterklasse sind wohl neu. Alle derartigen Verbindungen können gemäss dem nachfolgenden Reaktionsschema hergestellt werden, und zwar ausgehend von einem bekannten oder nach an sich bekannten Methoden herstellbaren Nitril der allgemeinen Formel VI:

Reaktionsschema 1

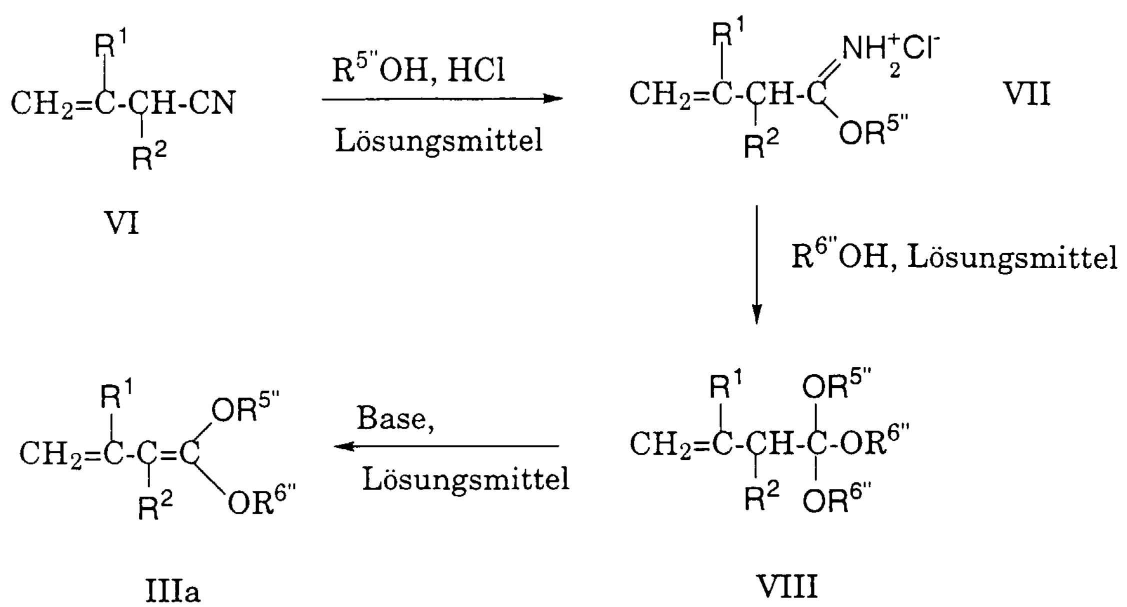


worin $R^1$ und $R^2$ die oben angegebenen Bedeutungen besitzen und $R^{5''}$ und $R^{6''}$ unabhängig voneinander $C_{1-6}$-Alkyl bedeuten.

Die Umsetzung des Nitrils der allgemeinen Formel VI mit dem Alkanol $R^{5''}OH$ in Gegenwart von gasförmigem

Chlorwasserstoff ergibt den entsprechenden, in den meisten Fällen in kristalliner Form vorliegenden Iminoalkylester der allgemeinen Formel VII. Dazu wird als Lösungsmittel geeigneterweise ein aprotisches Lösungsmittel, insbesondere ein niederer aliphatischer Kohlenwasserstoff, z.B. n-Pentan oder n-Hexan; ein niederer halogenierter aliphatischer Kohlenwasserstoff, z.B. Methylenchlorid oder Chloroform; oder ein Aromat, z.B. Benzol oder Toluol, verwendet. Sowohl das Alkanol als auch der Chlorwasserstoff wird zweckmässigerweise im leichten Ueberschuss verwendet, und zwar bis etwa 50%ig; vorzugsweise bis etwa 10 bzw. 20%ig (Aequivalente Alkanol bzw. Chlorwasserstoff gegenüber Aequivalenten Nitril). Die Umsetzung erfolgt geeigneterweise im Temperaturbereich von etwa -20°C bis etwa +60°C, vorzugsweise im Temperaturbereich von etwa 0°C bis zur Raumtemperatur.

Die weitere Umsetzung des Iminoalkylesters mit dem gewünschtenfalls sich vom Alkanol $R^{5''}OH$ unterscheidenden Alkanol $R^{6''}OH$ zum Orthoester der allgemeinen Formel VIII erfolgt zweckmässigerweise in einem aprotischen Lösungsmittel, z.B. einem der obenerwähnten, und bei Temperaturen im Bereich von etwa 0°C bis etwa 60°C, vorzugsweise bei Raumtemperatur.

Mittels Natriumamid in flüssigem Ammoniak als Base bzw. Lösungsmittel lässt sich aus dem Orthoester der Formel VIII ein Aequivalent Alkanol $R^{6''}OH$ eliminieren, wobei das gewünschte Vinylketenacetal der Formel IIIa sehr leicht erhalten wird. Auch weitere Basen/Lösungsmittel-Kombinationen können zu diesem Zweck verwendet werden, insbesondere ein Alkalimetallamid (im allgemeinen) oder -alkyl, z.B. Butyllithium, jeweils in einem polaren oder apolaren aprotischen Lösungsmittel, wie beispielsweise einem niederen aliphatischen Kohlenwasserstoff, z.B. n-Hexan, einem niederen aliphatischen Ether, z.B. Diethylether, oder einem Aromat, z.B. Benzol oder Toluol.

Aus denjenigen Vinylketenacetal-Analogen der Formel III, worin $R^5$ $C_{1-6}$-Alkyl und $R^6$ Tri($C_{1-6}$-alkyl)silyl bedeuten, ist zumindest 1-Trimethylsilyloxy-1-ethoxy-2-methyl-1,3-butadien (der Formel III, worin $R^1$ Wasserstoff, $R^2$ Methyl, $R^5$ Ethyl und $R^6$ Trimethylsilyl bedeuten) bekannt [siehe u.a. Tetr. Lett. 20, 3209 ff. (1979), Chimia 34, 265 ff. (1980) und Tetr. Lett. 26, 397 ff. (1985)]. Die neuen derartigen Analoge können beispielsweise aus dem entsprechenden 3-Butensäure-alkylester durch Umsetzung mit Lithiumdiisopropylamid und dem entsprechenden Trialkylsilylchlorid bei tiefen Temperaturen, z.B. etwa -70°C, hergestellt werden [siehe insbesondere Tetrahedron 40, 3455 ff. (1984)]. Die genannten 3-Butensäure-alkylester (Ausgangsmaterialien) sind ihrerseits bekannt oder nach an sich bekannten Methoden herstellbar; 2-Methyl-3-butensäure-ethylester kann beispielsweise durch Ethanolyse von 2-Methyl-3-butennitril (der Formel VI, worin $R^1$ Wasserstoff und $R^2$ Methyl bedeuten), einem Abfallprodukt bei der Adiponitrilsynthese, hergestellt werden (Pinner-Reaktion; siehe Deutsche Offenlegungsschrift 3.244.273 sowie US-Patentschrift 4.937.308).

Eine weitere Unterklasse der Vinylketenacetale bzw. Analoge davon der Formel III enthält ebenfalls bekannte Verbindungen, z.B. 1,1-Di(tri-methylsilyloxy)-2-methyl-1,3-butadien [der Formel III, worin $R^1$ Wasserstoff, $R^2$ Methyl und $R^5$ und $R^6$ beide Trimethylsilyl bedeuten; siehe J.A.C.S. 110, 5841 ff. (1988)]. Insgesamt besteht diese UnterKlasse aus Vinylketenacetal-Analogen der Formel III, worin $R^5$ und $R^6$ beide Tri($C_{1-6}$-alkyl)silyl bedeuten. Das obenerwähnte bekannte Analog kann beispielsweise ausgehend vom Trimethylsilylester der 2-Methyl-3-butensäure hergestellt werden durch Deprotonierung mittels Lithiumdiisopropylamid bei etwa -75°C und anschliessende Umsetzung mit Trimethylsilylchlorid gemäss den in J. Organomet. Chem. 338, 149 ff. (1988) angegebenen Bedingungen. Die weiteren derartigen Analoge können analog hergestellt werden.

Die verbleibenden Vinylketenacetale der Formel III sind wohl mit drei Ausnahmen neue Verbindungen. Bei den Ausnahmen handelt es sich um 2-Alkyliden-[1,3]dioxolan, 2-(1-Methyl-allyliden)-[1,3]dioxolan sowie 2-(2-Methylallyliden)-[1,3]dioxolan (der Formel III, worin $R^1$ und $R^2$ beide Wasserstoff, $R^1$ Wasserstoff und $R^2$ Methyl, bzw. $R^1$ Methyl und $R^2$ Wasserstoff bedeuten und $R^5$ und $R^6$ jeweils zusammen 1,2-Ethylen bilden). Die Synthese der zweitgenannten Verbindung ist in Tetrahedron 50, 5109-5118 (1994) publiziert; das Acetal wurde in mg Mengen ausgehend von Tiglinsäure hergestellt, jedoch nicht isoliert, sondern nur anhand der $^1$H-NMR-Spektroskopie (Röhrchen) nachgewiesen. Weitere relevante Literaturstellen betreffend die drei bekannten Vinylketenacetale sind J. Chem. Soc., Perkin Trans. 1(5), 1582-4 (1981) und Macromolecules 28(12), 4319-4325 (1995). Im allgemeinen können die Vinylketenacetale der diesbezüglichen Unterklasse, d.h. der Formel III, worin $R^5$ und $R^6$ zusammen 1,2-Ethylen oder 1,3-Trimethylen bilden, gemäss dem nachfolgenden Reaktionsschema hergestellt werden (wie im Falle des Reaktionsschemas 1 ausgehend vom Nitril der Formel VI):

Reaktionsschema 2

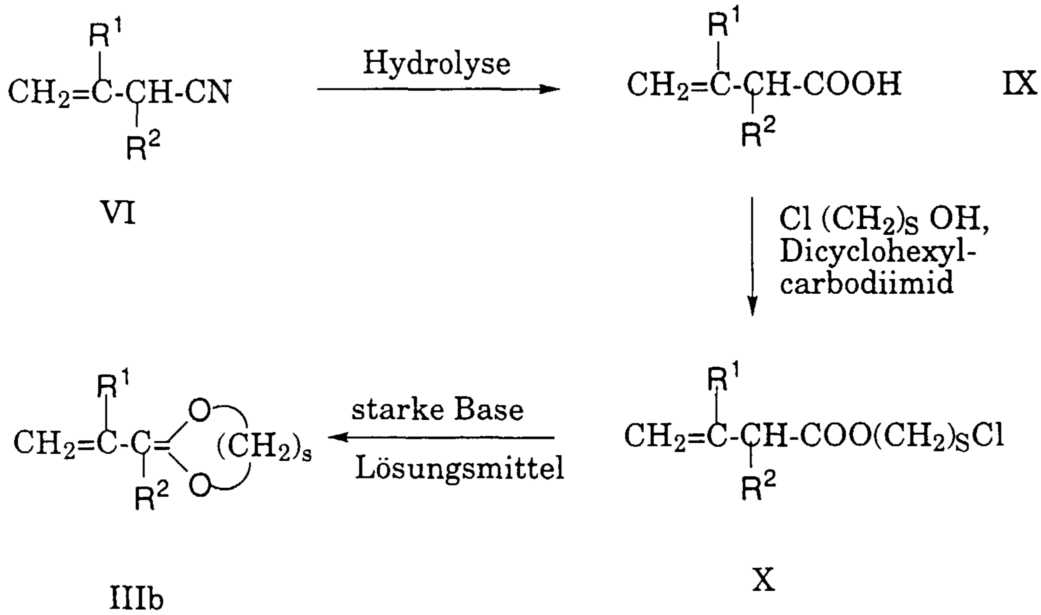


VI IX X IIIb

worin $R^1$ und $R^2$ die oben angegebenen Bedeutungen besitzen und s 2 oder 3 bedeutet.

Die Hydrolyse des Nitrils der allgemeinen Formel VI zur entsprechenden Carbonsäure der allgemeinen Formel IX und die anschliessende Veresterung dieser Carbonsäure zum Ester der allgemeinen Formel X können jeweils in an sich bekannter Weise durchgeführt werden [siehe beispielsweise Organikum, Organisch-chemisches Grundpraktikum, 6. Auflage, S. 411 ff. (1967) bzw. J. March, Advanced Organic Chemistry, 3. Auflage, S. 349 ff. (1989)]. Dann kann die Cyclisierung des Esters zum gewünschten [1,3]Dioxolan oder [1,3]Dioxan zweckmässigerweise erfolgen in einem aprotischen polaren oder unpolaren Lösungsmittel, insbesondere einem niederen aliphatischen Kohlenwasserstoff, z.B. n-Hexan, einem niederen aliphatischen Ether, z.B. Diethylether, oder einem Aromat, z.B. Toluol, in Gegenwart einer starken Base, insbesondere eines Alkalimetallalkyls, z.B. Methyl- oder Butyllithium, eines Alkalimetallhydrids, z. B. Natrium- oder Kaliumhydrid, oder eines Alkalimetallamids, z.B. Lithium-, Natrium- oder Kaliumamid oder Lithiumdiisopropylamid bei Temperaturen im Bereich von etwa -70°C bis etwa +100°C, vorzugsweise im Temperaturbereich von etwa -30°C bis etwa 0°C.

Bei der oben beschriebenen Herstellung der Vinylketenacetale oder Analoge davon kann jeweils das Produkt oder ein Zwischenprodukt auf an sich bekannte Weise isoliert und gereinigt werden.

Die Zwischenprodukte des erfindungsgemässen Verfahrens, d.h. die Verbindungen der allgemeinen Formeln IV' und IV", sind neue Verbindungen und stellen einen weiteren Aspekt der vorliegenden Erfindung dar.

Unter diesen neuen Verbindungen der allgemeinen Formeln IV' und IV" befinden sich:

15-Methoxy-15,15'-dihydro-12'-β-carotinsäure-ethylester,
15-Methoxy-15,15'-dihydro-12'-apocarotinsäure-methylester,
12'-Methoxy-11',12'-dihydro-8'-apo-β-carotinsäure-ethylester,
12'-Methoxy-11',12'-dihydro-8'-apo-β-carotinsäure-methylester,
12'-Methoxy-11',12'-dihydro-8'-apo-β-carotinsäure-2-hydroxyethylester,
8'-Methoxy-7',8'-dihydro-4'-apo-β-carotinsäure-ethylester,
8'-Methoxy-7',8'-dihydro-4'-apo-β-carotinsäure-methylester,
4'-Methoxy-β,ψ-carotin-16'-säure-ethylester,
4'-Methoxy-β,ψ-carotin-16'-säure-methylester,
12'-Methoxy-11,12'-dihydro-8'-apo-β-carotin-8'-säure,
11,12,11',12'-Tetrahydro-12,12'-dimethoxy-8,8'-diapocarotin-8,8'-disäureethylester,
11,12,11',12'-Tetrahydro-12,12'-dimethoxy-8,8'-diapocarotin-8,8'-disäure-methylester,
7,8,7',8'-Tetrahydro-8,8'-dimethoxy-4,4'-diapocarotin-4,4'-disäure-ethylester und
7,8,7',8'-Tetrahydro-8,8'-dimethoxy-4,4'-diapocarotin-4,4'-disäure-methylester.

Auch gewisse der neuen, zur Herstellung der obigen Zwischenprodukte der Formeln IV' und IV" und schliesslich der Polyenester und -säuren der Formel I' und I'' verwendeten Ausgangsmaterialien (Vinylketenacetale und Analoge davon) der Formel III stellen einen weiteren Aspekt der vorliegenden Erfindung dar. Diese neuen erfindungsgemässen Ausgangsmaterialien sind die Verbindungen der bisher angegebenen allgemeinen Formel IIIa mit Ausnahme von 1,1-Dimethoxy-1,3-butadien und 1,1-Diethoxy-3-methyl-1,3-butadien, und diejenigen der ebenfalls bisher angegebenen Formel IIIb mit Ausnahme von 2-Allyliden-[1,3]dioxolan, 2-(1-Methyl-allyliden)-[1,3]dioxolan und 2-(2-Methylallyliden)-[1,3] dioxolan. Unter diesen neuen Verbindungen befinden sich:

1,1-Dimethoxy-2-methyl-1,3-butadien,
1,1-Dimethoxy-3-methyl-1,3-butadien,
1,1-Diethoxy-1,3-butadien,
1,1-Diethoxy-2-methyl-1,3-butadien,
2-(1,2-Dimethyl-allyliden)-[1,3]dioxolan und
2-(1-Methyl-allyliden)-[1,3]dioxan.

Die Endprodukte des erfindungsgemässen Verfahrens, d.h. die Polyenester und -säuren der allgemeinen Formeln I' und I'', gehören grösstenteils dem Gebiet der Carotinoide an und finden entsprechende Verwendung, beispielsweise als Farbstoffe bzw. Pigmente für Lebensmittel, den Eidotter, die Integumente (insbesondere Haut, Ständer und Schnäbel) und/oder das subkutane Fett von Geflügel, das Fleisch und/oder die Integumente (insbesondere Haut, Schuppen und Schale) von Fischen und Crustaceen usw. Diese Verwendung kann nach an sich bekannten Methoden erfolgen, wie diese beispielsweise in der europäischen Patentpublikation Nr. 630.578 beschrieben ist.

Die Verwendung der neuen Endprodukte stellt einen weiteren Aspekt der vorliegenden Erfindung dar.

Die Erfindung wird anhand der nachfolgenden Beispiele veranschaulicht:

A. Herstellung der Polyen-(di-)O,O-dialkylacetale (Verbindungen der Formeln II' und II'')

Beispiel 1

15-Apo-β-carotinal-dimethylacetal (Vitamin A-Aldehyd-dimethylacetal)

In einem mit Magnetrührer, Argonbegasung und Thermometer ausgestatteten 100 ml-Vierhals-Sulfierkolben wurden 2,84 g (10 mMol) Vitamin A-Aldehyd (>99% rein) in 30 ml Methanol und 11 ml (100 mMol, 10 Aeq.) Orthoameisensäure-trimethylester vorgelegt. Bei 0°C gab man dazu 40 mg (0,3 mMol, 3 Mol%) wasserfreies Zinkchlorid und rührte 3 1/2 Stunden bei 0°C. Dann wurde innert einer Stunde auf -40°C abgekühlt, abfiltriert, mit wenig kaltem (bei etwa -10°C) Methanol gewaschen und schliesslich getrocknet. Dies ergab 2,5 g (75%ige Ausbeute) (all-E)-Vitamin A-Aldehyd-dimethylacetal mit Smp. 53-56°C; Gehalt nach HPLC: 98,4%.

Zur Analyse wurde eine Probe aus Methanol umkristallisiert. Diese Probe wies folgende physikalische bzw. analytische Daten auf: Smp. 53-55°C; Gehalt nach HPLC: 99,7%; UV (n-Hexan): 324 nm (log ε = 4,70; $E^{1\%}_{1cm}$ = 1520).

| Mikroanalyse: | | |
|---|---|---|
| Ber. | C 79,95% | H 10,37% |
| Gef. | C 79,66% | H 10,50% |

Beispiel 2

12'-Apo-β-carotinal-dimethylacetal

In einem mit mechanischem Rührer, Argonbegasung und Thermometer ausgestatteten 1,5 l-Vierhalssulfierkolben wurden 50 g (0,14 Mol) 12'-Apo-β-carotinal und 31 ml (= 30,1 g, 0,28 Mol) frisch destillierter Orthoameisensäure-trimethylester in 500 ml Methanol vorgelegt. Bei Raumtemperatur gab man nun zur resultierenden Suspension eine Lösung von 16 mg p-Toluolsulfonsäure-Monohydrat in 4 ml Methanol. Dabei lösten sich die roten Kristalle zum grössten Teil innert 20 Minuten auf; anschliessend begann sich ein oranger Niederschlag zu bilden. Nach 2 Stunden Rühren bei Raumtemperatur wurde auf ca. +5°C abgekühlt, 0,5 ml Triethylamin zugegeben, 15 Minuten bei 0°C nachgerührt, abgenutscht (Druckfilternutsche, unter Argon), mit wenig kaltem (-10°C) Methanol gewaschen und etwa 16 Stunden unter vermindertem Druck (Wasserstrahlvakuum) bei Raumtemperatur getrocknet. Dies ergab 52,3 g (90,5%ige Ausbeute) 12'-Apo-β-carotinal-dimethylacetal als oranges Pulver mit Smp. 77-78°C; Gehalt nach HPLC: 97,5% (sehr säurelabil); UV (n-Hexan): 393 nm (log ε = 4,91; $E^{1\%}_{1cm}$ = 2045), 376 nm (log ε = 4,91; $E^{1\%}_{1cm}$ = 2045).

| Mikroanalyse: | | |
| --- | --- | --- |
| Ber. | C 81,77% | H 10,17% |
| Gef. | C 81,50% | H 9,84% |

Beispiel 3

8'-Apo-β-carotinal-dimethylacetal

In einem mit mechanischem Rührer, Argonbegasung und Thermometer ausgestatteten 350 ml-Vierhalssulfierkolben wurden 6,25 g (15 mMol) 8'-Apo-β-carotinal und 6,6 ml (= 6,4 g, 60 mMol, 4 Aeq.) Orthoameisensäuretrimethylester in 200 ml Methanol vorgelegt. Bei Raumtemperatur gab man eine Lösung von 20 mg p-Toluolsulfonsäure-Monohydrat in 10 ml Methanol dazu und liess 2 1/2 Stunden rühren. Dabei lösten sich die roten Kristalle langsam auf, und ein oranges Kristallisat begann sich zu bilden. Dann wurden 2 ml Triethylamin zugegeben, innert etwa 30 Minuten auf 0°C abgekühlt, abgenutscht, mit wenig kaltem (-10°C) Methanol gewaschen und kurz unter vermindertem Druck (Wasserstrahlvakuum) getrocknet. Nach 30 Minuten ergab dies 11,6 g methanolfeuchtes Acetal mit einem Gehalt nach HPLC von 94,8%. Zur Umkristallisation wurden die Kristalle in 200 ml Diethylether gelöst, und dann gab man innert 1 1/2 Stunden 600 ml Methanol zu, kühlte auf 0°C ab, filtrierte die Kristalle ab, und trocknete sie bei Raumtemperatur unter vermindertem Druck (Wasserstrahlvakuum) und kurz unter Hochvakuum. Dies ergab 5,9 g (84%ige Ausbeute) 8'-Apo-β-carotinal-dimethylacetal als rostrote Kristalle mit Smp. 131-132°C; Gehalt nach HPLC: 98,4%; UV (n-Hexan): 450 nm (log ε = 5,06; $E^{1\%}_{1cm}$ = 2476), 424 nm (log ε = 5,07; $E^{1\%}_{1cm}$ = 2543).

| Mikroanalyse: | | |
| --- | --- | --- |
| Ber. | C 83,06% | H 10,02% |
| Gef. | C 82,91% | H 10,13% |

Beispiel 4

4'-Apo-β-carotinal-dimethylacetal

In einem mit mechanischem Rührer, Argonbegasung und Thermometer ausgestatteten 500 ml-Vierhalssulfierkolben wurden 10 g (20,7 mMol) 4'Apo-β-carotinal und 35 ml (0,31 Mol, etwa 15 Aeq.) Orthoameisensäure-trimethylester in 250 ml Methanol vorgelegt. Zur resultierenden dunkelroten Suspension gab man bei Raumtemperatur eine Lösung von 25 mg p-Toluolsulfonsäure-Monohydrat in 15 ml Methanol zu, rührte 45 Minuten bei Raumtemperatur, dann während 1 1/2 Stunden bei 30-35°C; die dunkelrote Suspension wandelte sich in eine braune Suspension um. Anschliessend gab man 2 ml Triethylamin zu und kühlte auf 0°C ab. Das ausgefallene Rohprodukt wurde abgenutscht, mit wenig kaltem Methanol gewaschen und unter vermindertem Druck bei Raumtemperatur kurz getrocknet. Das so erhaltene methanolfeuchte Produkt (etwa 17,8 g; Gehalt nach HPLC: 91%) wurde in 600 ml Diethylether mit leichtem Erwärmen gelöst und bei Raumtemperatur innert 1 Stunde mit 1 l Methanol (enthaltend 2‰ Triethylamin) versetzt. Dann wurde abgenutscht und mit wenig kaltem (0°C) Methanol gewaschen. Dies ergab nach 2-stündiger Trocknung bei Raumtemperatur und unter vermindertem Druck 9,3 g (80,5%ige Ausbeute) 4'-Apo-β-carotinal-dimethylacetal als violette Kristalle mit einem Gehalt nach HPLC von 94,7%.

Für die analytischen Daten wurde eine Probe aus Diethylether (warm gelöst und auf 0°C abgekühlt) umkristallisiert: Gehalt nach HPLC: 95,7%; Smp. 186-188°C; UV (Dioxan): 500 nm (log ε = 4,93; $E^{1\%}_{1cm}$ = 1623), 468 nm (log ε = 4,99; $E^{1\%}_{1cm}$ = 1837), 283 nm (log ε = 4,37; $E^{1\%}_{1cm}$ = 444).

Beispiel 5

12,12'-Diapocarotinal-dimethylacetal ($C_{10}$-Dialdehyd-dimethylacetal)

In einem mit Magnetrührer und Argonbegasung ausgestatteten 500 ml-Rundkolben wurden 32,8 g (0,2 Mol) $C_{10}$-Dialdehyd und 65 g Orthoameisensäure-trimethylester in 250 ml Methanol vorgelegt. Bei etwa 20°C gab man dazu 100 mg p-Toluolsulfonsäure-monohydrat, was eine leicht exotherme Reaktion auslöste. Das Reaktionsgemisch wurde mit einem kalten Wasserbad auf etwa 20-25°C gehalten. Die Suspension löste sich in etwa 5 Minuten auf. Dann wurde eine Stunde bei Raumtemperatur nachgerührt und anschliessend etwa 0,5 ml Triethylamin zugegeben. Nun wurde unter vermindertem Druck eingeengt und der anfallende Kristallbrei in 200 ml heissem n-Hexan gelöst, durch Watte

heiss filtriert und stehen gelassen. Man liess die Lösung in einem Tiefkühlschrank etwa 16 Stunden bei -20°C stehen, filtrierte die resultierenden Kristalle ab, wusch sie mit n-Hexan bei -20°C und trocknete sie unter Wasserstrahlvakuum bis zur Gewichtskonstanz. Dies ergab 42,7 g (80%ige Ausbeute) $C_{10}$-Dialdehyd-dimethylacetal als hellgelbe, knackige Kristalle mit Smp. 68-69°C und einem Gehalt nach Gaschromatographie (GC) von etwa 96%); UV (Ethanol): 292 nm (log ε = 4,61; $E_{1cm}^{1\%}$ = 1602), 280 nm (log ε = 4,72; $E_{1cm}^{1\%}$ = 2046), 260 nm (log ε = 4,59; $E_{1cm}^{1\%}$ = 1508).

| Mikroanalyse: | | |
|---|---|---|
| Ber. | C 65,60% | H 9,44% |
| Gef. | C 65,43% | H 9,14% |

Beispiel 6

8,8'-Diapocarotinal-dimethylacetal (Crocetindialdehyd-dimethylacetal)

In einem Magnetrührer und Argonbegasung ausgestatteten 500 ml-Rundkolben wurden 20,0 g (67,5 mMol) Crocetindialdehyd (Smp. 196-197°C) und 40 g (0,37 Mol) Orthoameisensäure-trimethylester in 350 ml Methanol vorgelegt. Unter Rühren gab man bei Raumtemperatur 200 mg p-Toluolsulfonsäure-Monohydrat zu, rührte etwa 45 Minuten bei Raumtemperatur und etwa 1 1/2 Stunden bei 35-40°C, was eine gelb-orange Suspension ergab. Dann wurde auf 0°C abgekühlt, abfiltriert und mit kaltem Methanol (-10°C) gewaschen. Dies ergab 24,7 g (92%ige Ausbeute) Crocetindialdehyd-dimethylacetal als oranges Pulver, Smp. 136°C, mit einem Gehalt nach HPLC von 97,4%. Umkristallisation aus 150 ml heissem Ethylacetat und 150 ml Methanol unter Abkühlen bis auf -20°C ergab nach Filtration und Trocknung (Wasserstrahl-, gefolgt von Hochvakuum) 23,3 g (86%ige Ausbeute) Crocetindialdehyd-dimethylacetal als rostrote, knackige Kristalle, Smp. 138-139°C, mit einem Gehalt nach HPLC von 97,3%; UV (Ethanol): 422 nm (log ε = 5,12; $E_{1cm}^{1\%}$ = 3416), 397 nm (log ε = 5,11; $E_{1cm}^{1\%}$ = 3305), 377 nm (log ε = 4,89; $E_{1cm}^{1\%}$ = 1985), 232 nm (log ε = 4,20; $E_{1cm}^{1\%}$ = 405).

| Mikroanalyse: | | |
|---|---|---|
| Ber. | C 74,19% | H 9,34% |
| Gef. | C 74,10% | H 9,47% |

B. Herstellung der Vinylketenacetale oder Analoge davon (Verbindungen der Formel III)

Beispiel 7

1,1-Dimethoxy-2-methyl-1,3-butadien [drei Stufen a), b) und c)]

a) 2-Methyl-3-buteniminosäure-methylesterhydrochlorid

In einem mit mechanischem Rührer, Thermometer und Gaseinleitungsrohr (für Argonbegasung) ausgestatteten 6 l-Vierhalssulfierkolben wurden 173 g (220 ml,5,4 Mol, 1,1 Aeq.) Methanol in 2,5 l Toluol und 2,5 l n-Hexan vorgelegt. Bei 0°C (Badtemperatur -10°C) und unter Rühren wurden innert 1 1/2 Stunden 216 g (5,92 Mol, 1,2 Aeq.) gasförmiger Chlorwasserstoff (getrocknet über konzentrierter Schwefelsäure) eingeleitet. Dann gab man innert 45 Minuten bei zwischen 0 und 5°C 500 g (4,9 Mol, 1 Aeq.) 80%iges 2-Methyl-3-butennitril zu, ersetzte das Eisbad mit einem kühlen (20°C) Wasserbad und liess etwa 20 Stunden bei Raumtemperatur weiterrühren. Der anfallende Niederschlag wurde 1 Stunde abgekühlt (Eisbad), abgenutscht, mit 1 l n-Hexan gewaschen und 18 Stunden bei Raumtemperatur unter vermindertem Druck (Wasserstrahlvakuum) getrocknet. Dies ergab 594 g (81%ige Ausbeute) 2-Methyl-3-buteniminosäure-methylesterhydrochlorid mit Smp. 106°C (unter Zersetzung).

b) 2-Methyl-3-butensäure-orthomethylester

In einer Kutscher-Steudel-Apparatur (2,5 l-Inhalt, 1 l-Rundkolben für Pentanvorlage) wurden 297 g (2 Mol) 2-Methyl-3-buteniminosäure-methylesterhydrochlorid in 1 l Methanol bei Raumtemperatur gelöst. Diese Lösung wurde nun kontinuierlich mit 2 l n-Pentan über etwa 24 Stunden extrahiert, wobei die Methanolphase magnetisch gerührt wurde. Dabei fiel Ammoniumchlorid aus. Anschliessend wurde das Pentan im Rundkolben (etwa 600-700 ml) eingeengt. Dies ergab 234g rohen 2-Methyl-3-butensäure-orthomethylester mit einem Gehalt nach GC von 66%.

Eine wiederholte Durchführung dieser Herstellung ergab im selben Massstab 236 g rohes Produkt mit einem

Gehalt nach GC von 70%. Die beiden Rohprodukte wurden vereinigt (zu 470 g) und an einer Vigreux-Kolonne (20 cm, verspiegelt) bei 30 mbar fraktioniert. Bei einer Siedetemperatur von 65-66°C/30 mbar wurden 310 g (43%ige Ausbeute) 2-Methyl-3-butensäure-orthomethylester mit einem Gehalt nach GC von 89,5% erhalten.

c) 1,1-Dimethoxy-2-methyl-1,3-butadien

In einem mit mechanischem Rührer, Thermometer, Tropftrichter und Argonbegasung ausgestatteten 1,5 l-Vierhalssulfierkolben wurden bei -70°C etwa 600 ml flüssiges Ammoniak unter Verwendung eines Steigrohrs kondensiert. Zum Kondensat gab man 1 Spatelspitze Eisen(III)-nitrat zu. Danach wurden innert 30 Minuten 21 g (0,91 Mol, 3,2 Aeq.) Natrium-Metall zugegeben und bei -45 bis -65°C gerührt, was eine dunkelgraue Suspension ergab. Danach wurde bei -45 bis -40°C innert 1 Stunde eine Lösung von 50,8 g (0,284 Mol) 2-Methyl-3-butensäure-orthomethylester mit einem Gehalt nach GC von 89,5% in 200 ml Diethylether zugetropft und anschliessend bei -45°C/1 Stunde nachgerührt. Danach wurde die Kühlung entfernt und innert 2 Stunden das Ammoniak abgedampft (Wasserbad/20°C). Nach Zugabe von 200 ml Diethylether wurden zwischen 0 und 20°C vorsichtig 100 ml Wasser zugetropft, die Wasserphase abgetrennt und noch zweimal mit je 150 ml, total 300 ml, n-Pentan extrahiert. Die vereinigten Auszüge wurden nach Zugabe von 40 mg 2,6-Di-tert.butyl-p-kresol über wasserfreiem Natriumsulfat getrocknet und vorsichtig bei 25-30°C unter vermindertem Druck eingeengt. Dies ergab 37,3 g rohes 1,1-Dimethoxy-2-methyl-1,3-butadien mit einem Gehalt nach GC von 95%. Eine Destillation an einer 10 cm Vigreux-Kolonne bei 20 mbar ergab bei einer Siedetemperatur von 48°C 34,2 g (90,2%ige Ausbeute) 1,1-Dimethoxy-2-methyl-1,3-butadien mit einem Gehalt nach GC von 96% als farblose Flüssigkeit, welche nach Zugabe von 35 mg (1‰) 2,6-Di-tert.butyl-p-kresol bei 0°C unter Argon gelagert wurde.

Beispiel 8

(E/Z)-1-Trimethylsilyloxy-1-ethoxy-2-methyl-1,3-butadien

In einem mit mechanischem Rührer, Thermometer, zwei Tropftrichtern und Argonbegasung ausgestatteten 500 ml-Vierhalssulfierkolben wurden 25,6 ml (181 mMol, 1,1 Aeq.) Diisopropylamin in 160 ml Tetrahydrofuran (über $LiAlH_4$ destilliert) vorgelegt. Dazu tropfte man bei -25°C innert 45 Minuten 113 ml (181 mMol, 1,1 Aeq.) einer Butyllithiumlösung (1,6M in n-Hexan) zu, kühlte dann auf -70°C ab und tropfte innert 30 Minuten eine Lösung von 21,2 g (165 mMol) 2-Methyl-3-butensäure-ethylester [siehe Deutsche Offenlegungsschrift 3.244.273 sowie US Patentschrift 4.937.308] mit einem Gehalt nach GC von 100% in 30 ml absolutem Tetrahydrofuran (über $LiAlH_4$ destilliert) zu. Dann wurde bei -70°C 20 Minuten nachgerührt, und man tropfte anschliessend bei dieser Temperatur 25,1 ml (= 21,6 g, 0,199 Mol, 1,2 Aeq.) Trimethylchlorsilan zu. Das Kühlbad wurde nun entfernt, so dass die Temperatur innert etwa 2 1/2 Stunden von -70°C auf Raumtemperatur anstieg. Dann wurde über Celite® (ein aus Kieselgur bestehendes Filterhilfsmittel; Manville Corp., USA) abgenutscht, mit Tetrahydrofuran nachgewaschen, wenig (ca. 100 mg) 2,6-Di-tert. butyl-p-kresol zugegeben und unter vermindertem Druck bei 40°C vorsichtig eingeengt. Der Rückstand wurde in n-Pentan aufgenommen und die Lösung filtriert und eingeengt. Eine Destillation unter Hochvakuum (0,25 mbar) ergab bei einer Siedetemperatur von 28°C (unter gutem Kühlen) 25,8 g (73%ige Ausbeute) (E/Z)-1-Trimethylsilyloxy-1-ethoxy-2-methyl-1,3-butadien mit einem Gehalt nach GC von 94%.

Beispiel 9

2-(1-Methyl-allyliden)-[1,3]dioxolan [zwei Stufen a) und b)]

a) 2-Methyl-but-3-ensäure-2-chlorethylester

45,1 g (0,45 Mol) 2-Methyl-3-butensäure, 45,33 g (0,56 Mol) 2-Chlorethanol und 4,1 g (34 mMol) Dimethylaminopyridin wurden in 450 ml Diethylether unter Argon vorgelegt. Dazu gab man bei 0°C innert 30 Minuten in 5 Portionen 102,2 g (0,495 Mol) N,N-Dicyclohexylcarbodiimid zu, rührte 2 Stunden bei Raumtemperatur weiter und filtrierte den ausgefallenen Harnstoff ab. Das Filtrat wusch man der Reihe nach mit 200 ml 0,5N wässriger Salzsäure, 100 ml gesättigter Natriumbicarbonat-Lösung und 100 ml gesättigter Natriumchlorid-Lösung. Man trocknete die organische Lösung über wasserfreiem Natriumsulfat und destillierte das nach dem Einengen erhaltene Produkt an einer Vigreux-Kolonne (20 cm) bei 80-81°C/10 mbar. Dies ergab 62,3 g (83%ige Ausbeute) 2-Methyl-but-3-ensäure-2-chlorethylester als farbloses Oel mit einem Gehalt nach GC von 97,5%.

b) 2-(1-Methyl-allyliden)-[1,3]dioxolan

Unter Argon wurden 90,6 g (etwa 113 ml) 20%iges Kaliumhydrid in Oel (Dichte etwa 0,8) zweimal mit n-Hexan gewaschen, dann mit 700 ml Dimethoxyethan versetzt. Anschliessend gab man dazu unter Rühren bei 20°C eine Lösung von 56,5 g (0,34 Mol) 2-Methyl-but-3-ensäure-2-chlorethylester mit einem Gehalt nach GC von 97,5% in 175 ml Dimethoxyethan innert 1 1/2 Stunden zu und rührte 1 Stunde bei Raumtemperatur nach. Dann gab man vorsichtig bei 0-5°C tropfenweise 250 ml Wasser zu. Man trennte dann die wässrige Phase ab und extrahierte sie dreimal mit je 300 ml, total 900 ml, n-Hexan. Waschen der vereinigten organischen Phasen mit 250 ml gesättigter Natriumchlorid-Lösung, Trocknen und Einengen ergab eine gelbe Flüssigkeit (50,5 g; GC: 83,4%ig), welche nach Zugabe von 40 mg 2,6-Di-tert.butyl-p-kresol an einer Vigreux-Kolonne (20 cm) fraktioniert wurde. Dies ergab bei einer Siedetemperatur von 93-94°C/19 mbar 28,22 g (66%ige Ausbeute) 2-(1-Methyl-allyliden)-[1,3]dioxolan als farbloses Oel mit einem Gehalt nach GC von 100%.

C. Herstellung der Verbindungen der Formeln IV' und IV'' aus den Polyen-(di-)O,O-dialkylacetalen der Formel II' bzw. II'' und den Vinylketenacetalen oder Analogen davon der Formel III

Beispiel 10

15-Methoxy-15,15'-dihydro-12'-β-carotinsäure-ethylester

In einem mit Thermometer, Magnetrührer und Argonbegasung ausgestatteten 50 ml-Vierhalssulfierkolben wurden 1,75 g (5 mMol) Vitamin A-Aldehyd-dimethylacetal (HPLC: 98%ig) und 1,5 g (6,5 mMol) (E/Z)-1-Trimethylsilyoxy-1-ethoxy-2-methyl-1,3-butadien (GC: 87%ig) in 20 ml tert.Butylmethylether vorgelegt. Bei -30°C gab man innert 20 Minuten 7 Tropfen (= etwa 80 mg, 10 Mol%) Bortrifluoridetherat dazu. Nach 1 Stunde [DC ($SiO_2$): $R_f$ = etwa 0,3, Cyclohexan/Ethylacetat (9:1)] wurde bei -30°C 1 ml Triethylamin zugegeben, auf Raumtemperatur erwärmt und auf 20 ml Wasser gegossen. Die abgetrennte Wasserphase wurde nochmals zweimal mit je 20 ml, total 40 ml, n-Hexan extrahiert, und die vereinigten organischen Phasen wurden mit 20 ml gesättigter Kochsalzlösung gewaschen, über wasserfreiem Natriumsulfat getrocknet und eingeengt. Eine chromatographische Reinigung des gelben, öligen Rohproduktes (2,7 g) an 100 g Kieselgel (0,04-0,063 mm) mit Cyclohexan/Ethylacetat (9:1) ergab 1,68 g (74%ige Ausbeute) 15-Methoxy-15,15'-dihydro-12'-β-carotinsäure-ethylester als dickes gelbes Oel (Gehalt nach HPLC: 97%); UV (n-Hexan): 325 nm (log ε = 4,63; $E_{1cm}^{1\%}$ = 989).

Beispiel 11

15-Methoxy-15,15'-dihydro-12'-apocarotinsäure-methylester

In einem 50 ml-Kolben wurden unter Argon 1,75 g (5 mMol) Vitamin A-Aldehyd-dimethylacetal (HPLC: 98%ig), 0,8 g (6,5 mMol, 1,3 Aeq.) 1,1-Dimethoxy-2-methyl-1,3-butadien (GC: 99%ig) in 20 ml tert.Butylmethylether vorgelegt. Unter Rühren bei -30°C gab man 5 Tropfen (= etwa 60 mg, 8 Mol%) Bortrifluoridetherat dazu und rührte weiter bei ca. -30°C (anfangs tiefdunkel gefärbte Lösung, welche nach etwa 10 Minuten zu einer orangen Lösung aufhellte: DC ($SiO_2$): $R_f$ = etwa 0,3, n-Hexan/Ethylacetat = 9:1).

Zur Hydrolyse wurden dann bei -30°C 5 ml Essigsäure/Wasser (9:1) zugegeben und bei 0°C 20 Minuten gerührt. Dann wurden 20 ml Wasser zugegeben, zweimal mit je 20 ml, total 40 ml, n-Hexan extrahiert und die vereinigten organischen Phasen je einmal mit 20 ml gesättigter Natriumbicarbonat-Lösung und Natriumchlorid-Lösung gewaschen, über wasserfreiem Natriumsulfat getrocknet und unter vermindertem Druck eingeengt. Das Rohprodukt (2,5 g) wurde an 125 g Kieselgel (0,040-0,063 mm) mit n-Hexan/Ethylacetat (9:1) chromatographiert. Dies ergab 1,50 g (69%ige Ausbeute) 15-Methoxy-15,15'-dihydro-12'-apocarotinsäure-methylester als gelbes Oel; Gehalt nach HPLC: 95,2%; UV (n-Hexan): 325 nm (log ε = 4,67; $E_{1cm}^{1\%}$ = 1132).

| Mikroanalyse: | | |
|---|---|---|
| Ber. | C 78,60% | H 9,77% |
| Gef. | C 78,26% | H 10,08% |

Bei dieser Chromatographie konnten in einer Vorfraktion 130 mg (etwa 6%) (13-cis)-15-Methoxy-15,15'-dihydro-12'-apocarotinsäure-methylester als gelbes Oel isoliert werden; Gehalt nach HPLC: 95,3%; UV (n-Hexan): 329 nm (log ε =4,58; $E_{1cm}^{1\%}$ = 926).

Beispiel 12

12'-Methoxy-11',12'-dihydro-8'-apo-β-carotinsäure-ethylester

In einem 10 ml-Rundkolben wurden unter Argon 495 mg (1,25 mMol) 12'-Apo-β-carotinal-dimethylacetal und 325 mg (1,5 mMol) (E/Z)-1-Trimethylsilyloxy-1-ethoxy-2-methyl-1,3-butadien (GC: 92%) in 5 ml tert.Butylmethylether vorgelegt. Bei 0°C gab man dazu 17 mg (10 Mol%) wasserfreies Zinkchlorid und rührte 2 Stunden bei +5°C [DC($SiO_2$): $R_f$ = etwa 0,2; Toluol]. Das orangerote Reaktionsgemisch wurde auf 20 ml Wasser gegossen und zweimal mit je 20 ml, total 40 ml, n-Hexan extrahiert, über wasserfreiem Natriumsulfat getrocknet und unter vermindertem Druck eingeengt. Das Rohprodukt (760 mg) wurde an 30 g Kieselgel (0,040-0,063 mm) mit Toluol chromatographiert. Dies ergab 609 mg (93%ige Ausbeute) (all-E)-12'-Methoxy-11',12'-dihydro-8'-apo-β-carotinsäure-ethylester als dickflüssiges oranges Oel; Gehalt nach HPLC: 94%. Die spektroskopischen Daten entstammen aus einem analogen Ansatz: UV (n-Hexan): 395 nm (log ε = 4,81; $E_{1cm}^{1\%}$ = 1300), 376 nm (log ε = 4,82; $E_{1cm}^{1\%}$ = 1339).

| Mikroanalyse: | | |
|---|---|---|
| Ber. | C 80,44% | H 9,82% |
| Gef. | C 80,49% | H 10,15% |

Beispiel 13

12'-Methoxy-11',12'-dihydro-8'-apo-β-carotinsäure-methylester

In einem 100 ml-Rundkolben wurden unter Argon 6,2 g (15 mMol) 12'-Apo-β-carotinal-dimethylacetal und 3,1 g (24 mMol, 1,6 Aeq.) 1,1-Dimethoxy-2-methyl-1,3-butadien (GC: 100%) in 60 ml tert.Butylmethylether bei -25°C vorgelegt. Bei -25°C gab man dazu unter Rühren 80 mg (etwa 6 Tropfen, 4 Mol%) Bortrifluorid-etherat und rührte 1 Stunde bei -25°C [DC($SiO_2$): $R_f$ = etwa 0,25; Toluol]. Dann gab man dazu 15 ml Essigsäure/Wasser (9:1) und liess bei Raumtemperatur 30 Minuten rühren. Dann wurde auf 100 ml tert.Butylmethylether gegossen, viermal mit je 100 ml, total 400 ml, Wasser gewaschen und die wässrigen Phasen mit 100 ml tert.Butylmethylether extrahiert. Nachdem die vereinigten organischen Phasen mit je 100 ml gesättigter Natriumbicarbonat-Lösung und 100 ml gesättigter Natriumchlorid-Lösung gewaschen worden waren, trocknete man sie über wasserfreiem Natriumsulfat, filtrierte ab und dampfte unter vermindertem Druck ein. Das anfallende dunkle Oel (9,2 g) wurde an 250 g Kieselgel (0,040-0,063 mm) mit Toluol/Ethylacetat (19:1) chromatographiert. Dies ergab 6,9 g (89%ige Ausbeute) 12'-Methoxy-11',12'-dihydro-8'-apo-β-carotinsäure-methylester als rotoranges, viskoses Oel mit einem Gehalt nach HPLC von 93,1%. UV (Cyclohexan mit 3% Chloroform): 398 nm (log ε = 4,77; $E_{1cm}^{1\%}$ = 1225), 380 nm (log ε = 4,78; $E_{1cm}^{1\%}$ = 1257).

| Mikroanalyse: | | |
|---|---|---|
| Ber. | C 80,29% | H 9,69% |
| Gef. | C 80,45% | H 9,53% |

Beispiel 14

12'-Methoxy-11',12'-dihydro-8'-apo-β-carotinsäure-2-hydroxyethylester

In einem 100 ml-Rundkolben wurden unter Argonbegasung 2,98 g (7,3 mMol) 12'-Apo-β-carotinal-dimethylacetal (Gehalt nach HPLC: etwa 97%) und 1,42 g (11,25 mMol) 2-(1-Methyl-allyliden)-[1,3]dioxolan (GC: 100%) in 30 ml tert.Butylmethylether vorgelegt. Bei -25°C gab man dazu unter Rühren 2 Tropfen (etwa 25 mg, 2 Mol%) Bortrifluoridetherat und rührte 1 Stunde bei dieser Temperatur nach. Dann gab man 8 ml Essigsäure/Wasser (9:1) dazu, entfernte die Kühlung und liess in 30 Minuten auf Raumtemperatur erwärmen [$R_f$ = etwa 0,4, n-Hexan/Ethylacetat 1:1]. Dann goss man das Reaktionsgemisch auf 100 ml tert.Butylmethylether, wusch zweimal mit je 150 ml Wasser, einmal mit 100 ml gesättigter Natriumbicarbonat-Lösung und einmal mit 50 ml gesättigter Natriumchlorid-Lösung. Anschliessend wurde die organische Phase über wasserfreiem Natriumsulfat getrocknet, filtriert, eingeengt und das Rohprodukt (4,1 g) an 120 g Kieselgel (0,040-0,063 mm) mit n-Hexan/Ethylacetat (1:1) chromatographiert. Dabei wurden nach Kristallisation aus n-Hexan 1,85 g (49%) 12'-Methoxy-11',12'-dihydro-8'-apo-β-carotinsäure-2-hydroxyethylester als hellgelbe Kristalle, Smp. 98-100°C, mit einem Gehalt nach HPLC von 97,6% erhalten. UV (n-Hexan): 395 nm (log ε =4,92; $E_{1cm}^{1\%}$ = 1634), 377 nm (log ε = 4,93; $E_{1cm}^{1\%}$ = 1669).

Aus der Mutterlauge der Kristallisation konnte nach Chromatographie an Silicagel (Eluierungsmittel: Toluol/Ethylacetat = 8:2) ein harziges Nebenprodukt (65 mg) vermutlich mit folgender Struktur (nach $^1$H-NMR und Massenspektrum) isoliert werden:

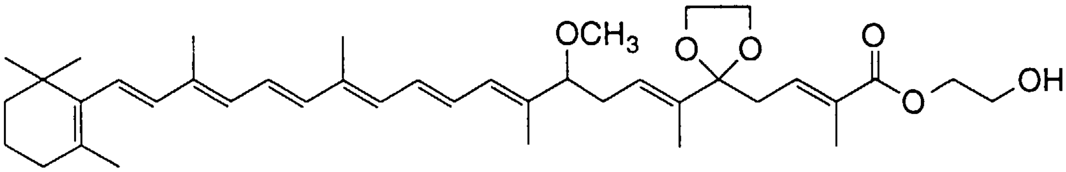

$C_{40}H_{48}O_6$ (634,90)

Beispiel 15

8'-Methoxy-7',8'-dihydro-4'-apo-β-carotinsäure-ethylester

In einem 250 ml-Rundkolben wurden unter Argonbegasung 4,73 g (10 mMol) 8'-Apo-β-carotinal-dimethylacetal (Gehalt nach HPLC: 98%) und 2,56 g (12 mMol, 1,2 Aeq.) (E/Z)-1-Trimethylsilyloxy-1-ethoxy-2-methyl-1,3-butadien (GC: 94%) in 100 ml tert.Butylmethylether vorgelegt. Bei 0°C gab man 66 mg (0,5 mMol, 5 Mol%) wasserfreies Zinkchlorid dazu und rührte 3 Stunden bei Raumtemperatur. [DC($SiO_2$): $R_f$ = etwa 0,2; Toluol]. Dann wurden 50 ml Wasser zugegeben, welches nach Abtrennung nochmals mit 50 ml n-Hexan extrahiert wurde. Nach Trocknung über wasserfreiem Natriumsulfat, Filtration und Abdampfen des Lösungsmittels wurde das anfallende rote Oel (5 g) in 100 ml Ethanol heiss gelöst und langsam auf 0°C abgekühlt. Die anfallenden Kristalle wurden abgenutscht, mit wenig kaltem Ethanol gewaschen und unter Wasserstrahlvakuum bei Raumtemperatur getrocknet. Dies ergab 3,8 g (65%ige Ausbeute) 8'-Methoxy-7',8'-dihydro-4'-apo-β-carotinsäure-ethylester als rote Kristalle mit Smp. 107-108°C; Gehalt nach HPLC: 96,4%; UV (n-Hexan): 451 nm (log ε = 5,04; $E^{1\%}_{1cm}$ = 1955), 425 nm (log ε= 5,08; $E^{1\%}_{1cm}$ = 2146).

Beispiel 16

8'-Methoxy-7',8'-dihydro-4'-apo-β-carotinsäure-methylester

In einem 50 ml-Rundkolben wurden unter Argonbegasung 4,80 g (10 mMol) 8'-Apo-β-carotinal-dimethylacetal (Gehalt nach HPLC: 97%) und 2,20 g (17 mMol) 1,1-Dimethoxy-2-methyl-1,3-butadien (GC: 99%) in 100 ml tert.Butylmethylether vorgelegt. Bei 0°C gab man unter Rühren einen Tropfen (= 12 mg), dann nach 30 Minuten nochmals einen Tropfen, total zwei Tropfen (24 mg, etwa 2Mol%), Bortrifluoridetherat dazu und liess insgesamt 1 Stunde bei 0°C rühren.

Zur Hydrolyse gab man zum Gemisch bei 0°C 20 ml Essigsäure/Wasser (9:1), rührte etwa 50 Minuten bei Raumtemperatur [DC($SiO_2$): $R_f$ = etwa 0,2; Toluol]. Dann wurde die Lösung in einen Scheidetrichter gegeben, zweimal mit je 100 ml, total 200 ml, Wasser und 100 ml gesättigter Natriumbicarbonat-Lösung gewaschen. Die wässrige Phasen wurden jeweils mit 100 ml n-Hexan extrahiert. Trocknen über wasserfreiem Natriumsulfat, Filtration und Eindampfen unter vermindertem Druck ergab 8,6 g dickes Oel, welches in 250 ml Ethanol unter leichtem Erwärmen gelöst wurde. Nach dem Abkühlen auf 0° bis -20°C, Filtration und Trocknung der Kristalle wurden 3,0 g (53%ige Ausbeute) 8'-Methox-7',8'-dihydro-4'-apo-β-carotinsäure-methylester als orange Kristalle, Smp. 84-86°C, mit einem Gehalt nach HPLC von 96,2% erhalten. UV (Cyclohexan mit 3% Chloroform): 456 nm (log ε = 4,77; $E^{1\%}_{1cm}$ = 1087), 430 nm (log ε = 4,82; $E^{1\%}_{1cm}$ = 1225), 407 nm (log ε = 4,67; $E^{1\%}_{1cm}$ = 855).

Beispiel 17

Isolierung/Charakterisierung des als Zwischenprodukt angenommenen Orthoesters 8'-Methoxy-7',8'-dihydro-4'-apo-β-carotinsäure-orthomethylester (vgl. Beispiel 16: Säurekatalysierte Hydrolyse ausgelassen)

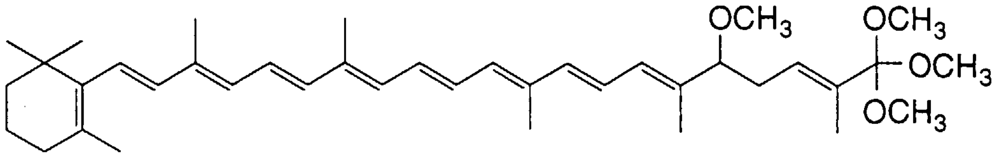

$C_{39}H_{58}O_4$ (590,89)

In einem 150 ml-Rundkolben wurden unter Argonbegasung 4,60 g (9,6 mMol) 8'-Apo-β-carotinal-dimethylacetal (Gehalt nach HPLC: 97%) und 1,95 g (15 mMol) 1,1-Dimethoxy-2-methyl-1,3-butadien (GC: 99%) in 75 ml tert. Butylmethylether vorgelegt. Bei 0°C gab man unter der Reaktionssuspension einen Tropfen (= 12 mg, etwa 1 Mol%) Bortrifluoridetherat. Es resultierte nach kurzer Zeit eine dunkelrote Reaktionslösung, aus der orange Kristalle ausfielen. Zehnminütiges Rühren bei 0°C, Abnutschen, Waschen mit wenig kaltem Methanol/Wasser (9:1)-Gemisch und Trocknen unter Wasserstrahlvakuum und anschliessend unter Hochvakuum bei Raumtemperatur ergab 3,50 g (61%ige Ausbeute) 8'-Methoxy-7',8'-dihydro-4'-apo-β-carotinsäure-orthomethylester als orange Kristalle, Smp. 131-132°C. UV (Cyclohexan mit 3% Chloroform): 456 nm (log ε = 5,04; $E^{1\%}_{1cm}$ = 1855), 430 nm (log ε = 5,08; $E^{1\%}_{1cm}$ = 2047), 410 nm (log ε = 4,91 $E^{1\%}_{1cm}$ = 1362).

| Mikroanalyse: | | |
|---|---|---|
| Ber. | C 79,28% | H 9,89% |
| Gef. | C 79,09% | H 9,86% |

Beispiel 18

4'-Methoxy-β,ψ-carotin-16'-säure-ethylester

In einem mit Magnetrührer und Argonbegasung ausgestatteten 150 ml-Rundkolben wurden 2,91 g (5 mMol) 4'-Apo-β-carotinal-dimethylacetal (Gehalt nach HPLC: 91%) und 1,21 g (6 mMol) 1-Trimethylsilyloxy-1-ethoxy-2-methyl-1,3-butadien (GC: 99%) in 50 ml tert.Butylmethylether vorgelegt und bei 0°C mit 70 mg (0,5 mMol, 10 Mol%) wasserfreiem Zinkchlorid versetzt. Dann wurde etwa 18 Stunden bei Raumtemperatur gerührt [DC($SiO_2$): $R_f$ = etwa 0,2; Toluol], auf Wasser gegossen und wie üblich (vgl. Beispiel 10) aufgearbeitet. Dies ergab einen dunkelroten, harzigen Rückstand (5,4 g), welcher an 250 g Kieselgel (0,04-0,063 mm) mit Toluol chromatographiert wurde. Es resultierte ein Klebriges Produkt (1,9 g, Gehalt nach HPLC: etwa 70%). Dieser Rückstand wurde einmal in 40 ml Ethanol und einmal in 25 ml Ethanol heiss (50°C) digeriert, abgekühlt (0°C) und getrocknet. Dies ergab 0,86 g (26%ige Ausbeute) 4'-Methoxy-β,ψ-carotin-16'-säure-ethylester als dunkelrote Kristalle mit Smp. 124-125°C und einem Gehalt nach HPLC von 95,4%. UV (Cyclohexan mit 3% Chloroform): 496 nm (log ε = 4,98; $E^{1\%}_{1cm}$ = 1530), 464 nm (log ε = 5,05; $E^{1\%}_{1cm}$ = 1790), 439 nm (log ε = 4,91; $E^{1\%}_{1cm}$ = 1290).

Beispiel 19

4'-Methoxy-β,ψ-carotin-16'-säure-methylester

In einem mit Magnetrührer und Argonbegasung ausgestatteten 150 ml-Kolben wurden 2,91 g (5 mMol) 4'-Apo-β-carotinal-dimethylacetal (Gehalt nach HPLC: 91%) und 1,10 g (8,5 mMol) 1,1-Dimethoxy-2-methyl-1,3-butadien (GC: 98%) in 50 ml tert.Butylmethylether suspendiert. Bei 0°C gab man einen Tropfen (= 12 mg, 2 Mol%) Bortrifluoridetherat dazu und rührte anschliessend 1 Stunde bei Raumtemperatur. Dann wurden 20 ml Essigsäure/Wasser (9:1) zugegeben und 2 Stunden bei Raumtemperatur gerührt [DC($SiO_2$): $R_f$ = etwa 0,2; Toluol]. Uebliche Aufarbeitung (vgl.

Beispiel 11) und Chromatographie des Rückstandes an 250 g Kieselgel (0,04-0,063 mm) mit Toluol ergab einen Klebrigen, dunkelroten Rückstand (2,2 g), welcher in 60 ml Ethanol heiss (50°C) digeriert wurde. Nach Abkühlung auf 0°C, Filtration und Trocknung wurden 0,90 g (28%ige Ausbeute) 4'-Methoxy-β,ψ-carotin-16'-säure-methylester als dunkelrote Kristalle mit Smp. 120-123°C und einem Gehalt nach HPLC von 98,0% erhalten. UV (Cyclohexan mit 3% Chloroform): 469 nm (log ε = 5,10; $E^{1\%}_{1cm}$ = 2058), 465 nm (log ε = 5,16; $E^{1\%}_{1cm}$ = 2220), 440 nm (log ε = 4,99; $E^{1\%}_{1cm}$ = 1610).

Beispiel 20

12'-Methoxy-11,12'-dihydro-8'-apo-β-carotin-8'-säure

In einem mit einem Magnetrührer ausgestatteten 100 ml-Rundkolben wurden unter Argonbegasung 2,97 g (7,3 mMol) 12'-Apo-β-carotinal-dimethylacetal (Gehalt nach HPLC: etwa 97%) und 2,63 g (9,7 mMol) 1,1-Bis-(trimethylsilyloxy)-2-methyl-1,3-butadien (Gehalt nach GC: 90,5%) in 30 ml tert.Butylmethylether vorgelegt. Bei 0°C gab man dazu 100 mg (0,7 mMol, 10 Mol%) wasserfreies Zinkchlorid und rührte 5 Stunden bei dieser Temperatur weiter. Dann wurde die Reaktionslösung auf Wasser gegossen, was die sofortige Hydrolyse des Zwischenproduktes der Formel V' auslöste, und wie üblich extrahiert (vgl. Beispiel 12). Dies ergab ein rotes Harz (4,5 g), welches an 200 g Kieselgel (0,04-0,063 mm) mit Toluol/Ethylacetat (3:1) chromatographiert wurde. Es resultierten 1,90 g (50%ige Ausbeute) 12'-Methoxy-11,12'-dihydro-8'-apo-β-carotin-8'-säure als Klebriger, orangeroter Schaum (Gehalt nach Methylierung mit Diazomethan, gemäss HPLC: 88,6%); UV (Cyclohexan mit 5% Chloroform): 398 nm (log ε = 4,71; $E^{1\%}_{1cm}$ = 1100), 379 nm (log ε = 4,72; $E^{1\%}_{1cm}$ =1340).

Beispiel 21

11,12,11',12'-Tetrahydro-12,12'-dimethoxy-8,8'-diapocarotin-8,8'-disäure-ethylester

In einem mit einem Thermometer ausgestatteten 100 ml-Zweihalsrundkolben wurden unter Argonbegasung 3,85 g (14,4 mMol) 12,12'-Diapocarotinal-dimethylacetal (Smp. 68-69°C; GC: 96%) und 7,84 g (36 mMol) 1-Trimethylsilyloxy-1-ethoxy-2-methyl-1,3-butadien (Gehalt nach GC: 94%) in 60 ml tert.Butylmethylether vorgelegt. Bei 0°C gab man dazu 100 mg (0,7 mMol, 5 Mol%) wasserfreies Zinkchlorid und rührte etwa 16 Stunden bei Raumtemperatur [DC ($SiO_2$): $R_f$ = etwa 0,2-0,3; n-Hexan/Ethylacetat (4:1)]. Zum gelblichen Reaktionsgemisch gab man 200 mg 2,6-Di-tert. butyl-p-kresol, gefolgt von 25 ml Wasser, rührte 5 Minuten bei Raumtemperatur, trennte die Wasserphase ab und extrahierte sie zweimal mit 50 ml, total 100 ml, tert.Butylmethylether. Die vereinigten organischen Phasen wurden mit je 50 ml gesättigter Natriumbicarbonat-Lösung und 50 ml gesättigter Natriumchlorid-Lösung gewaschen, über wasserfreiem Natriumsulfat getrocknet und unter vermindertem Druck eingeengt. Das anfallende Oel (etwa 10 g) wurde an 250 g Kieselgel (0,04-0,063 mm) mit n-Hexan/Ethylacetat (4:1) chromatographiert. Dies ergab 6,28 g (93%ige Ausbeute) 11,12,11',12'-Tetrahydro-12,12'-dimethoxy-8,8'-diapocarotin-8,8'-disäure-ethylester als gelbes Oel (Isomerengemisch: Gemisch zweier diastereomerer Enantiomerenpaare; Gehalt nach HPLC: 96,1%); UV (Ethanol): 298 nm (log ε = 4,61; $E^{1\%}_{1cm}$ = 904), 289 nm (log ε = 4,72; $E^{1\%}_{1cm}$ = 1170), 276 nm ( log ε = 4,59; $E^{1\%}_{1cm}$ = 876).

| Mikroanalyse: | | |
|---|---|---|
| Ber. | C 69,61% | H 8,99% |
| Gef. | C 69,79% | H 8,84% |

Beispiel 22

11,12,11',12'-Tetrahydro-12,12'-dimethoxy-8,8'-diapocarotin-8,8'-disäure-methylester

In einem mit einem Thermometer ausgestatteten 100 ml-Zweihalsrundkolben wurden 3,85 g (14,4 mMol) 12,12'-Diapocarotinal-dimethylacetal (Smp. 68-69°C; GC: 96%) und 5,37 g (40 mMol, 2,7 Aeq.) 1,1-Dimethoxy-2-methyl-1,3-butadien (Gehalt nach GC: 94,5%) in 75 ml tert.Butylmethylether vorgelegt. Bei -20°C gab man dazu innert einer Stunde zweimal 6 Tropfen, total 12 Tropfen (etwa 140 mg, 7 Mol%), Bortrifluoridetherat und rührte 2 Stunden bei -20°C [DC($SiO_2$): $R_f$= etwa 0,2; n-Hexan/Ethylacetat (4:1)]. Dann gab man 20 ml Essigsäure/Wasser (9:1) dazu, rührte 20 Minuten bei 0°C, goss auf 50 ml Wasser und arbeitete analog dem Beispiel 21 auf. Das anfallende gelbe Oel (7,1 g) wurde an 250 g Kieselgel (0,04-0,063 mm) mit n-Hexan/Ethylacetat (4:1) chromatographiert. Dies ergab 5,15 g (85%ige Ausbeute) 11,12,11',12'-Tetrahydro-12,12'-dimethoxy-8,8'-diapocarotin-8,8'-disäure-methylester (Gemisch zweier diastereomerer Enantiomerenpaare) als gelbes Oel; Gehalt nach HPLC: 99,6%. Zur Analyse untersuchte man eine aus n-Hexan kristallisierte Probe: Smp. 68-71°C; Gehalt nach HPLC: 99,7%; UV (Chloroform): 301 nm (log

$\varepsilon$ = 4,58; $E^{1\%}_{1cm}$ = 901), 289 nm (log $\varepsilon$ = 4,69; $E^{1\%}_{1cm}$ = 1157).

| Mikroanalyse: | | |
|---|---|---|
| Ber. | C 68,55% | H 8,63% |
| Gef. | C 68,18% | H 8,58% |

Beispiel 23

7,8,7',8'-Tetrahydro-8,8'-dimethoxy-4,4'-diapocarotin-4,4'-disäure-ethylester

In einem 150 ml-Rundkolben wurden unter Argonbegasung 2,54 g (7,5 mMol) Crocetindialdehyd-dimethylacetal (Gehalt nach HPLC: 97,3%) und 4,79 g (22,5 mMol, 3 Aeq.) 1-Trimethylsilyloxy-1-ethoxy-2-methyl-1,3-butadien (GC: 94%) in 60 ml tert.Butylmethylether vorgelegt. Bei 0°C gab man 105 mg (0,75 mMol, 10 Mol%) wasserfreies Zinkchlorid dazu und liess etwa 16 Stunden bei Raumtemperatur rühren [DC($SiO_2$): $R_f$ = etwa 0,3; Cyclohexan/Ethylacetat (4:1)]. Die Aufarbeitung erfolgte analog dem Beispiel 21 und ergab nach dem Abdampfen des Lösungsmittels 3,83 g rohen 7,8,7',8'-Tetrahydro-8,8'-dimethoxy-4,4'-diapocarotin-4,4'-disäure-ethylester (Gemisch zweier diastereomerer Enantiomerenpaare) als oranges Oel. Zur Reinigung wurde dieses Rohprodukt in 10 ml heissem Methanol gelöst und etwa 16 Stunden bei 0°C auskristallisiert. Dies ergab 2,20 g (49,4%ige Ausbeute) des Produktes (als Gemisch zweier diastereomerer Enantiomerenpaare) als oranges kristallines Pulver, Smp. 61-65°C; Gehalt nach HPLC: 97,8%. Die Analyse erfolgte an einem analog hergestellten chromatographierten und kristallisierten Produkt mit Smp. 63-80°C; Gehalt nach HPLC: 98,4%; UV (Cyclohexan mit 3% Chloroform): 428 nm (log $\varepsilon$ = 5,02; $E^{1\%}_{1cm}$ = 1811), 402 nm (log $\varepsilon$ = 5,01; $E^{1\%}_{1cm}$ = 1779), 381 nm (log $\varepsilon$ = 4,79; $E^{1\%}_{1cm}$ = 1074).

Beispiel 24

7,8,7',8',-Tetrahydro-8,8'-dimethoxy-4,4'-diapocarotin-4,4'-disäure-methylester

In einem mit einem Thermometer ausgestatteten 100 ml-Kolben wurden unter Argonbegasung 2,91 g (8,4 mMol) Crocetindialdehyd-dimethylacetal (Gehalt nach HPLC: 97,3%) und 3,92 g (30 mMol, 3 Aeq.) 1,1-Dimethoxy-2-methyl-1,3-butadien (GC: 98%) in 60 ml tert.Butylmethylether vorgelegt. Bei 0°C gab man tropfenweise im Abstand von 10 Minuten viermal je 1 Tropfen, total 4 Tropfen (etwa 50 mg, 4 Mol%), Bortrifluoridetherat zu. Nach weiteren 30 Minuten bei 0°C wurden nochmals 2 Tropfen (etwa 25 mg, 2 Mol%) Bortrifluorid-etherat zugegeben. Nach einer weiteren Stunde bei 0°C gab man 20 ml Essigsäure/Wasser (9:1) zu, rührte 20 Minuten bei 0°C, goss auf 50 ml Wasser und arbeitete analog dem Beispiel 21 auf [DC($SiO_2$): $R_f$ = etwa 0,3 (Produkt); $R_f$ = etwa 0,25 (Edukt); Cyclohexan/Ethylacetat (4:1)]. Dies ergab 6,5 g Rohprodukt, welches an 200 g Kieselgel (0,04-0,063 mm) mit n-Hexan/Ethylacetat (4:1) chromatographiert wurde. Es resultierten 3,05 g 7,8,7',8'-Tetrahydro-8,8'-dimethoxy-4,4'-diapocarotin-4,4'-disäure-methylester als oranger Feststoff, Smp. 73-77°C; Gehalt nach HPLC: 84,7%. Zur weiteren Reinigung wurde aus 15 ml heissem Ethanol nach Abkühlen auf 0° bis -20°C umkristallisiert. Dies ergab 2,4 g (51%ige Ausbeute) 7,8,7',8'-Tetrahydro-8,8'-dimethoxy-4,4'-diapocarotin-4,4'-disäure-methylester (Gemisch zweier diastereomerer Enantiomerenpaare) als orange Kristalle, Smp. 83-84°C; Gehalt nach HPLC: 98,7%; UV (Cyclohexan mit 3% Chloroform): 428 nm (log $\varepsilon$ = 5,05; $E^{1\%}_{1cm}$ = 2045), 402 nm (log $\varepsilon$ = 5,04; $E^{1\%}_{1cm}$ = 1996), 381 nm (log $\varepsilon$ = 4,82; $E^{1\%}_{1cm}$ = 1209).

Beispiel 25

Isolierung/Charakterisierung des als Zwischenprodukt auftretenden Bis-orthoesters 7,8,7',8'-Tetrahydro-8,8'-dimethoxy-4,4'-diapocarotin-4,4'-disäure-orthomethylester (vgl. Beispiel 24: säurekatalysierte Hydrolyse ausgelassen)

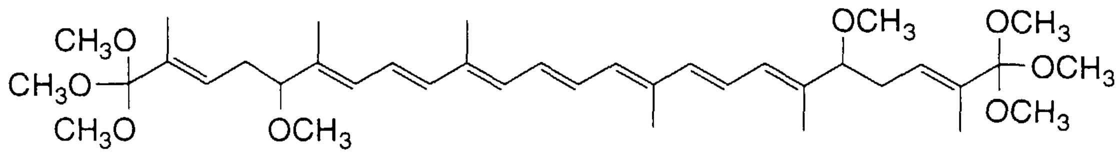

$C_{38}H_{60}O_8$ (644,89)

In einem 100 ml-Rundkolben wurden unter Argonbegasung 1,94 g (5,6 mMol) Crocetindialdehyd-dimethylacetal (Gehalt nach HPLC: 97,3%) und 2,0 g (15,5 mMol) 1,1-Dimethoxy-2-methyl-1,3-butadien (GC: 99,5%) in 40 ml tert.Butylmethylether bei 0°C mit 140 mg (1 mMol) wasserfreiem Zinkchlorid versetzt und etwa 16 Stunden bei Raumtemperatur gerührt [DC($SiO_2$): $R_f$ = etwa 0,15 (Produkt); $R_f$= 0,25 (Edukt); Cyclohexan/Ethylacetat (4:1)]. Zur Aufarbeitung wurde auf Wasser gegossen und mit Ethylacetat extrahiert. Dies ergab nach dem Eindampfen unter vermindertem Druck ein Rohprodukt (4,8 g), welches in n-Hexan/Ethylacetat (6:1) gelöst wurde. Nach dem Abkühlen auf 0°C fiel ein gelbes Produkt aus, welches abfiltriert (0,9 g) und nochmals in 45 ml warmem Methanol gelöst und auf 0° bis -20°C abgekühlt wurde. Dies ergab 0,59 g (etwa 16%ige Ausbeute) 7,8,7',8'-Tetrahydro-8,8'-dimethoxy-4,4'-diapocarotin-4,4'-disäure-orthomethylester als gelbe Kristalle, Smp. 143-147°C. Gehalt nach HPLC: 96,8%; UV (Cyclohexan mit 3% Chloroform): 428 nm (log ε = 5,09, $E_{1cm}^{1\%}$ = 1920), 402 nm (log ε = 5,08; $E_{1cm}^{1\%}$ = 1864), 381 nm (log ε = 4,86; $E_{1cm}^{1\%}$ = 1116).

| Mikroanalyse: | | |
|---|---|---|
| Ber. | C 70,77% | H 9,38% |
| Gef. | C 70,84% | H 8,98% |

D. Herstellung der Polyenester oder -säuren der Formeln I' und I'' aus den Verbindungen der Formel IV' bzw. IV''

Beispiel 26

12'-Apo-β-carotinsäure-ethylester

In einem 150 ml-Rundkolben wurden unter Argonbegasung 3,80 g (8,75 mMol) 15-Methoxy-15,15'-dihydro-12'-apocarotinsäure-methylester in 15 ml Ethanol vorgelegt. Bei Raumtemperatur gab man eine durch Lösen von 0,64 g (27 mMol; 3 Aeq.) Natrium in 35 ml Ethanol hergestellte Natriumethylatlösung zu und rührte 2 Stunden bei 40°C, was eine dunkelbraune Lösung ergab [DC($SiO_2$): $R_f$ = etwa 0,5; Cyclohexan/Ethylacetat (9:1)]. Dann wurden bei Raumtemperatur 1,2 ml (1,3 g, 21 mMol) Essigsäure zugegeben, woraus eine gelbe Suspension resultierte, und auf -40°C abgekühlt, 1 1/2 Stunden gerührt und der Festkörper abgenutscht. Dieser wurde einmal mit 20 ml Methanol bei -20°C, zweimal mit je 50 ml, total 100 ml, Wasser bei Raumtemperatur und noch einmal mit 50 ml Methanol bei -20°C gewaschen und schliesslich bei 30°C unter Wasserstrahlvakuum und dann bei Raumtemperatur unter Hochvakuum getrocknet. Dies ergab 2,30 g (63%ige Ausbeute) 12'-Apo-β-carotinsäure-ethylester als oranges Pulver, Smp. 80-81°C; Gehalt nach HPLC: 95%; UV (n-Hexan): 398 nm (log ε = 4,90; $E_{1cm}^{1\%}$ = 2028).

| Mikroanalyse: | | |
|---|---|---|
| Ber. | C 82,18% | H 9,71% |
| Gef. | C 82,03% | H 9,75% |

Beispiel 27

12'-Apo-β-carotinsäure-methylester

In einem 100 ml-Rundkolben wurden unter Argonbegasung 1,36 g (3,25 mMol) 15-Methoxy-15,15'-dihydro-12'-apocarotinsäure-methylester in 20 ml Methanol vorgelegt. Bei Raumtemperatur gab man 10 ml (10 mMol) einer 1-molaren Lösung von Natriummethylat in Methanol zu und rührte 3 Stunden bei 50°C, was eine dunkle Reaktionslösung ergab [DC($SiO_2$): $R_f$ = etwa 0,4-0,5; n-Hexan/Ethylacetat (9:1)]. Dann wurde auf 0°C abgekühlt, 1,2 g (20 mMol) Essigsäure zugegeben, gefolgt von 20 ml Wasser, woraus eine orange klebrige Ausfällung resultierte. Die Suspension wusch man mit 75 ml Ethylacetat in einen mit 50 ml Wasser beschichteten Scheidetrichter. Man trennte die Wasserphase ab und extrahierte nochmals mit 75 ml Ethylacetat. Nach Trocknung über wasserfreiem Natriumsulfat wurde die organische Phase unter vermindertem Druck eingeengt und der Rückstand (1,8 g) an 50 g Kieselgel (0,04-0,063 mm) mit n-Hexan/Ethylacetat (14:1) chromatographiert. Dies ergab 1,3 g oranges Oel, welches in 10 ml Ethanol gelöst und etwa 16 Stunden bei 0° bis -20°C kristallisiert wurde. Es resultierte 0,55 g (44% Ausbeute) 12'-Apo-β-carotinsäure-methylester als rote Kristalle, Smp. 73-83°C; Gehalt nach HPLC: 98%. UV (n-Hexan): 398 nm (log ε = 4,90; $E^{1\%}_{1cm}$ = 2100).

| Mikroanalyse: | | |
|---|---|---|
| Ber. | C 82,06% | H 9,54% |
| Gef. | C 82,15% | H 9,61% |

Beispiel 28

8'-Apo-β-carotinsäure-ethylester (Durchprozess aus 12'-Apo-β-carotinal-dimethylacetal und 1-Trimethylsilyloxy-1-ethoxy-2-methyl-1,3-butadien über 12'-Methoxy-11',12'-dihydro-8'-apo-β-carotinsäure-ethylester)

In einem mit Magnetrührer und Argonbegasung ausgestatteten 300 ml-Rundkolben wurde eine Lösung von 12,40 g (30 mMol) 12'-Apo-β-carotinal-dimethylacetal (Smp. 78-79°C; HPLC: 96,5%) und 7,6 g (36 mMol, 1,2 Aeq.) 1-Trimethylsilyloxy-1-ethoxy-2-methyl-1,3-butadien (Gehalt nach GC: 95%) in 120 ml tert.Butylmethylether bei 0°C mit 80 mg (0,6 mMol, 2 Mol%) wasserfreiem Zinkchlorid versetzt und etwa 16 Stunden bei Raumtemperatur gerührt [DC ($SiO_2$): $R_f$ (Acetal) = etwa 0,3; $R_f$ (Produkt) = etwa 0,2; Toluol]. Dann wurden 200 mg 2,6-Di-tert.butyl-p-kresol zugegeben und auf 50 ml Wasser gegeben. Nach Abtrennung der Wasserphase wurde nochmals mit 50 ml tert.Butylmethylether extrahiert, mit je 50 ml gesättigter Natriumbicarbonat-Lösung und gesättigter Natriumchlorid-Lösung gewaschen, über wasserfreiem Natriumsulfat getrocknet, abfiltriert und unter vermindertem Druck eingeengt. Der ölige Rückstand wurde zur Entfernung von restlichem organischem Lösungsmittel und Wasser in 200 ml absolutem Ethanol gelöst und unter vermindertem Druck eingeengt. Dies ergab 17,4 g rohen 12'-Methoxy-11',12'-dihydro-8'-apo-β-carotinsäure-ethylester als rotoranges Klebriges, ethanolfeuchtes Oel (Gehalt nach HPLC: 89,7%).

Dieses Oel wurde mit 250 ml absolutem Ethanol in einen mit mechanischem Rührer, Argonbegasung, Thermometer und Tropftrichter ausgestatteten 500 ml-Vierhalssulfierkolben gegeben. Bei Raumtemperatur gab man eine durch Lösen von 1,40 g (60 mMol, 2 Aeq.) Natrium in 70 ml absolutem Ethanol hergestellte Natriumethylatlösung zu und liess etwa 16 Stunden bei Raumtemperatur rühren. Nach etwa 1-2 Stunden begann sich ein Niederschlag zu bilden. Zur Vervollständigung der Reaktion wurde der dicke Niederschlag 4 Stunden bei 40°C nachgerührt [DC($SiO_2$): $R_f$ = etwa 0,4; nur noch Spuren von Vorprodukt; Toluol]. Dann wurde auf 25°C abgekühlt und tropfenweise eine Lösung von 4,2 g (70 mMol) Essigsäure in 10 ml Ethanol, gefolgt von einem Gemisch von 40 ml Ethanol und 42 ml Wasser innert etwa einer Stunde zugegeben. Man kühlte anschliessend mit einem Eisbad auf +5°C, filtrierte das Gemisch, wusch das Filtergut bei 0°C mit 50 ml Ethanol/Wasser (9:1) und bei Raumtemperatur dreimal mit je 50 ml, total 150 ml, Wasser und trocknete es 16 Stunden bei Raumtemperatur unter Hochvakuum. Dies ergab 12,50 g rohen 8'-Apo-β-carotinsäure-ethylester als ziegelrotes Pulver mit Smp. 136-137°C; Gehalt nach HPLC: 99,1% (all-E).

Zur weiteren Reinigung wurden 12,40 g des obigen Produktes in 100 ml Aceton suspendiert und die Suspension unter Rühren rückflussiert. Nach viertelstündigem Rückflussieren ging das Produkt nicht vollständig in Lösung. In die rückflussierende Suspension wurden unter Rühren in etwa einer Minute 5 ml Wasser durch den Rückflusskühler tropfenweise zugegeben, dann wurde langsam auf 0°C abgekühlt, filtriert, zweimal mit je 20 ml, total 40 ml, kaltem Aceton/Wasser (9:1) und zweimal mit je 25 ml, total 50 ml, Wasser bei -20°C gewaschen. Nach Trocknung bei 45°C unter Wasserstrahlvakuum bis zur Gewichtskonstanz und bei Raumtemperatur unter Hochvakuum (0,05 mmHg) wurden 11,00 g (80%ige Ausbeute) 8'-Apo-β-carotinsäure-ethylester als dunkelrot glänzende Fristalle mit Smp. 139°C und einem Gehalt nach HPLC von 99,6% erhalten. UV (Cyclohexan mit 3% Chloroform): 473 nm (log ε = 4,98; $E^{1\%}_{1cm}$ = 2065), 447 nm (log ε = 5,06; $E^{1\%}_{1cm}$ = 2481), 262 nm (log ε = 4,25; $E^{1\%}_{1cm}$ = 385).

Beispiel 29

8'-Apo-β-carotinsäure-ethylester (Durchprozess aus 12'-Apo-β-carotinal-dimethylacetal und 1,1-Dimethoxy-2-methyl-1,3-butadien über 12'-Methoxy-11',12'-dihydro-8'-apo-β-carotinsäure-methylester)

In einem mit einem Magnetrührer ausgestatteten 100 ml-Rundkolben wurde unter Argonbegasung eine Lösung von 6,20 g (15 mMol) 12'-Apo-β-carotinal-dimethylacetal (Smp. 78-79°C; HPLC: 96,5%) und 3,04 g (22,5 mMol) 1,1-Dimethoxy-2-methyl-1,3-butadien (Gehalt nach GC: 95%) in 60 ml tert.Butylmethylether bei -25°C mit 80 mg (etwa 6 Tropfen, etwa 0,6 mMol, 4 Mol%) Bortrifluoridetherat versetzt. Die Lösung verfärbte sich bei der Zugabe des Katalysators tiefblau. Man rührte die Lösung etwa eine Stunde nach [DC($SiO_2$): $R_f$ etwa 0,2 (Toluol)]. Zur Hydrolyse gab man bei -25°C 15 ml Essigsäure/Wasser (9:1) dazu, erwärmte auf 0°C, liess bei Raumtemperatur etwa 30 Minuten rühren und arbeitete analog dem Beispiel 21 auf. Dies ergab 10,0 g rohen 12'-Methoxy-11',12'-dihydro-8'-apo-β-carotinsäure-methylester als rotes, klebriges, ethanolfeuchtes Oel, welches in einem mit mechanischem Rührer, Thermometer und Argonbegasung ausgestatteten 350 ml-Vierhalssulfierkolben in 130 ml absolutem Ethanol gelöst wurde. Nach Zugabe einer durch Lösen von 700 mg (30 mMol) Natrium in 35 ml absolutem Ethanol hergestellten Natriumethylatlösung wurde etwa 16 Stunden bei Raumtemperatur und 1 1/2 Stunden bei 50°C gerührt. Die Aufarbeitung erfolgte wiederum analog dem Beispiel 28. Dies ergab 6,1 g (86%) 8'-Apo-β-carotinsäure-ethylester als rotviolettes Pulver, Smp. 140°C; Gehalt nach HPLC: 97,5%. Umkristallisation in Aceton/Wasser analog dem Beispiel 28 ergab 5,81 g (83%ige Ausbeute) 8'-Apo-β-carotinsäure-ethylester als violette, metallisch glänzende Kristalle, Smp. 141°C; Gehalt nach HPLC: 98,3%.

Beispiel 30

8'-Apo-β-carotinsäure-ethylester (Durchprozess aus 12'-Apo-β-carotinal-dimethylacetal und 2-( 1-Methyl-ethyliden)-[1.3]dioxolan über 12'-Methoxy-11',12'-dihydro-8'-apo-β-carotinsäure-2-hydroxyethylester)

In einem 350 ml-Vierhalssulfierkolben wurden unter Argonbegasung 16,4 g (40 mMol) 12'-Apo-β-carotinal-dimethylacetal (Gehalt nach HPLC: 96,8%) und 7,57 g (60 mMol) 2-(1-Methyl-allyliden)-[1,3]dioxolan (Gehalt nach GC: 100%) in 160 ml Ethylacetat vorgelegt. Bei -25°C gab man dazu 210 mg (4 Mol%) Bortrifluoridetherat und liess bei dieser Temperatur 1 Stunde nachrühren. Dann gab man 40 ml Essigsäure/Wasser (9:1) zu, entfernte die Kühlung und rührte 40 Minuten bei etwa 17°C weiter [DC($SiO_2$): $R_f$ = etwa 0,35; n-Hexan/Ethylacetat (1:1)]. Zur Aufarbeitung wurde das Reaktionsgemisch auf 500 ml Ethylacetat gegossen, zweimal mit je 400 ml, total 800 ml, Wasser, einmal mit 300 ml gesättigter Natriumbicarbonat-Lösung und einmal mit 150 ml gesättigter Kochsalzlösung gewaschen. Dann wurde die organische Phase über wasserfreiem Natriumsulfat getrocknet und eingeengt. Der ölige rote Rückstand wurde zweimal mit je 250 ml, total 500 ml, Ethanol unter vermindertem Druck bei 35°C einrotiert. Dies ergab ein zähflüssiges orangerotes Oel (24 g), welches in 350 ml Ethanol gelöst und in einem mit einem mechanischen Rührer ausgestatteten 750 ml-Vierhalssulfierkolben unter Argonbegasung mit einer durch Lösen von 1,84 g (80 mMol) Natrium in 90 ml Ethanol hergestellten Natriumethylatlösung bei Raumtemperatur versetzt wurde. Man rührte das Gemisch 16 Stunden bei Raumtemperatur und 6 Stunden bei 48°C, bis praktisch mit DC kein Edukt mehr feststellbar war. Nun tropfte man bei Raumtemperatur unter Rühren langsam eine Lösung von 5,3 g (93 mMol) Essigsäure in 15 ml Ethanol zu, gefolgt von 120 ml Ethanol/Wasser (1:1). Dann wurde der rote Kristallbrei abgenutscht, mit 60 ml Ethanol/Wasser (9:1) bei 0°C, mit 180 ml Wasser bei Raumtemperatur und mit 50 ml Ethanol/Wasser (9:1) bei 0°C gewaschen. Nach 15-stündiger Trocknung bei 25 mbar und Raumtemperatur und 7-stündiger Trocknung bei 45°C unter Hochvakuum bis auf 0,16 mbar wurden 12,6 g (68,5%) 8'Apo-β-carotinsäure-ethylester als ziegelrote Kristalle, Smp. 135°C, erhalten. Eine Umkristallisation in Aceton/Wasser erfolgte analog dem Beispiel 28 und ergab 11,5 g (62%) 8'-Apo-β-carotinsäure-ethylester als violette, glänzende Kristalle mit Smp. 139,5°C und einem Gehalt nach HPLC von 99,5%.

Beispiel 31

8'-Apo-β-carotinsäure-methylester

In einem mit Magnetrührer, Kühler und Argonbegasung ausgestatteten 500 ml-Rundkolben wurden 950 mg (41 mMol) Natrium in 60 ml Methanol gelöst. Bei Raumtemperatur gab man dazu eine Lösung von 6,5 g (12,6 mMol) 12'-Methoxy-11',12'-dihydro-8'-apo-β-carotinsäure-methylester (LC: 93%ig) in 250 ml Methanol und 20 ml tert.Butylmethylether. Nun wurde etwa 16 Stunden bei 60°C gerührt, was eine rote Suspension ergab [DC($SiO_2$): $R_f$ = 0,45; Toluol] - Man kühlte die Suspension auf 0°C ab und gab 5 ml Essigsäure zu. Dann wurde der Niederschlag abgenutscht, mit 30 ml Methanol bei 0°C, zweimal mit je 50 ml, total 100 ml, Wasser bei Raumtemperatur und nochmals mit 50 ml Methanol bei 0°C gewaschen und bei 40°C und 12 mbar getrocknet. Dies ergab 4,3 g (76%) 8'-Apo-β-carotinsäure-methylester als rotes Pulver, Smp. 145-146°C; Gehalt nach HPLC: 99,7%; UV (Cyclohexan mit 3% Chloroform): 473 nm (log ε = 4,97; $E_{1cm}^{1\%}$ = 2096), 448 nm (log ε = 5,05; $E_{1cm}^{1\%}$ = 2515).

| Mikroanalyse: | | |
|---|---|---|
| Ber. | C 83,36% | H 9,48% |
| Gef. | C 83,08% | H 9,42% |

Beispiel 32

4'-Apo-β-carotinsäure-ethylester (Neurosporaxanthin-ethylester)

In einem 150 m-Rundkolben wurden unter Argon 1,16 g (2 mMol) 8'-Methoxy-7',8'-dihydro-4'-apo-β-carotinsäure-ethylester (HPLC: 96%ig) in 80 ml Ethanol vorgelegt. Zur resultierenden roten Suspension gab man bei Raumtemperatur eine durch Lösen von 140 mg (6 mMol) Natrium in 10 ml Ethanol hergestellte Natriumethylatlösung zu und liess etwa 16 Stunden bei 40°C rühren [DC($SiO_2$): $R_f$ = etwa 0,4-0,5; Toluol]. Dann kühlte man auf Raumtemperatur ab, gab 1 ml (1,05 g, 16 mMol) Essigsäure zu und kühlte auf 0°C ab. Nachdem die Suspension 2 Stunden bei 0°C gerührt worden war, nutschte man ab und wusch mit 100 ml Ethanol bei -20°C, mit 100 ml Wasser bei Raumtemperatur und nochmals mit 100 ml Ethanol bei -20°C. Nach Trocknung unter Wasserstrahlvakuum bei Raumtemperatur bis zur Gewichtskonstanz wurden 800 mg (75%ige Ausbeute) Neurosporaxanthin-ethylester, Smp. 144-145°C (Gehalt nach HPLC: 98,7%), erhalten. UV (n-Hexan): 502 nm (log ε = 5,09; $E_{1cm}^{1\%}$ = 2173), 471 nm (log ε = 5,18; $E_{1cm}^{1\%}$ = 2857), 450 (log ε =5,06, $E_{1cm}^{1\%}$ = 2173), 290 (log ε = 4,47, $E_{1cm}^{1\%}$ =563).

| Mikroanalyse: | | |
|---|---|---|
| Ber. | C 84,36% | H 9,57% |
| Gef. | C 84,36% | H 9,52% |

Beispiel 33

4'-Apo-β-carotinsäure-ethylester (Durchprozess aus 8'-Apo-β-carotinal-dimethylacetal und 1-Trimethylsilyloxy-1-ethoxy-2-methyl-1,3-butadien über 8'-Methoxy-7',8'-dihydro-4'-apo-β-carotinsäure-ethylester)

In einem mit einem Magnetrührer ausgestatteten 250 ml-Rundkolben wurden unter Argon 4,80 g (10 mMol) 8'-Apo-β-carotinal-dimethylacetal (Gehalt nach HPLC: 97%) und 2,60 g (12 mMol) 1-Trimethylsilyloxy-1-ethoxy-2-methyl-1,3-butadien (Gehalt nach GC: 94%) in 100 ml tert.Butylmethylether suspendiert. Zur Suspension gab man bei 0°C 70 mg (0,5 mMol, 5 Mol%) wasserfreies Zinchlorid, und dann rührt man sie 3 Stunden bei Raumtemperatur, was eine tiefrote Lösung ergab [DC($SiO_2$): $R_f$ = 0,2-0,3; Toluol]. Dann wurde die Lösung auf 100 ml Wasser gegossen, zweimal mit je 100 ml, total 200 ml, tert.Butylmethylether extrahiert, einmal mit 100 ml Wasser und einmal mit 100 ml gesättigter Natriumchlorid-Lösung gewaschen. Die vereinigten organischen Phasen wurden nach Trocknung über wasserfreiem Natriumsulfat eingeengt und ergaben 7,3 g rohen 8'-Methoxy-7',8'-dihydro-4'-apo-β-carotinsäure-ethylester als tiefrotes, zähflüssiges Oel. Dieser Rückstand wurde in 250 ml absolutem Ethanol gelöst und die Lösung in einen mit mechanischem Rührer, Thermometer und Argonbegasung ausgestatteten 350 ml-Vierhalssulfierkolben gegeben. Zur Lösung gab man nun bei Raumtemperatur eine durch Lösen von 600 mg (26 mMol) Natrium in 40 ml absolutem Ethanol hergestellte Natriumethylatlösung und rührte etwa 16 Stunden bei 45°C [DC($SiO_2$): $R_f$= etwa 0,4; Toluol]. Nach Abkühlung auf Raumtemperatur gab man 2,4 g (40 mMol) Essigsäure zu und kühlte auf 0°C ab. Nun wurde abgenutscht, zweimal mit je 10 ml, total 20 ml, Ethanol/Wasser (19:1), einmal mit 50 ml Wasser und noch zweimal mit 10 ml, total 20 ml, Ethanol/ Wasser (19:1) gewaschen. Nach Trocknung unter Wasserstrahlvakuum bei 45°C und anschliessend unter Hochvakuum bei Raumtemperatur wurden 4,2 g (77%ige Ausbeute) 4'-Apo-β-carotinsäure-ethylester als ziegelrote Kristalle, Smp. 144°C und Gehalt nach HPLC von 97,6%, erhalten. Zur weiteren Reinigung wurden das Rohprodukt (4,2 g) in 120 ml Aceton unter etwa 10-minütigem Rühren am Rückfluss unter Argon digeriert. Dann gab man durch den Rückflusskühler tropfenweise innert etwa 5 Minuten 4 ml Wasser, kühlte langsam auf 0°C ab und filtrierte ab. Nachdem das Filtergut mit etwa 20 ml Aceton/Wasser (9:1) bei 0°C, mit 50 ml Wasser bei Raumtemperatur und nochmals mit 10 ml Aceton/$H_2O$ (9:1) bei 0°C gewaschen worden war, trocknete man das Filtergut unter vermindertem Druck bei 50°C und unter Hochvakuum bei Raumtemperatur und erhielt 4,03 g (75%ige Ausbeute) Neurosporaxanthin-ethylester als violette Kristalle mit Smp. 146°C und einem Gehalt nach HPLC von 99,4%.

Beispiel 34

4'-Apo-β-carotinsäure-ethylester (Durchprozess aus 8'-Apo-β-carotinal-dimethylacetal und 1,1-Dimethoxy-2-methyl-1,3-butadien über 8'-Methoxy-7',8'-dihydro-4'-apo-β-carotinsäure-methylester

In einem mit einem Magnetrührer ausgestatteten 200 ml-Rundkolben wurden unter Argonbegasung 4,80 g (10 mMol) 8'-Apo-β-carotinal-dimethylacetal (HPLC: 97%) und 1,95 g (15 mMol) 1,1-Dimethoxy-2-methyl-1,3-butadien (Gehalt nach GC: 99,5%) in 100 ml tert.Butylmethylether vorgelegt. Unter Rühren gab man zur resultierenden Suspension bei 0°C 3 Tropfen (etwa 35 mg, 2,5 Mol%) Bortrifluoridetherat zu. Dabei löste sich die Suspension innert etwa 30 Minuten auf, und es resultierte eine dunkelrote Lösung [DC($SiO_2$): $R_f$ = etwa 0,1; Toluol]. Zur Hydrolyse wurden bei 0°C 20 ml Essigsäure/Wasser (9:1) zugegeben, auf Raumtemperatur erwärmt und bei dieser Temperatur während 30 Minuten gerührt [DC($SiO_2$): $R_f$ = etwa 0,25; Toluol]. Analoge Aufarbeitung wie im Beispiel 33 ergab 6,6 g rohen 8'-Methoxy-7',8'-dihydro-4'-apo-β-carotinsäure-methylester als tiefrotes, zähflüssiges Harz. Dieser Rückstand wurde mit 200 ml Ethanol in einen mit mechanischem Rührer, Thermometer und Argonbegasung ausgestatteten 350 ml-Vierhalssulfierkolben gegeben und bei Raumtemperatur mit einer durch Lösen von 600 mg (26 mMol) Natrium in 90 ml Ethanol hergestellten Natriumethylatlösung versetzt. Anschliessend wurde etwa 16 Stunden bei 45°C gerührt [DC ($SiO_2$): $R_f$ = etwa 0,5; Toluol] und auf Raumtemperatur abgekühlt, und man tropfte dann 2,4 g (40 mMol) Essigsäure, gefolgt von 5 ml Wasser zu. Filtration und Waschen wie im Beispiel 33 beschrieben ergab 4,8 g (89%ige Ausbeute) 4'-Apo-β-carotinsäure-ethylester als rot-violettes Pulver, Smp. 144°C; Gehalt nach HPLC: 98,1%. Eine weitere Reinigung in Aceton/Wasser wie im Beispiel 33 beschrieben ergab 4,44 g (84%ige Ausbeute) 4'-Apo-β-carotinsäure-ethylester als violette, feine Kristalle mit Smp. 146-147°C und einem Gehalt nach HPLC von 99,2%.

Beispiel 35

4'-Apo-β-carotinsäure-methylester (Neurosporaxanthin-methylester; Durchprozess aus 8'-Apo-β-carotinal-dimethylacetal und 1,1-Dimethoxy-2-methyl-1,3-butadien über 8'-Methoxy-7',8'-dihydro-4'-apo-β-carotinsäure-methylester

In einem mit einem magnetischen Rührer ausgestatteten 300 ml-Rundkolben wurden unter Argonbegasung 4,80 g (10 mMol) 8'-Apo-β-carotinal-dimethylacetal (HPLC: 97%) und 2,20 g (17 mMol) 1,1-Dimethoxy-2-methyl-1,3-butadien in 100 ml tert.Butylmethylether suspendiert und bei 0°C mit 2 Tropfen (etwa 25 mg, etwa 2 Mol%) Bortrifluoridetherat versetzt. Nachdem sich die Suspension nach etwa 20-30 Minuten aufgelöst hatte (DC-Kontrolle), wurden bei 0°C 20 ml Essigsäure/Wasser (9:1) zugegeben und 50 Minuten bei Raumtemperatur gerührt. Eine Aufarbeitung wie im Beispiel 33 beschrieben ergab etwa 7 g rohen 8'-Methoxy-7',8'-dihydro-4'-apo-β-carotinsäure-methylester als rotes, hochviskoses Oel.

Dieses wurde in einem mit einem mechanischen Rührer ausgestatteten 350 ml-Vierhalssulfierkolben unter Argon in 250 ml Methanol und 20 ml tert.Butylmethylether suspendiert. Zur Suspension gab man eine durch Lösen von 700 mg (30 mMol) Natrium in 50 ml Methanol hergestellte Natriummethylatlösung, und man rührte etwa 16 Stunden bei 50°C, gab dann nochmals eine Lösung von 360 mg (15 mMol) Natrium in 15 ml Methanol zu und rückflussierte bei etwa 62°C 4 Stunden [DC($SiO_2$): $R_f$ = etwa 0,5; Toluol]. Nun wurde auf 0°C abgekühlt, 3,6 g (60 mMol) Essigsäure zugegeben, abfiltriert und mit 30 ml Methanol bei 0°C, zweimal mit je 25 ml, total 50 ml, Wasser und nochmals mit 40 ml Methanol bei 0°C gewaschen. Nach Trocknung bei 45°C unter Wasserstrahlvakuum und bei Raumtemperatur unter Hochvakuum wurden 4,60 g (88%ige Ausbeute) 4'-Apo-β-carotinsäure-methylester als rot-violette Kristalle mit Smp. 147-148°C und einem Gehalt nach HPLC von 97,8% erhalten. Eine weitere Reinigung dieser Kristalle mit Aceton/Wasser wie im Beispiel 33 beschrieben ergab 4,06 g (78%ige Ausbeute) 4'-Apo-β-carotinsäure-methylester als violette Kristalle mit Smp. 150-151°C und einem Gehalt nach HPLC von 99,1%; UV (Cyclohexan mit 3% Chloroform): 509 nm (log ε = 5,07; $E^{1\%}_{1cm}$ = 2290), 479 nm (log ε = 5,16; $E^{1\%}_{1cm}$ = 2830), 292 nm (log ε = 4,47; $E^{1\%}_{1cm}$ = 574).

| Mikroanalyse: | | |
|---|---|---|
| Ber. | C 84,32% | H 9,44% |
| Gef. | C 84,07% | H 9,30% |

Beispiel 36

3',4'-Didehydro-β,ψ-carotinsäure-ethylester (Torularhodin-ethylester)

In einem mit Magnetrührer, Kühler und Argonbegasung ausgestatteten 100 ml-Rundkolben wurden 800 mg (1,22 mMol) 4'-Methoxy-β,ψ-carotin-16'-säure-ethylester in 20 ml Ethanol vorgelegt. Bei Raumtemperatur gab man

eine durch Lösen von 85 mg (3,7 mMol) Natrium in 10 ml Ethanol hergestellte Natriumethylatlösung zu und rührte 18 Stunden bei 50°C und 30 Minuten bei 70°C [DC($SiO_2$): $R_f$= etwa 0,35; Toluol]. Dann wurde auf 0°C abgekühlt, mit 0,5 ml Essigsäure angesäuert, abgenutscht, mit kaltem Wasser und kaltem Ethanol gewaschen und bei 35°C unter Hochvakuum bis zur Gewichtskonstanz getrocknet. Dies ergab 640 mg (88%ige Ausbeute) Torularhodin-ethylester als tiefviolettes kristallines Pulver mit Smp. 158-160°C und einem Gehalt nach HPLC von 99,5%. UV (Cyclohexan mit 3% Chloroform): 537 nm (log ε = 5,12; $E^{1\%}_{1cm}$ = 2240), 503 nm (log ε =5,22; $E^{1\%}_{1cm}$ = 2815), 480 nm (log ε = 5,11; $E^{1\%}_{1cm}$ =2178), 321 nm (log ε = 4,58; $E^{1\%}_{1cm}$ = 642).

Beispiel 37

3',4'-Didehydro-β,ψ-carotinsäure-methylester (Torularhodin-methylester)

In einem mit einem Magnetrührer ausgestatteten 50 ml-Rundkolben wurden unter Argonbegasung 530 mg (0,79 mMol) 4'-Methoxy-β,ψ-carotin-16'-säure-methylester (Gehalt nach HPLC = 91,3%) in 20 ml Methanol vorgelegt. Bei Raumtemperatur gab man eine durch Lösen von 150 mg (6,5 mMol) Natrium in 15 ml Methanol hergestellte Natriummethylatlösung zu und rührte 24 Stunden bei 60°C [DC($SiO_2$): $R_f$ = etwa 0,4; Toluol]. Dann wurde mit 5 ml Essigsäure angesäuert, auf 0°C abgekühlt, abfiltriert, zweimal mit je 20 ml, total 40 ml, Wasser und zweimal mit je 10 ml, total 20 ml, eiskaltem Methanol gewaschen und unter Hochvakuum bei Raumtemperatur getrocknet. Dies ergab 435 mg (91%ige Ausbeute) Torularhodin-methylester als tiefviolettes, kristallines Pulver mit Smp. 174-177°C und einem Gehalt nach HPLC von 95,3%. UV (Cyclohexan mit 3% Chloroform): 537 nm (log ε = 5,11; $E^{1\%}_{1cm}$ = 2232), 504 nm (log ε = 5,21; $E^{1\%}_{1cm}$ = 2824), 480 nm (log ε = 5,10; $E^{1\%}_{1cm}$ = 2200), 321 nm (log ε = 4,57; $E^{1\%}_{1cm}$ = 642).

Beispiel 38

8'-Apo-β-carotinsäure

In einem mit Magnetrührer ausgestatteten 150 ml-Rundkolben wurden unter Argonbegasung 1,90 g (3,6 mMol) 12'-Methoxy-11,12'-dihydro-8'-apo-β-carotin-8'-säure (HPLC: 88,6%) in 80 ml Tetrahydrofuran gelöst. Dazu gab man bei 0°C 1,83 g (16 mMol, 4,5 Aeq.) Kalium-tert.butylat und rührte 4 Stunden bei dieser Temperatur [DC($SiO_2$): $R_f$ = etwa 0,3-0,4; Toluol/Ethylacetat (4:1)]. Dann wurde auf Wasser gegossen und mit 50 ml Ethylacetat extrahiert. Nun wurde das Extrakt mit 50 ml Wasser und 50 ml gesättigter Natriumchlorid-Lösung gewaschen, über wasserfreiem Natriumsulfat getrocknet und filtriert. Nach dem Einengen unter vermindertem Druck wurden 1,8 g Rückstand erhalten, welcher in 25 ml Toluol heiss digeriert wurde. Nach dem Abkühlen über 18 Stunden auf 0°C wurde filtriert, mit wenig Toluol gewaschen und getrocknet. Dies ergab 570 mg (36%ige Ausbeute) 8'-Apo-β-carotinsäure als ein dunkelrotes Pulver mit Smp. 193-194°C und einer Reinheit gemäss HPLC nach Methylierung mit Diazomethan von 97,2%; UV (Cyclohexan mit 3% Chloroform): 450 nm (log ε = 4,96; $E^{1\%}_{1cm}$ = 2100), 263 nm (log ε = 4,18; $E^{1\%}_{1cm}$ = 350).

Beispiel 39

8,8'-Diapocarotin-8,8'-disäure-ethylester (Crocetindiethylester)

In einem mit mechanischem Rührer, Thermometer und Argonbegasung ausgestatteten 350 ml-Vierhalssulfierkolben wurden 5,91 g (12,7 mMol) 11,12,11',12'-Tetrahydro-12,12'-dimethoxy-8,8'-diapocarotin-8,8'-disäure-ethylester (Gehalt nach HPLC 96,1%) in 150 ml Ethanol vorgelegt. Bei Raumtemperatur tropfte man eine durch Lösen von 920 mg (40 mMol) Natrium in 50 ml Ethanol hergestellte Natriumethylatlösung zu und rührte das Gemisch 20 Stunden bei Raumtemperatur und 2 1/2 Stunden bei 50°C [DC($SiO_2$): $R_f$ = etwa 0,35; n-Hexan/Ethylacetat (4:1)]. Nach Abkühlung auf Raumtemperatur gab man 60 ml Essigsäure zu, kühlte auf 0°C ab, filtrierte den Niederschlag ab und wusch dreimal mit je 100 ml, total 300 ml, Wasser und zweimal mit je 50 ml, total 100 ml, Ethanol bei 0°C. Nach dem Trocknen (12 mbar bei 50°C und eine Stunde unter Hochvakuum bei Raumtemperatur wurden 4,02 g (80%ige Ausbeute) Crocetindiethylester als oranges Pulver mit Smp. 204-210°C und einem Gehalt nach HPLC von 96,7%. Zur Analyse wurde eine Probe aus heissem Toluol umkristallisiert. Die erhaltene Probe besass Smp. 208-211°C; Gehalt nach HPLC: 97,8%; UV (Chloroform): 461 nm (log ε =5,04; $E^{1\%}_{1cm}$ = 2877), 433 nm (log ε = 5,07; $E^{1\%}_{1cm}$ = 3037), 411 nm (log ε = 4,87; $E^{1\%}_{1cm}$ = 1970).

| Mikroanalyse: | | |
|---|---|---|
| Ber. | C 74,97% | H 8,39% |
| Gef. | C 74,72% | H 8,45% |

Beispiel 40

8,8'-Diapocarotin-8,8'-disäure-ethylester (Crocetindiethylester)

In einem mit mechanischem Rührer, Kühler und Argonbegasung ausgestatteten 200 ml-Vierhalssulfierkolben legte man 2,18 g (5,2 mMol) 11,12,11',12'-Tetrahydro-12,12'-dimethoxy-8,8'-diapocarotin-8,8'-disäure-methylester (Gehalt nach HPLC: 99,7%) in 100 ml Ethanol vor. Zum Gemisch tropfte man bei Raumtemperatur eine durch Lösen von 410 mg (17,8 mMol, 3,5 Aeq.) Natrium in 65 ml Ethanol hergestellte Natriumethylatlösung und rührte 16 Stunden bei Raumtemperatur und 5 Stunden bei 45-55°C [DC($SiO_2$): $R_f$ = etwa 0,4; n-Hexan/Ethylacetat (4:1)]. Dann wurde auf 0°C abgekühlt, 25 ml Essigsäure/Wasser (1:9) zugegeben und 1 Stunde bei 0°C gerührt. Nun wurde der Niederschlag abfiltriert, zweimal mit je 50 ml, total 100 ml, Wasser und zweimal mit je 25 ml, total 50 ml, Ethanol bei 0°C gewaschen und bei 12 mbar/40°C getrocknet. Dies ergab 1,55 g (76%ige Ausbeute) Crocetindiethylester als orangerote Kristalle, Smp. 206-210°C; Gehalt nach HPLC: 97,6%.

Beispiel 41

8,8'-Diapocarotin-8,8'-disäure-methylester (Crocetindimethylester)

In einem mechanischen Rührer, Tropftrichter, Thermometer und Argonbegasung ausgestatteten 200 ml-Vierhalssulfierkolben wurden 2,58 g (6,12 mMol) 11,12,11',12'-Tetrahydro-12,12'-dimethoxy-8,8'-diapocarotin-8,8'-disäure-methylester (Gehalt nach HPLC: 99,7%) in 50 ml Methanol vorgelegt. Zum Gemisch tropfte man eine durch Lösen von 410 mg (18 mMol) Natrium in 45 ml Methanol hergestellte Natriummethylatlösung und rührte etwa 16 Stunden bei 50°C [DC($SiO_2$): Rf = etwa 0,2; n-Hexan/Ethylacetat (4:1)]. Nun wurde auf 0°C abgekühlt, 25 ml Essigsäure/Wasser (1:9) zugetropft, 1 1/2 Stunden nachgerührt, abfiltriert und zweimal mit je 50 ml, total 100 ml, Wasser und zweimal mit je 50 ml, total 100 ml, Methanol bei -10°C gewaschen. Nach Trocknung bei 12 mbar/40°C und unter Hochvakuum bei Raumtemperatur wurden 1,95 g (88%ige Ausbeute) Crocetindimethylester als oranges Pulver mit Smp. 212-219°C und einem Gehalt nach HPLC von 97,9% erhalten. Für die analytischen Daten wurde eine Probe aus heissem Toluol umkristallisiert. Diese Probe besass einen Smp. von 219-222°C; Gehalt nach HPLC: 98,7%; UV (Chloroform): 461 nm (log ε =4,98; $E_{1cm}^{1\%}$ = 2654), 434 nm (log ε = 5,00), 410 nm (log ε = 4,82; $E_{1cm}^{1\%}$ = 1851).

| Mikroanalyse: | | |
|---|---|---|
| Ber. | C 74,13% | H 7,92% |
| Gef. | C 73,93% | H 8,02% |

Beispiel 42

4,4'-Diapocarotin-4,4'-disäure-ethylester

In einem mit einem Magnetrührer ausgestatteten 100 ml-Rundkolben wurden unter Argonbegasung 240 mg (10,4 mMol) Natrium in 70 ml Ethanol gelöst. Bei Raumtemperatur gab man 1,00 g (1,68 mMol) 7,8,7',8'-Tetrahydro-8,8'-dimethoxy-4,4'-diapocarotin-4,4'-disäure-ethylester (Gehalt nach HPLC: 97,8%) zu und rückflussierte etwa 18 Stunden [DC($SiO_2$): $R_f$ = etwa 0,6; Toluol/Ethylacetat (9:1)]. Dann wurde die Reaktionslösung auf Raumtemperatur abgekühlt und mit 20 ml Ethanol/ Essigsäure (9:1) versetzt. Das ausgefallene Produkt wurde abgenutscht, dreimal mit je 20 ml total 60 ml, Wasser und dreimal mit je 20 ml, total 60 ml, eiskaltem Ethanol gewaschen und unter Hochvakuum bei Raumtemperatur getrocknet. Dies ergab 560 mg (61%ige Ausbeute) 4,4'-Diapocarotin-4,4'-disäure-ethylester als dunkelviolettes Pulver mit Smp. 188-189°C und einem Gehalt nach HPLC von 94,4%. UV (Cyclohexan mit 3% Chloroform): 525 nm (log ε = 5,12, $E_{1cm}^{1\%}$ = 2524); 490 nm (log ε = 5,21; $E_{1cm}^{1\%}$ = 3154), 461 nm (log ε = 5,07; $E_{1cm}^{1\%}$ = 2287), 313 nm (log ε = 4,56; $E_{1cm}^{1\%}$ = 690).

Beispiel 43

4,4'-Diapocarotin-4,4'-disäure-methylester

In einem mit einem Magnetrührer ausgestatteten 250 ml-Rundkolben wurden unter Argonbegasung 1,00 g (43 mMol) Natrium in 120 ml Methanol gelöst. Bei Raumtemperatur gab man 1,20 g (2,17 mMol) 7,8,7',8'-Tetrahydro-8,8'-dimethoxy-4,4'-diapocarotin-4,4'-disäure-methylester (Gehalt nach HPLC: 98,7%) zu und rückflussierte etwa 16 Stunden [DC($SiO_2$): $R_f$ = etwa 0,6; Toluol/Ethylacetat (9:1)]. Die Suspension wurde nun auf Raumtemperatur abgekühlt,

mit 20 ml Methanol/Eisessig (9:1) versetzt und nach Abkühlung auf 0°C filtriert. Das Filtergut wurde dreimal mit je 20 ml, total 60 ml, Wasser und dreimal mit je 20 ml, total 60 ml, eiskaltem Methanol gewaschen und unter Hochvakuum bei Raumtemperatur getrocknet. Dies ergab 0,60 g (55%ige Ausbeute) 4,4'-Diapocarotin-4,4'-disäure-methylester als rotes Pulver mit Smp. 201°C und einem Gehalt nach HPLC von 97,9%. UV (Cyclohexan mit 3% Chloroform): 525 nm (log ε = 5,18, $E^{1\%}_{1cm}$ = 3089), 491 nm (log ε = 5,24; $E^{1\%}_{1cm}$ = 3547), 462 nm (log ε = 5,08; $E^{1\%}_{1cm}$ = 2440), 313 nm (log ε = 4,61; $E^{1\%}_{1cm}$ = 834).

## Patentansprüche

**1.** Verfahren zur Herstellung eines Polyenesters oder einer Polyensäure der allgemeinen Formel

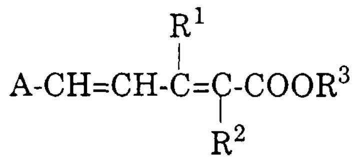

I'

oder

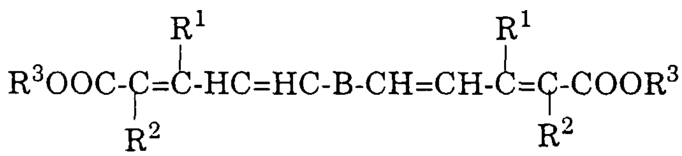

I''

worin

| | |
|---|---|
| A | eine monovalente, gegebenenfalls methylsubstituierte, konjugierte Polyengruppe, |
| B | eine bivalente, gegebenenfalls methylsubstituierte, konjugierte Polyengruppe, |
| $R^1$ und $R^2$ | jeweils Wasserstoff oder Methyl und |
| $R^3$ | Wasserstoff oder $C_{1-6}$-Alkyl bedeuten, wobei sich die Gruppe(n) $-CH=CH-C(R^1)=C(R^2)-COOR^3$ jeweils in der(den) Endstellung(en) der konjugierten Kette der Gruppe A bzw. B befindet(n), |

dadurch gekennzeichnet, dass man ein Polyen-(di-)O,O-dialkylacetal der allgemeinen Formel

$$A\text{-}CH(OR^4)_2 \qquad II'$$

bzw.

$$(R^4O)_2HC\text{-}B\text{-}CH(OR^4)_2 \qquad II''$$

worin

| | |
|---|---|
| A und B | die oben angegebenen Bedeutungen besitzen, wobei sich in diesem Fall die Gruppe(n) $-CH(OR^4)_2$ jeweils in der(den) Endstellung(en) der konjugierten Kette der Gruppe A bzw. B befindet(n) und |
| $R^4$ | $C_{1-6}$-Alkyl bedeutet, |

mit einem Vinylketenacetal oder Analog davon der allgemeinen Formel

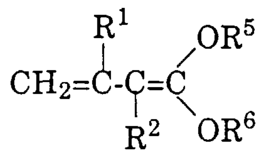

III

worin

| | |
|---|---|
| $R^1$ und $R^2$ | die oben angegebenen Bedeutungen besitzen und |
| $R^5$ | $C_{1-6}$-Alkyl und |
| $R^6$ | $C_{1-6}$-Alkyl oder Tri($C_{1-6}$-alkyl)silyl bedeuten, |

oder $R^5$ und $R^6$ beide Tri($C_{1-6}$-alkyl)silyl bedeuten,
oder $R^5$ und $R^6$ zusammen 1,2-Ethylen oder 1,3-Trimethylen bilden,
in Gegenwart einer Lewissäure umsetzt, im Falle der Verwendung eines Vinylketenacetals der Formel III, in der $R^5$ und $R^6$ beide $C_{1-6}$-Alkyl oder beide Tri($C_{1-6}$-alkyl)silyl bedeuten oder zusammen 1,2-Ethylen oder 1,3-Trimethylen bilden, das Reaktionsgemisch hydrolysiert, anschliessend (in allen Fällen) von der so hergestellten Verbindung der allgemeinen Formel

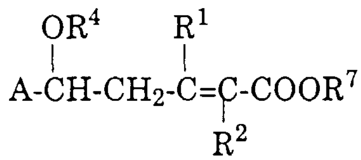

IV'

bzw.

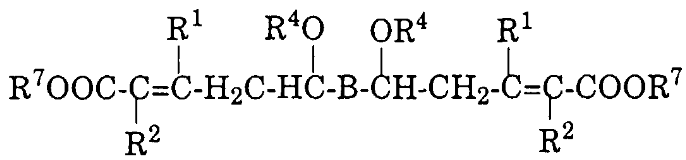

IV"

worin

| | |
|---|---|
| A, B, $R^1$, $R^2$ und $R^4$ | die oben angegebenen Bedeutungen besitzen, wobei sich in diesem Fall die Gruppe (n) $-CH(OR^4)-CH_2-C(R^1)=C(R^2)COOR^7$ jeweils in der(den) Endstellung(en) der konjugierten Kette der Gruppe A bzw. B befindet(n), und |
| $R^7$ | $C_{1-6}$-Alkyl, Wasserstoff, 2-Hydroxyethyl oder 3-Hydroxy-n-propyl bedeutet, |

unter stark basischen Bedingungen den Alkohol $R^4OH$ abspaltet und im Falle eines Unterschieds zwischen der (den) Gruppe(n) $-COOR^7$ und $-COOR^3$ die erstere(n) in die letztere(n) überführt.

**2.** Verfahren nach Anspruch 1, dadurch gekennzeichnet, dass als Polyen-(di-)O,O-dialkylacetal eine Verbindung der allgemeinen Formel

$$R\text{-}CH(OR^4)_2 \qquad II$$

worin

R eine Gruppe (a), (b) oder (c)

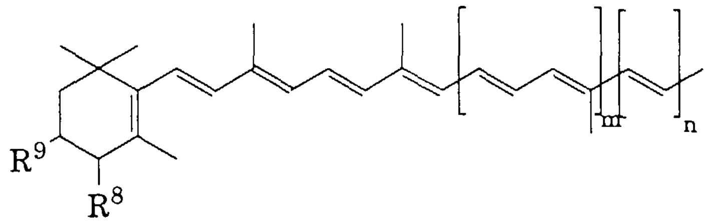

(a)

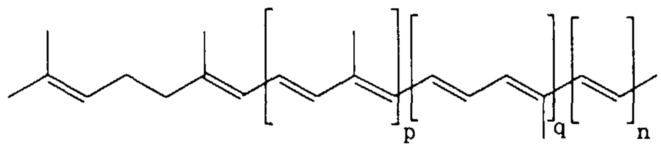

(b)

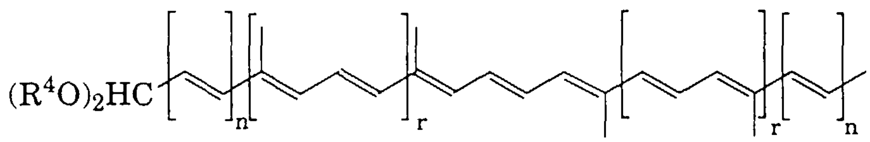

(c)

bedeutet, in der

| | |
|---|---|
| $R^4$ | $C_{1-4}$-Alkyl, |
| $R^8$ und $R^9$ | unabhängig voneinander Wasserstoff, eine gegebenenfalls geschützte Hydroxygruppe oder eine gegebenenfalls geschützte Oxogruppe, |
| m | 0, 1, 2, 3 oder 4, |
| n | 0 oder 1, |
| p | 0, 1 oder 2, |
| q | 0, 1, 2 oder 3 und |
| r | 0, 1 oder 2 bedeuten, |

nach Durchführung des im Anspruch 1 definierten mehrstufigen Verfahrens in das entsprechende Produkt der allgemeinen Formel

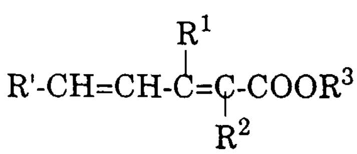

I

worin R' die oben angegebene Bedeutung von R hat, wobei, im Falle, dass R' eine Gruppe (c) bedeutet, die Dialkoxymethylgruppe $(R^4O)_2HC$- durch die Gruppe $R^3OOC-C(R^2)=C(R^1)-HC=HC$- ersetzt ist,
übergeführt wird, und dass man im Falle des Vorhandenseins einer Gruppe (a) im Produkt der Formel I gewünschtenfalls eine allfällig vorhandene Schutzgruppe abspaltet.

**3.** Verfahren nach Anspruch 1 oder 2, dadurch gekennzeichnet, dass man das Polyen-(di-)O,O-dialkylacetal der Formel II', II'' bzw. II mit dem Vinylketenacetal(-Analog) der Formel III in einem organischen Lösungsmittel bei Temperaturen im Bereich von etwa -40°C bis etwa 100°C und in Gegenwart einer Lewissäure umsetzt, wobei die Lewissäure Zinkchlorid, Zinkbromid, Titantetrachlorid, Lithiumperchlorat, Bortrifluoridetherat oder Eisen(III)chlorid ist.

**4.** Verfahren nach Anspruch 3, dadurch gekennzeichnet, dass als organisches Lösungsmittel ein niederer aliphatischer oder cyclischer Kohlenwasserstoff, ein niederer, halogenierter aliphatischer Kohlenwasserstoff, ein niederer

aliphatischer oder cyclischer Ether, ein niederes aliphatisches Nitril oder ein Aromat verwendet wird.

**5.** Verfahren nach Anspruch 4, dadurch gekennzeichnet, dass als organisches Lösungsmittel n-Pentan, n-Hexan, Cyclohexan, Methylenchlorid, Chloroform, Diethylether, tert.Butylmethylether, Tetrahydrofuran, Acetonitril oder Toluol verwendet wird und die Umsetzung des Polyen-(di-)-O,O-dialkylacetals mit dem Vinylketenacetal(-Analog) im Temperaturbereich von etwa -20°C bis zur Raumtemperatur erfolgt.

**6.** Verfahren nach einem der Ansprüche 1 bis 5, dadurch gekennzeichnet, dass man zur Abspaltung des Alkohols $R^4OH$ von der Verbindung der Formel IV' oder IV'' ein Alkalimetallalkoholat oder ein Alkalimetallhydrid als starke Base verwendet.

**7.** Verfahren nach Anspruch 6, dadurch gekennzeichnet, dass man die Abspaltung des Alkohols $R^4OH$ in einem Alkohol, einem aliphatischen oder cyclischen Ether, einem aliphatischen Ester, einem Aromat, einem aliphatischen Kohlenwasserstoff oder einem niederen halogenierten aliphatischen Kohlenwasserstoff, oder in einem Gemisch eines Alkohols mit einem anderen hier erwähnten Lösungsmittel, durchführt.

**8.** Verfahren nach den Ansprüchen 6 und 7, dadurch gekennzeichnet, dass man die Abspaltung des Alkohols $R^4OH$ unter Verwendung eines Natriumalkoxids als Base und des entsprechenden Alkanols als Lösungsmittel bei Temperaturen zwischen der Raumtemperatur und der Rückflusstemperatur des jeweiligen Reaktionsgemisches durchführt.

**9.** Verbindungen der allgemeinen Formeln

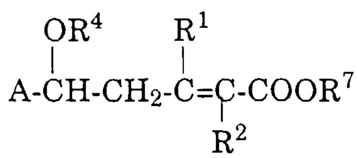

IV'

und

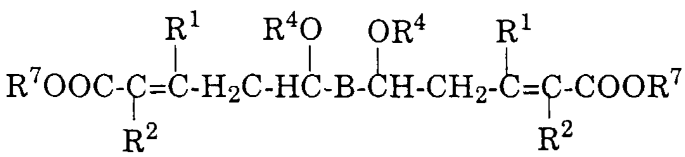

IV''

worin

| | |
|---|---|
| A | eine monovalente, gegebenenfalls methylsubstituierte, konjugierte Polyengruppe, |
| B | eine bivalente, gegebenenfalls methylsubstituierte, konjugierte Polyengruppe, |
| $R^1$ und $R^2$ | jeweils Wasserstoff oder Methyl, |
| $R^4$ | $C_{1-6}$-Alkyl und |
| $R^7$ | $C_{1-6}$-Alkyl, Wasserstoff, 2-Hydroxyethyl oder 3-Hydroxy-n-propyl bedeuten, wobei sich die Gruppe(n) $-CH(OR^4)-CH_2-C(R^1)=C(R^2)-COOR^7$ jeweils in der(den) Endstellung(en) der konjugierten Kette der Gruppe A bzw. B befindet(n), |

insbesondere diejenigen Verbindungen der allgemeinen Formel IV' bzw. IV'', worin A eine Gruppe (a) oder (b)

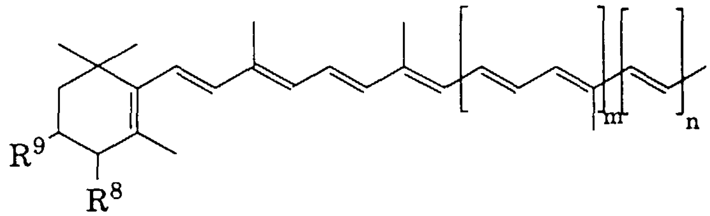

(a)

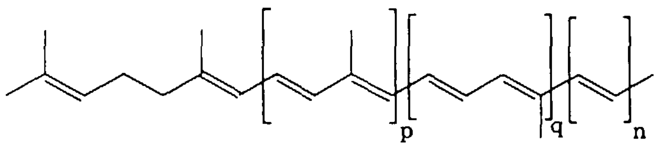

(b)

bzw. B eine Gruppe

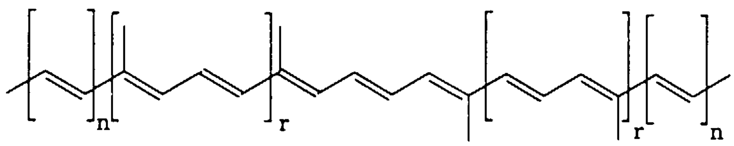

bedeutet, in der

| | |
|---|---|
| $R^8$ und $R^9$ | unabhängig voneinander Wasserstoff, eine gegebenenfalls geschützte Hydroxygruppe oder eine gegebenenfalls geschützte Oxogruppe, |
| m | 0, 1, 2, 3 oder 4, |
| n | 0 oder 1, |
| p | 0, 1 oder 2, |
| q | 0, 1, 2 oder 3 und |
| r | 0, 1 oder 2 bedeuten. |

**10.** Verbindungen der allgemeinen Formel

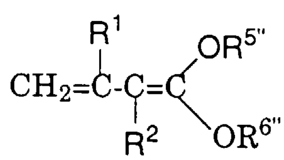

IIIa

worin $R^1$ und $R^2$ jeweils Wasserstoff oder Methyl und $R^{5''}$ und $R^{6''}$ beide $C_{1-6}$-Alkyl bedeuten,
mit Ausnahme von 1,1-Dimethoxy-1,3-butadien und 1,1-Diethoxy-3-methyl-1,3-butadien,
sowie der allgemeinen Formel

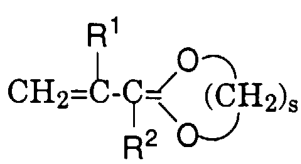

IIIb

worin $R^1$ und $R^2$ die oben angegebenen Bedeutungen besitzen und s 2 oder 3 bedeutet, mit Ausnahme von 2-Allyliden-[1,3]dioxolan, 2-(1-Methyl-allyliden)-[1,3]dioxolan und 2-(2-Methyl-allyliden)-[1,3] dioxolan.

## Claims

**1.** A process for the manufacture of a polyene ester or a polyene acid of the general formula

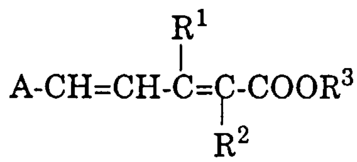

I'

or

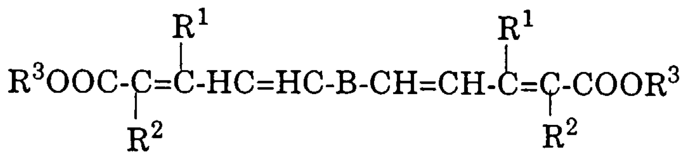

I''

wherein

| | |
|---|---|
| A | signifies a monovalent, optionally methyl-substituted, conjugated polyene group, |
| B | signifies a bivalent, optionally methyl-substituted, conjugated polyene group, |
| $R^1$ and $R^2$ | each signify hydrogen or methyl and |
| $R^3$ | signifies hydrogen or $C_{1-6}$-alkyl, with the $-CH=CH-C(R^1)=C(R^2)-COOR^3$ group(s) in each case being situated in the terminal position (s) of the conjugated chain of group A or B, |

which process comprises reacting a polyene (di)O,O-dialkyl acetal of the general formula

$$A\text{-}CH(OR^4)_2 \qquad II'$$

or

$$(R^4O)_2HC\text{-}B\text{-}CH(OR^4)_2 \qquad II''$$

wherein

| | |
|---|---|
| A and B | have the significances given above, with in this case the $-CH(OR^4)_2$ group(s) being situated in the terminal position(s) of the conjugated chain of group A or B and |
| $R^4$ | signifies $C_{1-6}$-alkyl, |

with a vinylketene acetal or analogue thereof of the general formula

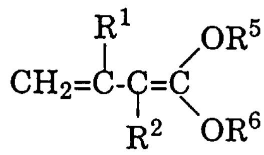

III

wherein

| | |
|---|---|
| $R^1$ and $R^2$ | have the significances given above and |
| $R^5$ | signifies $C_{1-6}$-alkyl and |
| $R^6$ | signifies $C_{1-6}$-alkyl or tri($C_{1-6}$-alkyl)silyl |

or $R^5$ and $R^6$ both signify tri($C_{1-6}$-alkyl)silyl
or $R^5$ and $R^6$ together form 1,2-ethylene or 1,3-trimethylene,
in the presence of a Lewis acid, hydrolyzing the reaction mixture where a vinylketene acetal of formula III in which $R^5$ and $R^6$ both signify $C_{1-6}$-alkyl or both signify tri($C_{1-6}$-alkyl)silyl or together form 1,2-ethylene or 1,3-trimethylene is used, subsequently (in all cases) cleaving off the alcohol $R^4OH$ under strong basic conditions from the thus-produced compound of the general formula

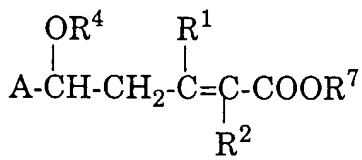

IV'

or

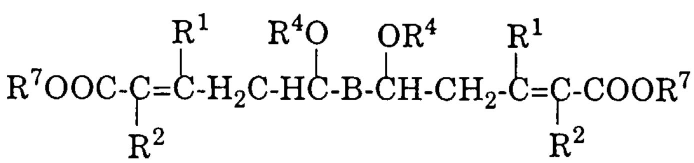

IV''

wherein

| | |
|---|---|
| A, B, $R^1$, $R^2$ and $R^4$ | have the significances given above, with in this case the $-CH(OR^4)-CH_2-C(R^1)=C(R^2)COOR^7$ group(s) being situated in the terminal position(s) of the conjugated chain of group A or B, and |
| $R^7$ | signifies $C_{1-6}$-alkyl, hydrogen, 2-hydroxyethyl or 3-hydroxy-n-propyl, |

and, where there is a difference between group(s) $-COOR^7$ and $-COOR^3$, converting the former into the latter.

**2.** A process according to claim 1, wherein the polyene (di)O,O-dialkyl acetal is a compound of the general formula

$$R\text{-}CH(OR^4)_2 \qquad II$$

wherein

R signifies a group (a), (b) or (c)

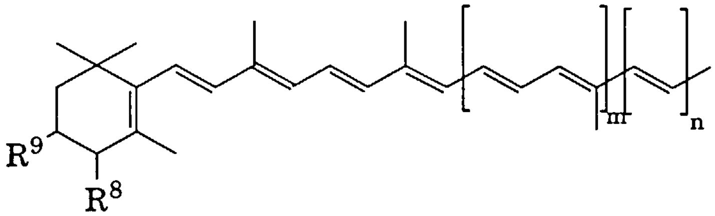
(a)

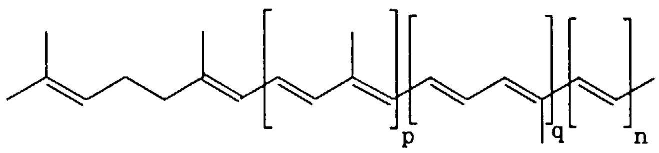
(b)

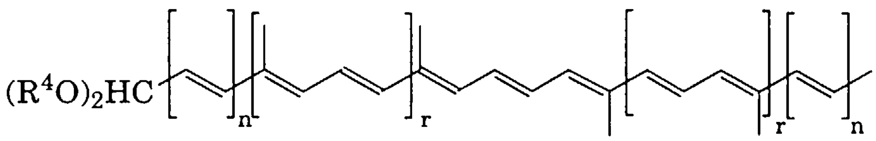
(c)

in which

| | |
|---|---|
| $R^4$ | signifies $C_{1-4}$-alkyl, |
| $R^8$ and $R^9$ | each independently signify hydrogen, an optionally protected hydroxy group or an optionally protected oxo group, |
| m | signifies 0, 1, 2, 3 or 4, |
| n | signifies 0 or 1, |
| p | signifies 0, 1 or 2, |
| q | signifies 0, 1, 2 or 3 and |
| r | signifies 0, 1 or 2, |

and is converted after carrying out the multistage process defined in claim 1 into the corresponding product of the general formula

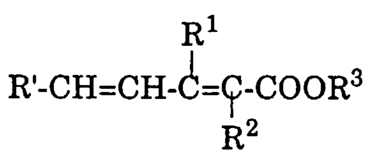
I

wherein R' has the significance given above for R, with the dialkoxymethyl group $(R^4O)_2HC$- being replaced by the $R^3OOC-C(R^2)=C(R^1)-HC=HC$- group when R' signifies a group (c),
and, when a group (a) is present in the product of formula I, any protecting group present is cleaved off, if desired.

**3.** A process according to claim 1 or 2, wherein the polyene (di)O,O-dialkyl acetal of formula II', II" or II is reacted with the vinylketene acetal (analogue) of formula III in an organic solvent at temperatures in the range of about -40°C to about 100°C and in the presence of a Lewis acid selected from zinc chloride, zinc bromide, titanium tetrachloride, lithium perchlorate, boron trifluoride etherate and iron(III) chloride.

**4.** A process according to claim 3, wherein a lower aliphatic or cyclic hydrocarbon, a lower, halogenated aliphatic

hydrocarbon, a lower aliphatic or cyclic ether, a lower aliphatic nitrile or an aromatic is used as the organic solvent.

**5.** A process according to claim 4, wherein n-pentane, n-hexane, cyclohexane, methylene chloride, chloroform, diethyl ether, tert.butyl methyl ether, tetrahydrofuran, acetonitrile or toluene is used as the organic solvent and the reaction of the polyene (di)O,O-dialkyl acetal with the vinylketene acetal (analogue) is effected in the temperature range of about -20°C to room temperature.

**6.** A process according to any one of claims 1 to 5, wherein an alkali metal alcoholate or an alkali metal hydride is used as the strong base for the cleavage of the alcohol $R^4OH$ from the compound of formula IV' or IV".

**7.** A process according to claim 6, wherein the cleavage of the alcohol $R^4OH$ is carried out in an alcohol, an aliphatic or cyclic ether, an aliphatic ester, an aromatic, an aliphatic hydrocarbon or a lower halogenated aliphatic hydrocarbon or in a mixture of an alcohol with another solvent referred to herein.

**8.** A process according to claims 6 and 7, wherein the cleavage of the alcohol $R^4OH$ is carried out using a sodium alkoxide as the base and the corresponding alkanol as the solvent at temperatures between room temperature and the reflux temperature of the respective reaction mixture.

**9.** Compounds of the general formulae

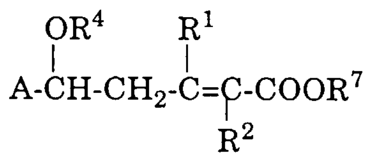

IV'

and

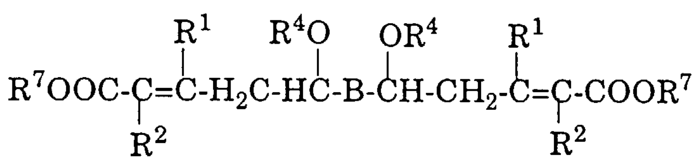

IV"

wherein

| | |
|---|---|
| A | signifies a monovalent, optionally methyl-substituted, conjugated polyene group, |
| B | signifies a bivalent, optionally methyl-substituted, conjugated polyene group, |
| $R^1$ and $R^2$ | each signify hydrogen or methyl, |
| $R^4$ | signifies $C_{1-6}$-alkyl and |
| $R^7$ | signifies $C_{1-6}$-alkyl, hydrogen, 2-hydroxyethyl or 3-hydroxy-n-propyl, with the $-CH(OR^4)-CH_2-C(R^1)=C(R^2)-COOR^7$ group(s) in each case being situated in the terminal position(s) of the conjugated chain of group A or B, |

especially those compounds of general formulae IV' and, respectively, IV" in which A signifies a group (a) or (b)

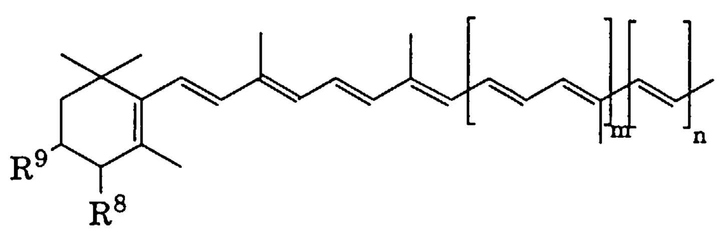

(a)

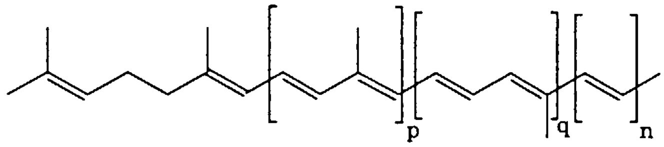

(b)

and B signifies

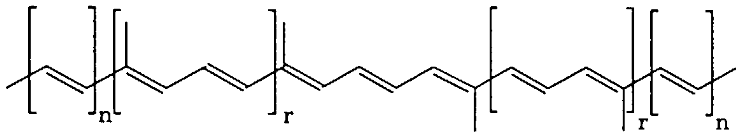

in which

| | |
|---|---|
| $R^8$ and $R^9$ | each independently signify hydrogen, an optionally protected hydroxy group or an optionally protected oxo group, |
| m | signifies 0, 1, 2, 3 or 4, |
| n | signifies 0 or 1, |
| p | signifies 0, 1 or 2, |
| q | signifies 0, 1, 2 or 3 and |
| r | signifies 0, 1 or 2. |

**10.** Compounds of the general formula

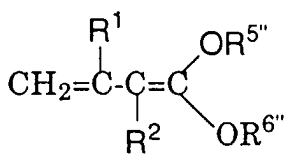

IIIa

wherein $R^1$ and $R^2$ each signify hydrogen or methyl and $R^{5"}$ and $R^{6"}$ both signify $C_{1-6}$-alkyl, with the exception of 1,1-dimethoxy-1,3-butadiene and 1,1-diethoxy-3-methyl-1,3-butadiene, and of the general formula

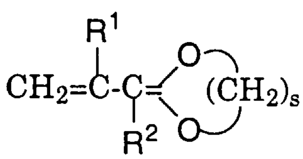

IIIb

wherein $R^1$ and $R^2$ have the significances given above and s signifies 2 or 3, with the exception of 2-allylidene-[1,3]dioxolane, 2-(1-methyl-allylidene)-[1,3]dioxolane and 2-(2-methyl-allylidene)-[1,3]-dioxolane.

## Revendications

**1.** Procédé de préparation d'un ester de polyène ou d'un acide polyénoïque de formule générale

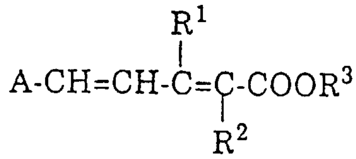 I'

ou

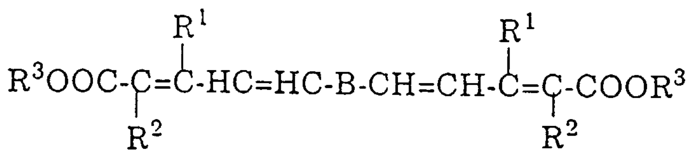 I"

dans laquelle

A représente un groupe polyène conjugué monovalent, éventuellement méthyl-substitué,
B représente un groupe polyène conjugué bivalent, éventuellement méthyl-substitué,
$R^1$ et $R^2$ représente chacun un hydrogène ou un méthyle et
$R^3$ représente un hydrogène ou un alkyle en $C_1$ à $C_6$,

où le(s) groupe(s) -CH=CH-C($R^1$)=C($R^2$)-COO$R^3$ se trouve(nt) toujours dans la ou les positions finales de la chaîne conjuguée du groupe A ou selon les cas B,
caractérisé en ce qu'on fait réagir un polyène-(di-)-O,O-dialkylacétal de formule générale

$$A\text{-}CH(OR^4)_2 \qquad II'$$

ou selon les cas

$$(R^4O)_2HC\text{-}B\text{-}CH(OR^4)_2 \qquad II"$$

dans lesquelles

A et B ont les significations indiquées ci-dessus, où dans ce cas le ou les groupes -CH($OR^4$)$_2$ se trouvent à chaque fois dans la ou les positions finales de la chaîne conjuguée du groupe A ou selon les cas B et
$R^4$ représente un alkyle en $C_1$ à $C_6$,
avec un vinylcétène acétal ou un de ses analogues de formule générale

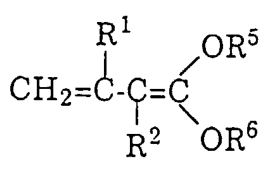 III

dans laquelle
$R^1$ et $R^2$ ont les significations indiquées ci-dessus et
$R^5$ représente un alkyle en $C_1$ à $C_6$ et
$R^6$ représente un alkyle en $C_1$ à $C_6$ ou un tri(alkyle en $C_1$ à $C_6$)silyle, ou
$R^5$ et $R^6$ représentent tous deux un tri(alkyle en $C_1$ à $C_6$)silyle,
ou $R^5$ et $R^6$ forment ensemble un 1,2-éthylène ou un 1,3-triméthylène,
en présence d'un acide de Lewis, dans le cas de l'utilisation d'un vinylcétène acétal de formule III, dans lequel

$R^5$ et $R^6$ représentent tous deux un alkyle en $C_1$ à $C_6$ ou tous deux représentent un tri(alkyle en $C_1$ à $C_6$)silyle ou forment ensemble un 1,2-éthylène ou un 1,3-triméthylène, on hydrolyse le mélange réactionnel, puis (dans tous les cas) on sépare du composé ainsi préparé de formule générale

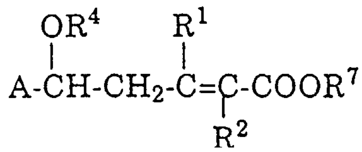

IV'

ou selon les cas

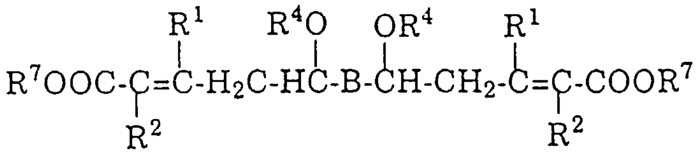

IV''

dans laquelle

A, B, $R^1$, $R^2$ et $R^4$ ont les significations indiquées ci-dessus, où dans ce cas le ou les groupes $-CH(OR^4)-CH_2-C(R^1)=C(R^2)COOR^7$ se trouvent à chaque fois dans la ou les positions finales de la chaîne conjuguée du groupe A ou selon les cas B, et

$R^7$ représentent un alkyle en $C_1$ à $C_6$, un hydrogène, un 2-hydroxyéthyle ou un 3-hydroxy-n-propyle,

dans des conditions fortement basiques l'alcool $R_4OH$ et, dans le cas d'une différence entre le ou les groupes $-COOR^7$ et $-COOR^3$, on transforme le ou les premiers en le ou les derniers.

**2.** Procédé selon la revendication 1, caractérisé en ce qu'on transforme comme polyène-(di-)O,O-dialkylacétal un composé de formule générale

$$R\text{-}CH(OR^4)_2 \qquad II$$

dans laquelle

R représente un groupe (a), (b) ou (c)

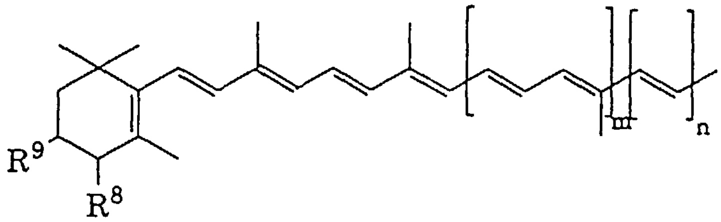

(a)

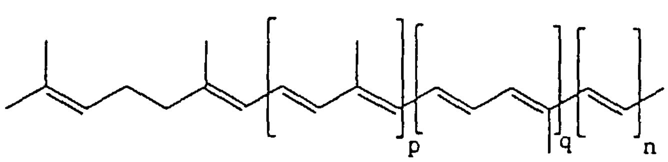

(b)

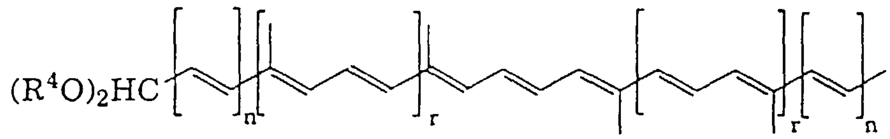

(c)

où
$R^4$ représente un alkyle en $C_1$ à $C_4$, $R^8$ et $R^9$ représentent indépendamment l'un de l'autre un hydrogène, un groupe hydroxy éventuellement protégé ou un groupe oxo, éventuellement protégé,
m vaut 0, 1, 2, 3 ou 4,
n vaut 0 ou 1,
p vaut 0, 1 ou 2,
q vaut 0, 1, 2 ou 3 et
r vaut 0, 1 ou 2,

après réalisation du procédé à plusieurs étapes défini dans la revendication 1 en le produit correspondant de formule générale

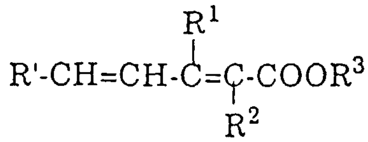

I

dans laquelle R' a la signification de R indiquée ci-dessus, dans le cas où R' représente un groupe (c), le groupe dialcoxyméthyle $(R^4O)_2HC$- est remplacé par le groupe $R^3OOC$-C $(R^2)$=C$(R^1)$-HC=HC-,
et en ce que en cas de présence d'un groupe (a) dans le produit de formule I, si on le désire on sépare un groupe protecteur éventuellement présent.

3. Procédé selon la revendication 1 ou 2, caractérisé en ce qu'on fait réagir le polyène-(di)-O,O-dialkylacétal de formule II', II" ou selon les cas II avec le vinylcétène acétal (ou son analogue) de formule III dans un solvant organique à des températures comprises entre environ -40°C et environ 100°C et en présence d'un acide de Lewis, où l'acide de Lewis est le chlorure de zinc, le bromure de zinc, le tétrachlorure de titane, le perchlorate de lithium, l'éthérate de trifluorure de bore ou le chlorure de fer (III).

4. Procédé selon la revendication 3, caractérisé en ce qu'on utilise comme solvant organique un hydrocarbure aliphatique inférieur ou cyclique, un hydrocarbure aliphatique inférieur halogéné, un éther inférieur aliphatique ou cyclique, un nitrile aliphatique ou un composé aromatique.

5. Composé selon la revendication 4, caractérisé en ce qu'on utilise comme solvant le n-pentane, le n-hexane, le cyclohexane, le chlorure de méthylène, le chloroforme, l'éther diéthylique, l'éther tert.-butylméthylique, le tétrahydrofuranne, l'acétonitrile ou le toluène, et en ce qu'on conduit la réaction du polyène-(di-)-O,O-dialkylacétal avec le vinylcétène acétal (ou son analogue) dans un intervalle de température allant d'environ -20°C à la température ambiante.

6. Procédé selon l'une des revendications 1 à 5, caractérisé en ce que pour la séparation de l'alcool $R^4OH$ d'avec le composé de formule IV' ou IV" on utilise un alcoolate de métal alcalin ou un hydrure de métal alcalin comme base forte.

7. Procédé selon la revendication 6, caractérisé en ce qu'on conduit la séparation de l'alcool $R^4OH$ dans un alcool, un éther aliphatique ou cyclique, un ester aliphatique, un composé aromatique, un hydrocarbure aliphatique ou un hydrocarbure aliphatique halogéné inférieur, ou dans un mélange d'un alcool avec un autre solvant ici mentionné.

8. Procédé selon les revendications 6 et 7, caractérisé en ce qu'on conduit la séparation de l'alcool $R^4OH$ en utilisant un alcoxyde de sodium comme base et l'alcanol correspondant comme solvent à des températures comprises

entre la température ambiante et la température de reflux du mélange réactionnel considéré.

**9.** Composés de formules générales

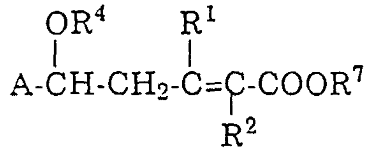

IV'

et

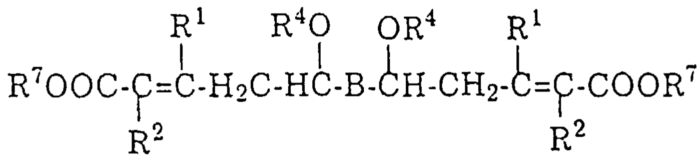

IV"

dans lesquelles

A représente un groupe polyène conjugué monovalent éventuellement méthyl-substitué,
B représente un groupe polyène conjugué bivalent éventuellement méthyl-substitué,
$R_1$ et $R_2$ représentent chacun un hydrogène ou un méthyle,
$R^4$ représentent un alkyle en $C_1$ à $C_6$, et
$R^7$ représente un alkyle en $C_1$ à $C_6$, un hydrogène, un 2-hydroxyéthyle ou un 3-hydroxy-n-propyle,

où le ou les groupes $-CH(OR^4)-CH_2-C(R^1)=C(R^2)-COOR^7$ se trouve(nt) à chaque fois dans la ou les positions finales de la chaîne conjuguée du groupe A ou selon les cas B,
en particulier les composés de formule générale IV' ou selon les cas IV", où A représente les groupes (a) ou (b)

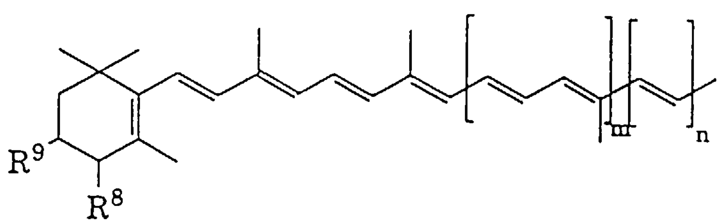

(a)

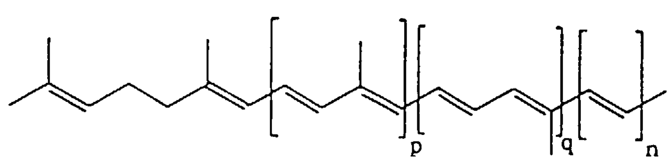

(b)

où selon les cas B représente un groupe

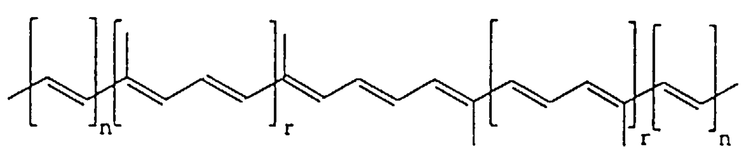

où

$R^8$ et $R^9$ représentent indépendamment l'un de l'autre un hydrogène, un groupe hydroxy éventuellement protégé ou un groupe oxo, éventuellement protégé,
m vaut 0, 1, 2, 3 ou 4,
n vaut 0 ou 1,
p vaut 0, 1 ou 2,
q vaut 0, 1, 2 ou 3 et
r vaut 0, 1 ou 2,

**10.** Composés de formule générale

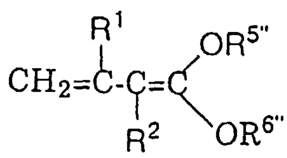

IIIa

dans laquelle $R^1$ et $R^2$ représentent l'un et l'autre un hydrogène et un méthyle et $R^{5"}$ et $R^{6"}$ représentent tous deux un alkyle en $C_1$ à $C_6$,
à l'exception du 1,1-diméthoxy-1,3-butadiène et du 1,1-diéthoxy-3-méthyl-1,3-butadiène,
ainsi que de formule générale

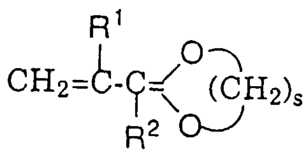

IIIb

dans laquelle $R^1$ et $R^2$ ont les significations indiquées ci-dessus et
s vaut 2 ou 3,
à l'exception du 2-allylidène-[1,3]dioxolane, du 2-(1-méthyl-allylidène)-[1,3]dioxolane et du 2-(2-méthyl-allylidène)-[1,3]dioxolane.